# EUROPEAN PATENT APPLICATION

(11) **EP 4 663 206 A1**
(43) Date of publication of application: **17.12.2025**
(21) Application number: 24753679.0
(22) Date of filing: 08.02.2024
(51) Int. Cl.: A61K 47/68, A61K 47/69, A61K 49/18, A61K 49/08

(54) **NEXT-GENERATION ADC LINKER PLATFORM TECHNOLOGY AND POLYSACCHARIDE CROSS-LINKED COLLOIDAL PARTICLE-DRUG CONJUGATE**

(30) Priority: 08.02.2023 KR 20230016884
(71) Applicant: Inventera Inc., Seoul 06588 (KR)
(72) Inventor: SHIN, Tae-Hyun, Gyeonggi-do 16003 (KR); KIM, Ji-wook, Seoul 07361 (KR)
(74) Representative: Schiweck Weinzierl Koch Patentanwälte Partnerschaft mbB
(86) International application number: PCT/KR2024/001953
(87) International publication number: WO 2024/167358

(57) **Abstract**

The present invention relates to a next-generation antibody-drug conjugate (ADC) linker platform technology and to polysaccharide-crosslinked colloidal particle-drug conjugates.

More specifically, the invention is characterized by the use of polysaccharide-crosslinked colloidal particles-formed by intramolecular and/or intermolecular crosslinking of 1 to 3 branched polysaccharides or 2 to 30 cyclic polysaccharides at hydroxyl groups of their monosaccharide building blocks using a crosslinker-as a multivalent linker system or drug carrier. For example, the invention includes dextran-crosslinked nanoparticles (CDex), which are formed by intramolecular and/or intermolecular crosslinking of 1 to 3 dextran or dextran derivatives at the hydroxyl groups of glucose building blocks using a crosslinker.

## Description

### TECHNICAL FIELD

The present invention relates to a next-generation antibody-drug conjugate (ADC) linker platform technology and to polysaccharide-crosslinked colloidal particle-drug conjugates. The next-generation ADC linker platform technology according to the present invention employs a multivalent linker system comprising polysaccharide-crosslinked colloidal particles formed by intramolecular and/or intermolecular crosslinking of 1 to 3 branched polysaccharides or 2 to 30 cyclic polysaccharides at hydroxyl groups of their monosaccharide building blocks using a crosslinker. In particular, the platform may utilize a multivalent linker system comprising dextran-crosslinked nanoparticles (CDex), in which 1 to 3 dextran or dextran derivatives are crosslinked at hydroxyl groups of glucose building blocks to form a crosslinked colloidal structure. Accordingly, the present invention can provide not only ADCs but also various conjugates comprising two or more molecular components linked via a multivalent linker system that includes polysaccharide-crosslinked colloidal particles with two or more crosslinker-derived reactive groups exposed on the surface.

### BACKGROUND ART

Nanomedicines have been applied to the diagnosis and treatment of diseases based on their inherent therapeutic properties, drug delivery capabilities, diagnostic utility, imaging applications, use in vaccines, and compatibility with miniaturized medical devices. In particular, when nanomedicines are used as contrast agents for in vivo disease diagnostics, it is essential to engineer them in a form that is both safe and capable of being excreted from the body without undergoing metabolic degradation.

Meanwhile, polymer-drug conjugates that use high-molecular-weight substances such as polyethylene glycol (PEG), polyglutamate, and hyaluronic acid (HA) as drug carriers are actively being developed and utilized.

Due to their high surface-to-volume ratio, nanoparticles can be engineered to carry a large number of drug molecules per unit mass, thereby serving as effective drug delivery carriers. These nanomedicines can be loaded with and co-deliver multiple types of drugs simultaneously, potentially producing synergistic therapeutic effects, including enhanced anticancer activity.

For example, liposomes and polymeric micelles, which are designed to encapsulate drugs and allow for their slow diffusion at the target site, have already been approved for clinical use in cancer therapy. Another representative example is viral nanomedicines developed for the delivery of small interfering RNA (siRNA). Modified viral nanostructures derived from adeno-associated virus, lentivirus, or plant viruses such as tobacco mosaic virus and potato virus X have attracted considerable attention as effective siRNA carriers.

In the design of nanomedicines or drug delivery carriers, the strategy for delivering drugs to the target disease site is one of the most critical factors for ensuring diagnostic and therapeutic accuracy and efficiency. Most currently developed nanomedicines are known to rely on the enhanced permeability and retention (EPR) effect for tumor targeting. This hypothesis is based on the observation that, compared to normal tissues, tumor tissues grow more rapidly and therefore require accelerated nutrient supply, which results in the formation of relatively leaky vasculature near tumors and impaired lymphatic drainage. These physiological characteristics are believed to allow particles with diameters ranging from several nanometers to several micrometers to more effectively penetrate and accumulate within tumor tissues.

However, according to a 2016 review published in *Nature Reviews Materials,* only approximately 0.7% of the administered dose of nanomedicines actually reaches the tumor via the EPR effect, and the amount that is internalized into tumor cells is reported to be even lower-less than 0.01%. This poor delivery efficiency is attributed to the presence of extracellular matrix (ECM), a dense meshwork of structural biomolecules between cells, which impedes the diffusion and transport of nanoscale materials due to their relatively large size compared to small molecules.

Conventional chemotherapeutic agents primarily exploit differences in the cell cycle to distinguish between normal and cancer cells. These agents are typically administered at or near the maximum tolerated dose (MTD) in order to achieve therapeutic efficacy. Although their intended target is cancer cells, chemotherapeutic agents generally act by killing rapidly dividing cells, regardless of whether they are malignant or normal. As a result, systemic toxicity and cytotoxic side effects are difficult to avoid. Therefore, there is a critical need for strategies that selectively deliver cytotoxic agents to cancer cells, enabling the specific elimination of tumor cells while sparing normal tissues.

Targeted therapies differ from conventional cytotoxic chemotherapeutics in their mechanism of action. While cytotoxic drugs act on DNA or microtubules in rapidly dividing cells and thereby induce significant toxicity in normal tissues, targeted therapies use molecular targets to selectively attack cancer cells. Molecular targets for such therapies include factors involved in angiogenesis, apoptosis, intracellular signaling pathways, and cell cycle regulation. A variety of targeted agents have been developed against these targets. Since most targeted therapies act by inhibiting cellular proliferation rather than directly killing cells, they are typically administered either as long-term monotherapies or in combination with traditional cytotoxic agents.

Monoclonal antibody-based therapeutics have been developed as targeted agents that bind to disease-specific antigens, thereby enhancing therapeutic efficacy while minimizing off-target side effects caused by nonspecific binding.

Monoclonal antibody therapeutics offer several advantages over small-molecule drugs. Due to their high target specificity, they cause fewer off-target side effects by minimizing action on non-target tissues. In addition, their large molecular weight prevents them from crossing the blood-brain barrier, thereby reducing the risk of central nervous system side effects. Furthermore, since monoclonal antibodies are degraded into amino acids and metabolized via the reticuloendothelial system, they do not undergo hepatic metabolism or renal excretion. As a result, they exhibit minimal drug-drug interactions and can be used relatively safely in patients with liver or kidney dysfunction. Their long half-life also allows for infrequent dosing, typically once every few weeks to several months. However, as protein-based drugs with high molecular weight, monoclonal antibodies are readily degraded by proteolytic enzymes in the gastrointestinal tract, rendering them unsuitable for oral administration. Moreover, due to their prolonged half-life, unexpected adverse effects may not be readily reversed through non-invasive means. Unlike small-molecule drugs, monoclonal antibodies may induce the formation of neutralizing antibodies or cause hypersensitivity reactions at the injection site. Additionally, in the later stages of pregnancy, monoclonal antibodies can cross the placenta, potentially affecting fetal and placental development.

Therapeutic monoclonal antibodies exert cytotoxic effects by specifically binding to antigens expressed on the surface of tumor cells. Because they selectively bind to tumor cells, they reduce the risk of nonspecific systemic toxicity. Although therapeutic antibodies offer advantages over chemotherapeutic agents, only a limited number of tumor-specific antibodies are currently used for cancer therapy. This is because many tumor-specific antibodies are not sufficiently effective in killing cancer cells when used alone.

Nanoparticles and liposomes incorporating antibodies for targeted delivery have been reported. Among these, antibody-drug conjugates (ADCs), in which cytotoxic drugs are directly conjugated to antibodies, have demonstrated superior therapeutic efficacy. In such ADCs, the antibody component enables targeted delivery, while the cytotoxic agent-referred to as the payload-possesses extremely high potency. Because of this high cytotoxicity, even a small amount of the payload, when delivered to the target site, can effectively kill cancer cells.

Broadly speaking, antibody-drug conjugate (ADC) technology refers to a method of delivering drugs to tumor cells or senescent cells using antibodies that specifically bind to antigens expressed on the surface of such cells. In most cases, cellular uptake of ADCs occurs via clathrin-coated pit-mediated endocytosis. Once internalized, the ADC dissociates from the clathrin structure and fuses with intracellular vesicles, subsequently entering the endosome-lysosome pathway. Within the acidic environment of the endosome, proteases cleave the linker, releasing the active "free" drug. The released drug then crosses the lysosomal membrane into the cytoplasm, where it binds to its molecular target. This results in cell cycle arrest and ultimately induces apoptosis of the tumor cell. A portion of the released drug may exit the cell through passive diffusion, active transport, or leakage from dead cells. If the released drug is membrane-permeable, it may enter neighboring cells and induce what is referred to as a "bystander cell-killing" effect.

An ADC is composed of three essential components:
(i) an antibody that specifically binds to a target antigen, which is preferably expressed at low levels in normal cells;
(ii) a cytotoxic agent, referred to as the payload, designed to kill the target cancer or senescent cells; and
(iii) a chemical linker that covalently attaches the cytotoxic agent to the antibody.

Linkers currently in clinical and preclinical use are generally categorized as either cleavable or non-cleavable. Representative cleavable linkers include acid-labile linkers, redox-sensitive linkers, and enzyme-labile linkers. Non-cleavable linkers typically include thioether linkers (see Table 1).

**[Table 1]**

| | **Linker type** | **Mechanism of Action** | **Example** |
|---|---|---|---|
| **Cleavable** | **Acid-labile** | **Acidic environment in the cytoplasm** | **Hydrazone, Silyl ether** |
| | **Oxidation-reduction reaction** | **Redox reaction by intracellular glutathione** | **Disulfide** |
| | **Enzyme labile** | **Hydrolyzed by intracellular proteolytic enzymes** | **Peptide, β-glucuronide** |
| | **Non-cleavable** | **Degradation of antibodies** | **Thioether** |

In recently approved third-generation ADCs, improved cytotoxic payloads and novel linkers have been employed.

Immunomedics developed Trodelvy^{®} to target moderately overexpressed antigens, using a less toxic payload compared to conventional agents such as monomethyl auristatin E (MMAE) or mertansine (DM1), and allowing for drug release both intracellularly and extracellularly. Trodelvy, approved by the U.S. FDA in 2020, utilizes an anti-TROP2 monoclonal antibody conjugated via a cleavable maleimide linker incorporating polyethylene glycol (PEG) to SN-38, a topoisomerase I inhibitor, as the cytotoxic payload. Trodelvy was approved for the treatment of relapsed or refractory metastatic triple-negative breast cancer in patients who had received at least two prior therapies-a setting with significant unmet medical need. The incorporation of PEG into the linker enabled a Drug-to-Antibody Ratio (DAR) as high as 7.6 (Goldenberg and Sharkey, 2020).

Daiichi Sankyo developed Enhertu^{®} using a cytotoxic agent known as exatecan (DXd), which exhibits approximately 10-fold higher activity in cancer cells compared to SN-38. DXd is highly soluble, relatively safe, and possesses a strong bystander killing effect, making it particularly advantageous for the treatment of heterogeneous tumors. In Enhertu, DXd is site-specifically conjugated to cysteine residues of an anti-HER2 antibody via a maleimide linker, resulting in a homogeneous DAR of 8. Despite the high DAR, DXd demonstrates excellent plasma stability, with only 2.1% of the payload released over 21 days (Ogitani et al., 2016). Enhertu was approved by the U.S. FDA in 2019 for the treatment of adult patients with unresectable or metastatic HER2-positive breast cancer who had previously received at least two HER2-targeted therapies.

The development of antibody-drug conjugates (ADCs) requires a comprehensive understanding of multiple factors, including the selection of an appropriate target antigen, the internalization of ADCs by tumor or senescent cells, the potency of the cytotoxic drug, and the stability of the linker between the drug and the antibody. In addition, the method of conjugation between the cytotoxic drug and the antibody, the drug-to-antibody ratio (DAR), the physicochemical properties of the antibody, and the influence of linker type on drug binding affinity are all critical for the development of safe and effective ADCs.

Key challenges in ADC development include the complexity and cost of the manufacturing process, the stability of the linker, and the heterogeneity of the DAR profile.

In particular, linker stability is closely associated with clinical toxicity.

One of the major risks associated with ADCs is the premature cleavage of the linker in normal, non-target tissues, which can result in the unintended release of highly cytotoxic agents and severe adverse effects. For example, the first ADC approved in the U.S. market, Mylotarg, was withdrawn in 2010 due to the instability of its linker, which led to the systemic release of the potent cytotoxic drug and associated toxicities. Another source of toxicity arises when ADCs target antigens that are also expressed in normal tissues.

To date, both approved ADCs and those in clinical pipelines are typically manufactured by conjugating small-molecule cytotoxic drugs to lysine or cysteine residues on the antibody using linkers. In conventional processes used for products such as Adcetris, Kadcyla, and the reapproved Mylotarg (2017), the number of drugs conjugated per antibody is only partially controlled. For example, studies on the DAR profile of Kadcyla have shown that, while the average DAR is approximately 4, each monoclonal antibody contains about 80 lysine residues, with roughly 8 to 10 surface-exposed lysines being highly reactive. As a result, the DAR values can range from 0 to 8. To address this issue, several companies have developed conjugation platforms aimed at more precise DAR control, as exemplified by Pfizer's Besponsa (inotuzumab ozogamicin), which was approved in 2017.

However, heterogeneity has been a major issue in ADCs from the outset of their development, including Ado-trastuzumab emtansine (T-DM1, marketed as Kadcyla^{®}). Specifically, low-molecular-weight drugs are randomly conjugated to lysine residues, of which there are approximately 70 to 80 per antibody, resulting in variability in both the drug-to-antibody ratio (DAR) and conjugation sites. ADCs produced via such random conjugation methods typically exhibit DAR values ranging from 0 to 8, and the number of drugs attached to each antibody molecule varies significantly, leading to heterogeneous populations. Recent studies have shown that variations in both the number and position of conjugated drugs can affect the pharmacokinetics, drug release rate, and therapeutic efficacy of ADCs in vivo. Accordingly, next-generation ADCs require precise control over both the number and the conjugation sites of payloads. When both are controlled, anticipated efficacy, product variability, lot-to-lot consistency, and regulatory concerns can be effectively addressed.

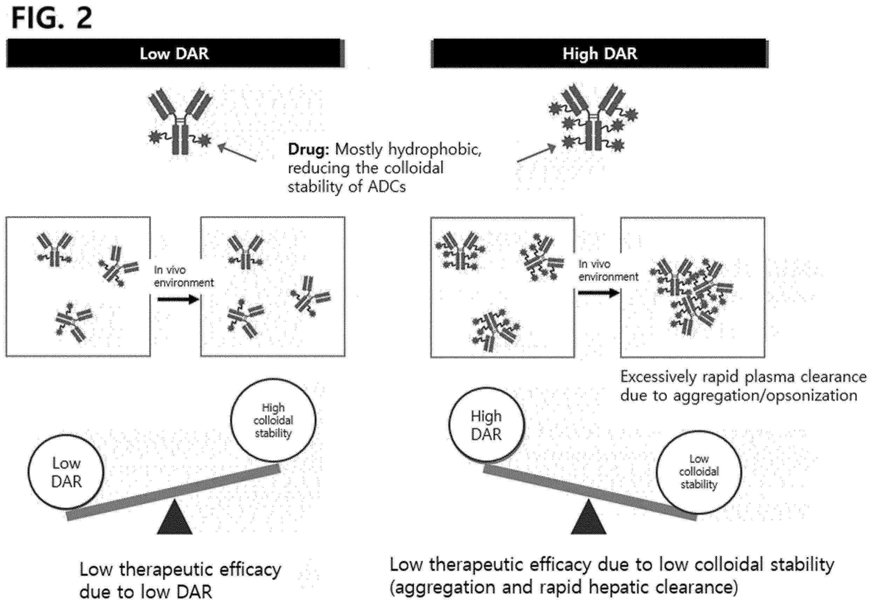

Meanwhile, the DAR is one of the most critical parameters influencing the pharmacokinetics and in vivo distribution of ADCs. ADCs with higher DARs often show greater potency in in vitro assays. However, such ADCs typically demonstrate reduced efficacy in vivo, which is thought to be due to increased plasma clearance associated with a higher number of conjugated drugs (see FIG. 2). Based on these findings, most currently approved and investigational ADCs are engineered to have DARs in the range of 2 to 4, and technologies that conjugate drugs to cysteine or lysine residues are primarily used to achieve this. Although many efforts have been made to develop high-DAR ADCs, most have failed. This is primarily due to the hydrophobic nature of both the cytotoxic payload and the linker, which leads to aggregation, reduced binding affinity for the target antigen, and increased plasma clearance. Recently, a homogeneous ADC with a DAR of 8 was developed via site-specific cysteine conjugation, but this ADC also exhibited increased plasma clearance, likely due to extensive modifications of the antibody and the unique structure of the drug-linker complex.

The concept of an "ideal" ADC is to deliver the maximum amount of cytotoxic drug specifically to target tumor cells. To develop a high-DAR ADC with prolonged plasma retention, a finely tuned balance must be investigated and established between the number of conjugated drug molecules and the extent of structural modification to the antibody.

### DETAILED DESCRIPTION OF THE INVENTION

### TECHNICAL PROBLEM

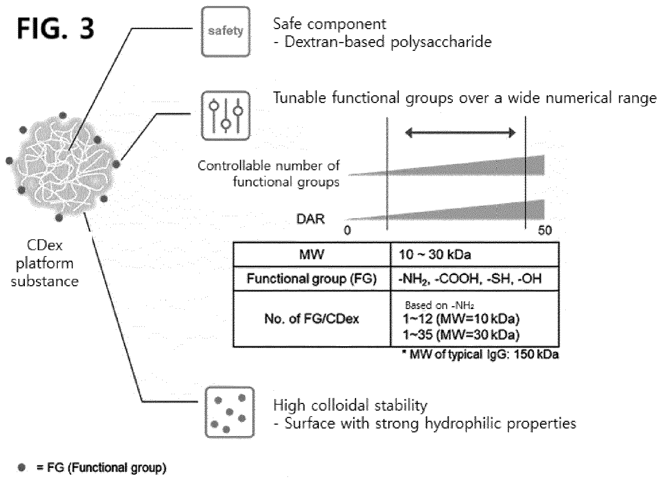

To address the aforementioned limitations of ADCs, the present invention provides a dextran-crosslinked nanoparticle (CDex) that can serve as a multivalent linker system. This nanoparticle is engineered to allow modulation of overall size and surface charge, and, if desired, can achieve a high drug-to-antibody ratio (DAR) of approximately 20 or more. Furthermore, due to the large number of hydrophilic functional groups exposed on the surface, the nanoparticle is highly hydratable and exhibits excellent colloidal stability (see FIG. 3).

In addition, by employing the dextran-crosslinked nanoparticle as a multivalent linker system, the present invention enables the design of various conjugates, such as ADCs, with tailored blood circulation times or desired in vivo distribution and clearance pharmacokinetics. Moreover, the platform can be further optimized to provide enhanced therapeutic efficacy in a patient-specific manner.

### TECHNICAL SOLUTION

According to a first aspect of the present invention, there is provided a conjugate comprising two or more molecules linked via a multivalent linker system comprising a polysaccharide-crosslinked colloidal particle, wherein: the polysaccharide-crosslinked colloidal particle comprises two or more crosslinker-derived surface functional groups thereof; and the polysaccharide-crosslinked colloidal particle is formed by intra- and/or inter-molecular crosslinking of 1 to 3 branched polysaccharides or 2 to 30 cyclic polysaccharides at hydroxyl groups of the monosaccharide building blocks thereof.

According to a second aspect of the present invention, there is provided a polysaccharide-crosslinked colloidal particle-drug conjugate, comprising: a colloidal particle formed by intra- and/or inter-molecular crosslinking of one to three branched polysaccharides or two to thirty cyclic polysaccharides at hydroxyl groups of the monosaccharide building blocks thereof using a crosslinker; and a drug conjugated to the colloidal particle via surface functional groups derived from the crosslinking molecules, wherein the colloidal particle functions as a drug carrier.

In the present specification, the term "polysaccharide crosslinked colloidal particle-drug conjugates" may refer not only to substances that are not conjugated to an antibody, an antibody fragment containing an antigen-binding region thereof, or a molecule exhibiting targeting functionality, as used for the manufacture of immunoconjugates (e.g., antibody-drug conjugates, ADCs) or carrier-drug conjugates, but also to conjugates that are, depending on the intended purpose, bound to any antibody, an antibody fragment containing an antigen-binding region, or a molecule exhibiting targeting functionality (hereinafter, 'targeting molecule'), and thereby usable as an immunoconjugate or a targeted drug delivery carrier.

According to a third aspect of the present invention, there is provided a targeted drug carrier comprising a polysaccharide-crosslinked colloidal particle, wherein: the colloidal particle is configured to serve as a drug carrier and is formed by intra- and/or inter-molecular crosslinking of one to three branched polysaccharides or two to thirty cyclic polysaccharides at hydroxyl groups of their monosaccharide building blocks using a crosslinker; and a targeting molecule is conjugated to the surface of the colloidal particle via a crosslinker-derived functional group.

In the first to third aspects, the polysaccharide-crosslinked colloidal particles may be engineered to have a hydrodynamic diameter of 2 to 20 nm, preferably 10 nm or less, more preferably 8 nm or less, and even more preferably 6 nm or less, and to expose -COOH functional groups on the surface such that the surface charge is between -20 mV and 0 mV, thereby allowing renal clearance while preventing permeation through the vascular walls of normal blood capillaries, and ensuring selective drainage into lymphatic vessels.

The polysaccharide-crosslinked colloidal particles may be in the form of a conjugate in which two or more molecules are linked via a multivalent linker system that comprises, for example, dextran-crosslinked nanoparticles (CDex) having two or more crosslinker-derived functional groups exposed on the surface, wherein the dextran-crosslinked nanoparticles may be formed by intramolecular and/or intermolecular crosslinking of one to three dextran molecules or dextran derivatives at hydroxyl groups of their glucose building blocks using a crosslinker.

The invention is described in detail below.

Through Example 1, a carbohydrate-based polymer linker platform (CDex) was developed to address two key limitations of conventional antibody-drug conjugate (ADC) technologies: the limited drug-to-antibody ratio (DAR) and poor colloidal stability. The feasibility of this platform was successfully demonstrated using an anticancer drug payload. Specifically, for the representative hydrophobic anticancer drug doxorubicin, the maximum DAR achieved was up to 80, indicating a potential to increase the DAR by up to 10-fold compared to existing ADCs. Furthermore, even under high-DAR conditions, the resulting conjugates remained well dispersed without aggregation in physiological saline, thereby validating the potential of the Cdex platform to overcome the colloidal stability issues commonly observed in conventional ADCs.

Based on the results of Example 1, Example 2 was conducted to develop a targeted drug delivery system, which may be considered a form of non-cytotoxic ADC. In this case, Toll-like receptors (TLRs), which are relevant to cancer immunotherapy, were selected as the target. A conjugation chemistry process was established for attaching a TLR agonist to the Cdex platform, and the resulting conjugates demonstrated therapeutic potential in both cellular and animal cancer models. In vitro and in vivo toxicity assessments confirmed the stability and safety of the developed materials.

Furthermore, in Example 3, it was confirmed that the dextran-crosslinked nanoparticles-formed by intramolecular and/or intermolecular crosslinking of 1 to 3 dextran or dextran derivatives via the hydroxyl groups of glucose building blocks using a crosslinker and used as multivalent linker systems or drug carriers in Example 1-can be extended to polysaccharide-crosslinked colloidal particles formed by intramolecular and/or intermolecular crosslinking of 1 to 3 branched polysaccharides or 2 to 30 cyclic polysaccharides via the hydroxyl groups of their monosaccharide building blocks using a crosslinker.

The present invention is based on these findings.

As used herein, the term "drug" refers to any substance used (excluding food or devices) for the diagnosis, cure, mitigation, treatment, or prevention of disease, or to affect the structure or function of the body. For example, it encompasses all chemical or biological substances that influence physiological or metabolic functions of the body.

For a drug to exert its therapeutic effect in vivo, its concentration in the body must remain within the therapeutic range for a certain period of time. Excessive levels of the drug in the body can cause toxicity, while insufficient levels may fail to produce the intended therapeutic effect.

As used herein, "drug efficacy" refers to the retention of the drug in the body without degradation during the expected period of action for the indicated condition. A lower metabolic rate prolongs the maintenance of blood concentration and thus extends the duration of efficacy.

The conjugates of the present invention may be administered or injected, and are not limited by the route or site of administration so long as they are applicable for in vivo use.

The present invention is characterized by the use of polysaccharide-crosslinked colloidal particles-formed by intra- and/or intermolecular crosslinking of one to three branched polysaccharides or two to thirty cyclic polysaccharides at the hydroxyl (-OH) groups of their monosaccharide building blocks-as a multivalent linker system or as a drug carrier.

Accordingly, the present invention enables the design of various conjugates, such as antibody-drug conjugates (ADCs), in which two or more molecules are linked via a multivalent linker system or a drug carrier comprising polysaccharide-crosslinked colloidal particles having two or more crosslinker-derived functional groups exposed on the surface.

Thus, when the polysaccharide-crosslinked colloidal particles of the present invention are used as ADC linkers, they can provide a next-generation linker platform that maximizes ADC efficacy.

The two or more molecules that can be linked via the multivalent linker system may include targeting molecules, such as antibodies, and/or drugs.

The targeting moiety may be selected from the group consisting of an antibody or an antigen-binding fragment thereof, antibody mimetics, repebodies, aptamers, peptides, and small molecules (e.g., poorly soluble drugs, Toll-like receptor (TLR) ligands, agonists, antagonists, enhancers, inhibitors, or blockers).

Specifically, the multivalent linker system or drug carrier of the present invention enables modulation of the pharmacokinetics of a conjugate by controlling the hydrodynamic diameter and/or surface charge of a polysaccharide-crosslinked colloidal particle, such as a dextran-crosslinked nanoparticle. In addition, by adjusting at least one parameter selected from the molecular weight of the branched or cyclic polysaccharide, the length of the polysaccharide backbone, the type of crosslinker used during crosslinking, the amount and administration rate of the crosslinker during synthesis, and the degree of chemical functional group modification, the overall size and overall charge of the polysaccharide-crosslinked colloidal particle can be tailored to impart desired blood circulation time and desired in vivo distribution and excretion pharmacokinetics to the conjugate. Furthermore, the multivalent linker system of the present invention may be engineered to bind to serum albumin, which has a relatively long half-life of approximately 20 days, by modulating the surface charge of the polysaccharide-crosslinked colloidal particle.

For example, the polysaccharide-crosslinked colloidal particles of the present invention may be engineered to have a hydrodynamic diameter of 2 to 20 nm, preferably 10 nm or less, more preferably 8 nm or less, and most preferably 6 nm or less, and a surface charge of -20 mV to 0 mV due to the exposure of -COOH groups on the surface. This configuration allows for renal filtration while preventing penetration through the vascular walls of normal capillaries, thereby facilitating clearance exclusively through the lymphatic system (see Example 4).

For instance, when a branched or cyclic polysaccharide of low molecular weight is used, the resulting colloidal particle is rapidly cleared by the kidney, resulting in a significantly shorter blood half-life. When linked to an antibody, the conjugate exhibits a prolonged half-life until it is targeted to the lesion, after which the polysaccharide-crosslinked colloidal particle is released via linker cleavage and excreted through the lymphatic system to the kidney. In such cases, site-specific cleavage can be achieved by using different linker attachment sites for the antibody and for the drug, thereby enabling controlled release via selective linker degradation.

The multivalent linker system or drug carrier of the present invention is capable of chemically conjugating with molecules having various functional groups via crosslinker-derived functional groups (e.g., -SH, -NH₂, -COOH, -OH, etc.) exposed on the surface of the polysaccharide-crosslinked colloidal particles. For example, it can be conjugated to drugs possessing various reactive functional groups.

Additionally, the multivalent linker system or drug carrier of the present invention allows chemical conjugation with diverse functional groups (e.g., -SH, -NH₂, -COOH, -OH, etc.) of proteins such as antibodies by controlling the types and amounts of functional groups introduced onto the polysaccharide-crosslinked colloidal particles.

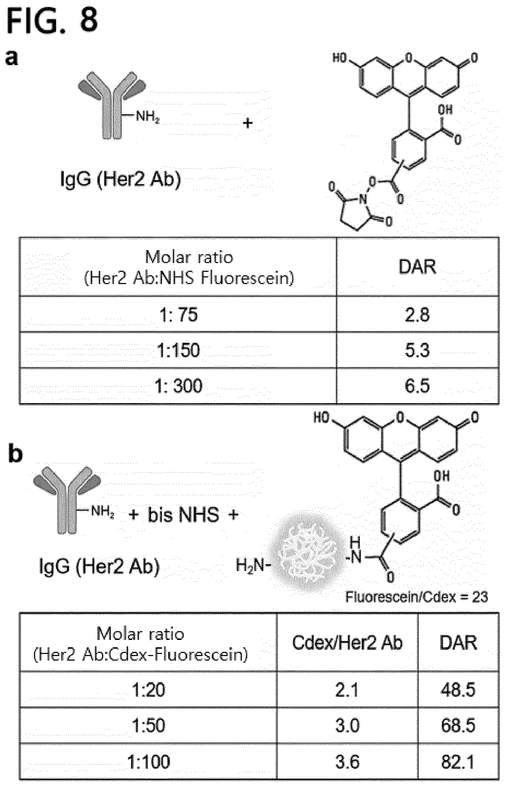

For instance, the average number of polysaccharide-crosslinked colloidal particles conjugated per antibody molecule can be adjusted from 0 to 4 (see FIG. 8b).

The multivalent linker system or drug carrier of the present invention also enables control over the number of drug molecules that can be conjugated by modulating the types and quantities of crosslinker-derived functional groups introduced into the polysaccharide-crosslinked colloidal particles (see FIG. 3).

The number of crosslinker-derived functional groups on the polysaccharide-crosslinked colloidal particles, serving as the multivalent linker system or drug carrier of the present invention, may be adjusted within a range of 0 to 23. Furthermore, the number of drug molecules conjugatable thereto may also be controlled by varying the molecular weight of the branched or cyclic polysaccharides constituting the polysaccharide-crosslinked colloidal particles (see FIG. 3).

Accordingly, the multivalent linker system of the present invention can be used to design and provide conjugates with a drug-to-antibody ratio (DAR) ranging from 8 to 80. That is, the DAR can be improved up to 10-fold compared to the maximum DAR (up to 8) of conventional ADCs.

In one embodiment of the present invention, in polysaccharide crosslinked particle-drug conjugates (PDCs), the drug-to-polysaccharide crosslinked particle ratio (DPR) refers to the average number of drug molecules conjugated to each polysaccharide-crosslinked particle, analogous to the DAR in ADCs. For example, the present invention enables control of the average number of drug molecules conjugated per polysaccharide-crosslinked colloidal particle to a range of 1 to 10, preferably 1 to 8, more preferably 1 to 6, even more preferably 1 to 4, and particularly 1 to 3 in cases where the drug is a hydrophobic small molecule (which may cause aggregation of the PDC complex) or a peptide drug (to modulate the immunogenicity of the PDC complex). A uniform DPR can improve the predictability of the in vivo behavior of the PDCs.

### [Polysaccharide-Crosslinked Colloidal Particles]

Polysaccharides are a class of compounds in which multiple monosaccharides of identical or different structures are linked by glycosidic bonds that are sensitive to enzymatic degradation, and are widely found in the cell walls of animals, plants, and microorganisms.

These polysaccharides may be neutral, positively charged, or negatively charged; they may have linear or branched molecular structures; and they possess a wide range of physicochemical properties, with molecular weights varying from several hundred to several thousand daltons. Due to such properties, polysaccharides significantly affect the biodistribution of carrier drug molecules in vivo.

The polysaccharide-crosslinked colloidal particles of the present invention may be formed by crosslinking one or more branched polysaccharides (e.g., 1 to 3 molecules) or cyclic polysaccharides (e.g., 1 to 30 molecules) using one or more types of crosslinkers in an aqueous solvent.

In this process, the hydroxyl groups (-OH) of the monosaccharide building blocks of the branched or cyclic polysaccharides may be modified with a first crosslinker, and the spatially adjacent modified functional groups may be crosslinked either directly or via a second crosslinker to form intra- and/or intermolecular crosslinks, thereby forming the polysaccharide-crosslinked particles. When an amine-based crosslinker is used as the first and/or second crosslinker, amine groups derived from the crosslinker may be exposed on the surface.

As the molecular weight of the branched or cyclic polysaccharides used in the synthesis increases, the hydrodynamic size of the resulting polysaccharide-crosslinked colloidal particles also increases. Accordingly, the hydrodynamic size of the polysaccharide-crosslinked colloidal particles of the present invention may be controllable within a range of 2 to 20 nm.

The polysaccharide-crosslinked colloidal particles synthesized from various branched or cyclic polysaccharides may be administered in vivo (e.g., via intravenous or local injection). After administration and circulation through the body, the particles may enter the vasculature of the nephron and be excreted in urine through the renal filtration mechanism. This was confirmed by detecting MRI signals in the bladder (see Example 3).

The branched or cyclic polysaccharides to be crosslinked may be homopolysaccharides or heteropolysaccharides.

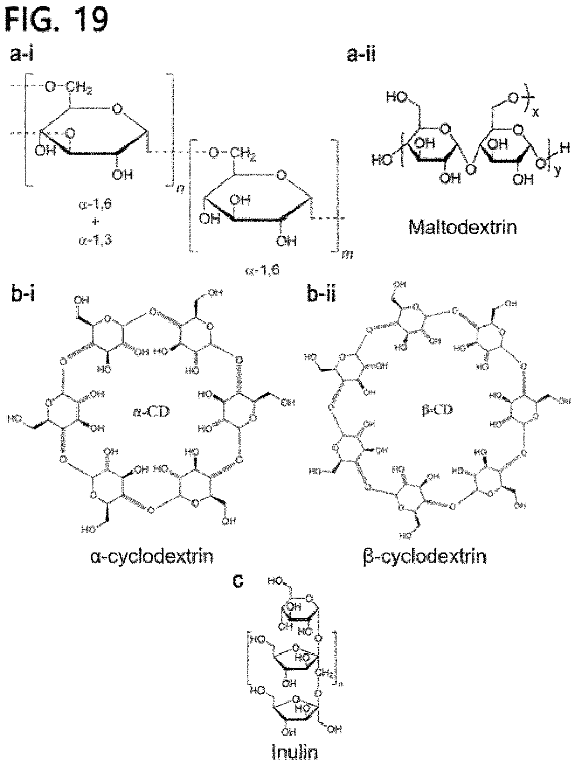

Non-limiting examples of branched or cyclic polysaccharides include, as illustrated in FIG. 19, dextran, cyclodextrin, maltodextrin, and inulin.

With respect to dextran-crosslinked nanoparticle systems, the entire contents of Korean Patent Application No. 10-2021-0104405 are incorporated herein by reference.

Dextran is a polysaccharide derived from the condensation of glucose and is a complex branched glucan, as shown in the following structural formula. It is a microbially derived branched poly-α-D-glucoside predominantly composed of glycosidic bonds at the C-1 → C-6 positions.

The main chain of the polymer is composed of α(1→6)-linked glucose monomers, and the branching points are connected via α(1→3) glycosidic bonds.

Dextran was initially discovered as a microbial product in wine, but its large-scale production became feasible only after processes using bacterial cultures were developed. Dextran is currently synthesized from sucrose by specific lactic acid bacteria belonging to the genus *Lactobacillus.*

Dextran is approved by the U.S. Food and Drug Administration (FDA) as a biocompatible material.

As used herein, the term "dextran" includes various derivatives thereof. Non-limiting examples of dextran derivatives include carboxymethyl dextran (CM-dextran), dextran sulfate, and diethylaminoethyl dextran (DEAE-dextran).

There is an increasing industrial demand for dextran molecules of various specific molecular weights for diverse applications. For example, dextran with a molecular weight ranging from 70,000 to 100,000 Da is used as a plasma substitute. Dextran of approximately 40,000 Da is most likely used to improve blood circulation by reducing blood viscosity and inhibiting erythrocyte aggregation. Smaller dextran sulfates, such as those around 10,000 Da, are used as iron transport agents or anticoagulants.

Inulin is an energy-storage polysaccharide found in plants of the *Compositae* family and consists of a linear chain of fructosyl groups linked by β-2,1 glycosidic bonds and terminated by an α-D-1,2 glucopyranoside ring at the reducing end. Inulin is used as an excipient in pharmaceutical formulations under the GRAS regulatory classification, and its water-soluble form is utilized to test renal glomerular filtration. A specific semicrystalline particulate form known as delta inulin exhibits immunomodulatory properties and has been used as a vaccine adjuvant. Although inulin is not digestible by humans, it can be metabolized by bifidobacteria in the gut, providing not only a probiotic effect but also additional benefits such as colon-targeted drug delivery. Modifications of inulin for medical use typically involve periodate oxidation followed by functionalization via amination reactions or reactions with anhydrides.

According to the present invention, the polysaccharide-crosslinked colloidal particles formed by intramolecular and/or intermolecular crosslinking of 1 to 3 branched polysaccharides or 2 to 30 cyclic polysaccharides via hydroxyl groups of their monosaccharide building blocks using a crosslinker can be synthetically tuned to exhibit diverse physicochemical properties, including size, surface charge, and morphology.

The polysaccharide-crosslinked colloidal particles of the present invention are formed by intramolecular and/or intermolecular crosslinking of 1 to 3 complex branched polysaccharides or 2 to 30 cyclic polysaccharides in an aqueous medium via the hydroxyl groups of their monosaccharide building blocks using a crosslinker, thereby enabling various improvements over conventional polymer coatings widely used for nanomaterials and pharmaceutical agents.

According to the present invention, polysaccharide-crosslinked colloidal particles compactly formed via intramolecular and/or intermolecular crosslinking of complex branched or cyclic polysaccharides in an aqueous solution at the hydroxyl groups of their monosaccharide building blocks can be readily controlled to exhibit desired hydrodynamic size and swelling degree. As such, they allow predictable modulation of mobility within biological fluids and can be engineered to be squeezable for transport within various anatomical structures. For example, in the polysaccharide-crosslinked colloidal particles of the present invention, the degree of substitution of the crosslinker may be controlled to 10% to 40% of the available functional groups on the polysaccharide, and 2% to 98% of the crosslinker molecules may be configured such that one terminal group does not participate in crosslinking and remains exposed on the outer surface. Through such control, the swelling behavior and compressibility of the colloidal particles can be precisely tuned as desired.

Additionally, by adjusting at least one of the following parameters-namely, the molecular weight of the polysaccharide, the length of the polysaccharide backbone, the type of crosslinker used during crosslinking, the amount and rate of crosslinker administration during synthesis, and the type and degree of subsequent functional group modification-it is possible to finely control the overall size and surface charge of the resulting polysaccharide-crosslinked colloidal particles and/or the in vivo administrable complexes thereof, thereby imparting desired blood circulation time, biodistribution, and excretion pharmacokinetics.

For renal excretion, the average molecular weight of the dextran or dextran derivatives used may be 10,000 Da or less, and the molecular weight of spherical dextran-crosslinked nanoparticles formed by crosslinking dextran-based molecules through a crosslinker may be 35,000 Da or less.

The polysaccharide-crosslinked colloidal particles of the present invention are nanoparticles in which complex branched polysaccharides are intramolecularly and/or intermolecularly crosslinked at the -OH functional groups of their monosaccharide building blocks in an aqueous phase. The branched polysaccharides may form dimers or trimers through intermolecular crosslinking, or a single branched polysaccharide molecule may be intramolecularly crosslinked via a crosslinker to form the colloidal particle. The degree of crosslinking and/or the molecular weight of the branched polysaccharide can be adjusted to control the compressibility, and preferably, the particles may be compact and spherical.

In particular, polymer solutions increase in viscosity with increasing concentration, and this also applies to aqueous solutions containing polysaccharide-crosslinked colloidal particles. According to the Mark-Houwink-Sakurada (MHS) equation, which relates the viscosity of a polymer solution to the molecular weight of the polymer, it is well known that viscosity is directly proportional to molecular weight. Excessive viscosity in injectable formulations may increase the risk of vascular occlusion or vascular damage due to high injection pressure. Therefore, for drug formulations utilizing the dextran-crosslinked nanoparticles of the present invention as multivalent linker systems or drug carriers, it is preferable to use dextran-based molecules with an average molecular weight of 10,000 Da or less, more preferably around 5,000 Da or less. When such low-molecular-weight dextran molecules are crosslinked to form spherical dextran-crosslinked nanoparticles with a molecular weight of approximately 15,000 Da or less, an injectable formulation with appropriate viscosity for in vivo administration can be achieved.

For example, when the average molecular weight of the branched or cyclic polysaccharides is adjusted to be relatively low, the resulting drug conjugates incorporating the polysaccharide-crosslinked colloidal particles of the present invention as a multivalent linker system or drug carrier can be prepared at high concentrations. This allows precise control of the formulation's viscosity by adjusting the degree of dilution and enables a reduction in the injection volume relative to the desired drug dose.

For instance, when dextran-based molecules with an average molecular weight of 10,000 Da or less are crosslinked in an aqueous phase at the -OH groups of glucose building blocks, 1 to 3 dextran-based molecules are crosslinked intramolecularly and/or intermolecularly to form spherical nanoparticles. Accordingly, dextran-crosslinked nanoparticles prepared from dextran-based molecules with an average molecular weight of 10,000 Da or less may have a molecular weight of 35,000 Da or less. The in vivo administrable complex modified therewith may be engineered as a nanostructure having a hydrodynamic diameter of 10 nm or less, preferably 5 nm or less, for renal excretion. Generally, a nanostructure must have a hydrodynamic diameter of 6 to 8 nm or less in order to be naturally excreted through the kidney.

Accordingly, by diversifying the synthesis method of conjugates in which polysaccharide-crosslinked colloidal particles-including dextran-crosslinked nanoparticles-are used as multivalent linker systems or drug carriers, it is possible not only to control the biodistribution and excretion of target substances such as functional nanoparticles or drugs without eliciting immune rejection or toxicity, but also to ensure in vivo stability without aggregation in plasma, even when the conjugated polysaccharide-crosslinked colloidal particles of the present invention are discharged from the body. In certain cases, after in vivo administration, polysaccharide-crosslinked colloidal particles released from the conjugates of the present invention via a cleavable portion introduced into the crosslinker-derived functional groups exposed on the particle surface may remain metabolically or enzymatically undegraded in vivo, such that they can be collected via urine or feces and reused. For example, the polysaccharide-crosslinked colloidal particles separated from the conjugates of the present invention after in vivo administration may be engineered to be absorbed into the circulatory system and excreted through the kidneys into urine without extravasation through the vascular wall. In some instances, the drug component may be released from the polysaccharide-crosslinked colloidal particle in vivo, and the separated components may be discharged via different physiological routes.

The polysaccharide-crosslinked colloidal particles of the present invention may be engineered to be filtered by the kidneys but not to penetrate the vascular walls of normal capillaries, thereby enabling drainage exclusively through lymphatic vessels.

The normal capillaries that the polysaccharide-crosslinked colloidal particles of the present invention are unable to penetrate are characterized by a continuous endothelium having tight junctions and a basal lamina. Accordingly, although the particles are engineered for renal filtration, they may also be filtered through the capillaries of the liver and spleen.

The polysaccharide-crosslinked colloidal particles of the present invention, when used as a multivalent linker system or drug carrier, may be designed with a hydrodynamic diameter in the range of 2 to 10 nm, preferably 8 nm or less, more preferably 6 nm or less, and with a surface zeta potential in the range of -20 mV to 0 mV due to the presence of exposed carboxyl (-COOH) functional groups, such that they are filtered by the kidneys but do not extravasate across the walls of normal capillaries, and are thereby drained exclusively into lymphatic vessels.

When locally administered, the polysaccharide-crosslinked colloidal particles of the present invention-serving as a multivalent linker system or drug carrier-may extend the residence time at the administration site, thereby inducing interstitial space enhancement, anatomical space distension, and/or lymph node enlargement. Furthermore, the prolonged retention at the target site may enhance the pharmacological efficacy of the drug (see Examples 2 and 4).

When the polysaccharide-crosslinked colloidal particles of the present invention are designed to have a hydrodynamic diameter of 10 nm or less, preferably 8 nm or less, more preferably 6 nm or less, and a surface zeta potential in the range of -20 mV to 0 mV, they may circulate systemically and subsequently be excreted via the kidneys into urine. As a result, more than 90% of the administered dose may be excreted within 48 hours, preferably within 24 hours.

The polysaccharide-crosslinked colloidal particles of the present invention are engineered to be readily excreted in urine, thereby avoiding hepatic metabolism and exhibiting no hepatotoxicity.

For example, when the hydrodynamic diameter of the polysaccharide-crosslinked colloidal particles and/or the polysaccharide crosslinked particle-drug conjugates (PDCs) is in the range of 2 nm to 10 nm, preferably 8 nm or less, more preferably 6 nm or less, and when their surface charge is adjusted to fall within a range of -20 mV to 0 mV, the particles are filtered by the kidneys but are unable to penetrate the vascular walls of normal capillaries, and are instead drained exclusively into the lymphatic vessels. Accordingly, when administered locally, these properties facilitate urinary excretion and help mitigate side effects arising from long-term retention or accumulation (see Examples 3 and 4).

For instance, the final size and charge of the polysaccharide-crosslinked colloidal particles may be controlled by adjusting at least one of the following: the molecular weight of the polysaccharide such as dextran or a dextran derivative (e.g., carboxymethyl dextran), the length of the polysaccharide backbone, the type of crosslinker used during synthesis, the amount and rate of crosslinker added during the reaction, the introduction of additional functional groups, and the molecular weight and surface charge of the resulting colloidal particles. Through such control, the particles can be engineered either to evade phagocytosis by macrophages or to be preferentially taken up by them, and to be excreted via urine or feces through renal or hepatic clearance mechanisms. Desired blood circulation time and tailored biodistribution and excretion pharmacokinetics may also be imparted.

In addition, the present invention enables control over the degree of swelling (hydrogelation) of the polysaccharide-crosslinked colloidal particles by modulating the degree of crosslinking, thereby allowing regulation of the pharmacokinetics of the PDCs.

In the present invention, the crosslinker-derived functional groups exposed on the surface of the polysaccharide-crosslinked colloidal particles may be either the terminal functional groups of the crosslinkers themselves or functional groups that have been modified or substituted therefrom. For example, the crosslinker-derived functional groups exposed on the surface of the polysaccharide-crosslinked colloidal particles may include those in which at least a portion of the exposed functional groups of the crosslinkers have been substituted with different functional groups.

In the present invention, the hydrophilic functional groups may originate from unreacted functional groups of the branched or cyclic polysaccharides, terminal functional groups of the crosslinkers not involved in the crosslinking reaction, and/or functional groups introduced by further modification of the exposed terminal portion of the crosslinker after the crosslinking reaction.

Non-limiting examples of the crosslinker-derived or hydrophilic functional groups exposed on the surface of the polysaccharide-crosslinked colloidal particles include amine, carboxyl, hydroxyl, and/or thiol groups. Such reactive groups-such as amine, thiol, carboxyl, and hydroxyl-facilitate not only surface modification but also chemical conjugation with various biologically active substances, including ligands that specifically bind to receptors on target cells, antibodies or antigen-binding fragments thereof, antigenic peptides, nucleic acids (e.g., DNA, RNA, or fragments thereof), and various small-molecule drugs. In some embodiments, the crosslinker-derived functional groups may be engineered to incorporate cleavable moieties that enable the release of active drugs in response to acidic tumor microenvironments (e.g., pH ≤ 7) or hydrolytic enzymes.

For example, non-limiting examples of acid-sensitive linkages that are degradable under acidic conditions include carbonate and ester bonds.

In certain embodiments, the polysaccharide-crosslinked colloidal particle-drug conjugates, in which the drug payload is conjugated via a cleavable linker, may employ peptide linkers that are susceptible to enzymatic cleavage by (i) extracellular matrix (ECM)-degrading enzymes such as matrix metalloproteinases (MMPs), which are secreted by cancer cells during local invasion and metastasis, or (ii) intracellular hydrolases involved in the cleavage of peptide bonds.

In the present invention, when the functional groups exposed on the surface of the polysaccharide-crosslinked colloidal particles carry a positive charge, such as amines, they may induce cytotoxicity similarly to other cationic polymers. This issue can be addressed by substituting some or all of the amine groups with neutral or negatively charged groups such as carboxyl, methyl, or ethyl groups.

Non-limiting examples of hydrophilic functional groups that form coordination bonds with metal ions (e.g., iron ions) include amine, thiol, carboxyl (carboxylate and carboxylic acid), and hydroxyl groups.

Terminal hydroxyl, amine, and non-coordinating carboxylate groups of the polysaccharide-crosslinked colloidal particles confer water solubility to the resulting conjugates. Therefore, hydration of hydrophilic groups exposed on the surface of the particles enhances the dispersion stability of the conjugates in biological fluids. As a result, the conjugates modified with polysaccharide-crosslinked colloidal particles can remain stably dispersed without precipitation or aggregation even when conjugated with a large number of drug molecules, thereby preserving their intended function in vivo.

Furthermore, the surface charge of the polysaccharide-crosslinked colloidal particles, and consequently that of the conjugates (e.g., antibody-drug conjugates or small-molecule drug conjugates), can be freely tuned by adjusting the type and/or degree of substitution of the crosslinker-derived functional groups exposed on the surface.

Moreover, by modulating the molecular weight, size, and surface charge of the polysaccharide-crosslinked colloidal particles, the overall size and surface charge of the conjugates using these particles as multivalent linker systems or drug carriers can be tailored to achieve desired pharmacokinetic profiles, including blood circulation time, renal clearance, and hepatic clearance.

According to the present invention, the polysaccharide-crosslinked colloidal particles-produced through crosslinking reactions using crosslinkers and, optionally, further modified at one terminal of the exposed crosslinker after the crosslinking reaction-may be conjugated, via functional groups exposed on their surface, to targeting molecules and/or drugs through coordination bonding, covalent bonding, hydrogen bonding, and/or electrostatic interactions. In particular, the fact that the crosslinker-derived functional groups exposed on the surface of the polysaccharide-crosslinked colloidal particles are capable of forming coordination bonds with metals enables facile surface modification of various functional metal nanoparticles that are otherwise difficult to functionalize, thereby imparting new physicochemical properties. For example, divalent or trivalent iron ions can coordinate with carboxylic acid or carboxylate groups derived from crosslinkers on the surface of the polysaccharide-crosslinked colloidal particles. Accordingly, the polysaccharide-crosslinked colloidal particles of the present invention can be surface-modified via covalent bonding, such as coordination bonding, with metal particles or metal oxide particles (e.g., iron oxide nanoparticles), regardless of their synthetic origin, enabling their use as diagnostic agents such as contrast agents.

The polysaccharide-crosslinked colloidal particles of the present invention, and/or conjugates using these particles as multivalent linker systems or drug carriers, are water-dispersible. Due to hydration of the hydrophilic functional groups exposed on the surface of the particles, such conjugates exhibit or acquire enhanced colloidal stability in biological fluids.

Accordingly, the present invention can address problems such as aggregation of drugs (e.g., poorly water-soluble drugs or iron oxide nanoparticles) caused by low colloidal stability under physiological conditions (e.g., salt concentration, pH), which can otherwise lead to unpredictable pharmacokinetics and undesirable tissue deposition.

### [Polysaccharide Crosslinked Colloidal Particle and Method for Preparing the Same]

According to the present invention, the multivalent linker system or drug carrier used in a conjugate comprising two or more molecules includes polysaccharide-crosslinked colloidal particles formed by intra- and/or intermolecular crosslinking of one to three branched polysaccharides or two to thirty cyclic polysaccharides in an aqueous solution via a crosslinker at the hydroxyl (-OH) groups of the monosaccharide building blocks.

Meanwhile, the polysaccharide-crosslinked colloidal particles of the present invention, and conjugates using the same as multivalent linker systems or drug carriers, may be provided by a method comprising, as a non-limiting example, the following steps, and may be synthesized by expanding the dextran-crosslinked nanoparticle (CDex) platform to incorporate branched or cyclic polysaccharides in place of dextran or dextran derivatives (see Example 3):
Step 1 of preparing an aqueous solution of dextran or a dextran derivative;
Step 2 of dropwise adding an epoxide and an alkaline aqueous solution at room temperature;
Step 3 of dropwise adding divalent or higher-valent polyamines at room temperature to form dextran-crosslinked nanoparticles bearing terminal amine groups;
Step 4 of precipitating the resulting product;
Step 5 of redispersing the precipitated product in water;
optionally, Step 6 of recovering the dextran-crosslinked nanoparticles bearing terminal amine groups by dialysis;
Step 7 of adding an organic acid anhydride to the dextran-crosslinked nanoparticles bearing terminal amine groups, so as to substitute at least a portion or all of the terminal amine groups with carboxylic acid and/or carboxylate groups;
optionally, Step 8 of purifying a solution of dextran-crosslinked nanoparticles bearing carboxylic acid and/or carboxylate groups to obtain water-dispersible dextran-crosslinked nanoparticles;
optionally, Step 9 of adding an iron precursor (e.g., iron chloride) or an aqueous solution of iron oxide nanoparticles to the water-dispersible dextran-crosslinked nanoparticles prepared in the previous step, to obtain (i) dextran-crosslinked nanoparticles having a shell formed of divalent or trivalent iron ions, or (ii) complexes in which the surface of iron oxide nanoparticles is modified with the dextran-crosslinked nanoparticles; and
optionally, Step 10 of purifying or concentrating the nanostructures obtained in the previous step by ultrafiltration.

The number of dextran molecules subjected to crosslinking in the dextran-crosslinked nanoparticles can be controlled to be the same or different, depending on the synthesis conditions and/or purification process.

In Step 2, the epoxide used to modify the hydroxyl groups of the glucose building blocks is not particularly limited as long as it enables subsequent reaction with a crosslinker. Preferably, the epoxide is a halo alkyl oxirane, such as epichlorohydrin.

In Step 3, the polyamine may be replaced with any crosslinker capable of forming a covalent bond with the epoxide-derived functional groups that have modified the hydroxyl groups of the glucose building blocks. Such variants also fall within the scope of the present invention.

Steps 2 and 3 are economically advantageous because the crosslinking reaction at the hydroxyl groups of the glucose building blocks proceeds without requiring any additional expensive catalyst.

In Step 4, a large amount of an organic solvent having a dielectric constant between 15 and 50 may be added to induce precipitation of the product.

According to the present invention, a conjugate comprising two or more molecules via a multivalent linker system or drug carrier that includes polysaccharide-crosslinked colloidal particles is regarded, once administered in vivo, as a type of drug that should be eliminated from the body. Upon entry into the body, the elimination process for the drug begins. The primary elimination pathways are (1) hepatic metabolism, (2) biliary excretion, and (3) urinary excretion. Drug elimination involves both biotransformation (drug metabolism) and excretion. Excretion refers to the removal of the intact drug from the body. A high proportion of nanoparticles remaining in organs such as the liver and spleen indicates poor excretory performance.

For example, the polysaccharide-crosslinked colloidal particles of the present invention can be designed and synthesized to be primarily collected through urinary excretion without accumulation in normal tissues following in vivo administration. As such, they can be rapidly eliminated via the urinary route without undergoing hepatic metabolism or biliary excretion. Alternatively, when desired, they may be designed and synthesized to avoid urinary excretion and instead follow hepatic metabolism and biliary elimination.

Accordingly, the conjugate comprising two or more molecules via the multivalent linker system or drug carrier including the polysaccharide-crosslinked colloidal particles of the present invention (i) remains stable in physiologic pH-buffered solutions and plasma without aggregation, (ii) can be surface charge-neutralized (i.e., adjusted toward zero) to avoid phagocytic uptake by macrophages, and (iii) can be precisely designed and synthesized to have a compact hydrodynamic diameter smaller than the renal filtration threshold, thereby enabling elimination through the urinary excretion pathway. Thus, the polysaccharide-crosslinked colloidal particles of the present invention, and the conjugates incorporating the same when applicable, can be collected via urinary excretion in an amount of 80% or more, preferably 90% or more, and more preferably 95% or more, relative to the administered dose.

In Step 9, non-limiting examples of hydrophilic functional groups that can coordinate with iron ions include hydroxyl, carboxylic acid, carboxylate, and amine groups.

In addition, all disclosures of Korean Patent Application No. 10-2021-0104405 are hereby incorporated into the present invention and this specification in their entirety, particularly with respect to dextran-crosslinked nanoparticles having a shell composed of divalent or trivalent iron ions.

The polysaccharide-crosslinked colloidal particles having a shell composed of divalent or trivalent iron ions, as engineered through Step 9, may be designed and synthesized to function as T1 MRI contrast agents that generate bright signals in MRI imaging. Accordingly, after administration in vivo, the position of the conjugates modified with polysaccharide-crosslinked colloidal particles may be tracked by MRI imaging.

The polysaccharide-crosslinked colloidal particles of the present invention may be engineered and synthesized to prolong the retention of contrast enhancement during MRI, thereby enabling longer scan times and enhancing the spatial resolution of MRI. This feature allows high-resolution imaging of the entire vascular system in vivo. Accordingly, the polysaccharide-crosslinked colloidal particles of the present invention are capable of visualizing microvasculature that is clinically significant but difficult to observe using conventional contrast agents. For example, when imaging the rat brain using dextran-crosslinked nanoparticles at a spatial resolution of 0.078 mm × 0.078 mm × 0.078 mm, microvasculature with a thickness of approximately 0.078 mm could be clearly visualized. Given that the spatial resolution of the most commonly used 3 Tesla (3T) MRI scanners in clinical settings is approximately 1 mm, the resolution achieved using the dextran-crosslinked nanoparticles of the present invention represents an approximately 13-fold improvement.

In summary, the polysaccharide-crosslinked colloidal particles of the present invention may serve as T1 or T2 MRI contrast agents when divalent or trivalent iron ions are coordinated to the crosslinker-derived functional groups exposed on the particle surface.

In addition, iron oxide nanoparticles engineered to function as T1 or T2 MRI contrast agents may be surface-modified with dextran-crosslinked nanoparticles in Step 9. This modification renders the nanoparticles squeezable, enabling them to move within in vivo structures without aggregation upon in vivo administration, thereby maintaining their functionality as T1 or T2 MRI contrast agents.

Therefore, the conjugates modified with polysaccharide-crosslinked colloidal particles according to the present invention can serve as MRI contrast agents. After administration in vivo, their location can be monitored by MRI imaging to assess: phagocytosis by macrophages, metabolic degradation, circulation in the bloodstream, delivery to parenchymal cells via blood capillaries, tissue accumulation, renal excretion into urine, excretion into feces, absorption into the vascular system, leakage through vessel walls, potential for recovery and reuse via excretion, and intracellular uptake.

### [Drug-to-Antibody Ratio (DAR)]

The drug-to-antibody ratio (DAR) is a critical parameter in the development of antibody-drug conjugates (ADCs), as it determines the pharmacokinetic properties and in vivo distribution of the conjugate.

Although ADCs with high DARs generally exhibit strong efficacy in in vitro assays, such ADCs often show reduced efficacy in vivo. This reduction is believed to result from increased plasma clearance associated with the higher number of conjugated drug molecules. Accordingly, DAR values for ADC formulations have typically been maintained within the range of 2 to 4. Hydrophobicity also presents a challenge. Many commonly used cytotoxic drugs and linkers are hydrophobic, which can cause aggregation of the ADC, reduce its affinity for the target antigen, and increase plasma clearance. Hydrophilic linkers containing sulfonate or polyethylene glycol (PEG) groups have been employed to mitigate these issues. PEG-based linkers are water-soluble, exhibit low toxicity, and possess minimal immunogenicity.

Accordingly, the present invention provides polysaccharide-crosslinked colloidal particles, such as dextran-crosslinked nanoparticles (CDex), which can serve as multivalent linker systems or drug carriers. These particles are characterized by their tunable overall size and charge, and can achieve high DARs (e.g., 20 or more) while maintaining high colloidal stability due to extensive hydration of hydrophilic functional groups exposed on the surface (see FIG. 3).

Furthermore, by employing the aforementioned polysaccharide-crosslinked colloidal particles as multivalent linker systems or drug carriers, the present invention enables diverse structural designs of conjugates such as ADCs to achieve tailored blood circulation times and desired in vivo distribution and clearance pharmacokinetics. The system may also be engineered to exhibit enhanced therapeutic efficacy in a patient-specific manner.

In summary, according to the present invention, the polysaccharide-crosslinked colloidal particles having two or more crosslinker-derived surface functional groups can serve as multifunctional linkers capable of achieving a high drug-to-antibody ratio (DAR), such as a DAR of approximately 20. For example, the multifunctional linkers of the present invention may combine the targeting ability of a monoclonal antibody with the potent cytotoxicity of a cytotoxic drug, thereby enabling the simultaneous and specific delivery of multiple or diverse drugs to target cells or surrounding tissues. This may be accomplished by conjugation to antibodies or antigen-binding fragments thereof, mimetic antibodies, repebodies, aptamers, peptides, or small molecules (e.g., TLR ligands, agonists, antagonists, enhancers, inhibitors, or blockers) that specifically bind to antigens expressed on the surface of cells to be eliminated via apoptosis (e.g., cancer cells or senescent cells). Although conventional ADCs typically require internalization into target cells to exert their therapeutic effects, in some embodiments wherein the polysaccharide-crosslinked colloidal particles of the present invention are used as ADC linkers, the resulting conjugates may function without necessitating internalization into the target cells.

### [Polysaccharide Crosslinked Particle-Drug Conjugates (PDCs)]

In an antibody-drug conjugate (ADC), the linker serves as a bridge between the antibody and the drug. When the ADC circulates through the bloodstream, the linker prevents premature separation of the drug from the antibody, thereby avoiding off-target effects on normal tissues. The linker functions to maintain the drug in a form analogous to a prodrug. In addition, the linker must position the drug and the antibody at an appropriate distance to avoid steric hindrance that could interfere with the antibody-antigen binding. When the polysaccharide-crosslinked colloidal particles of the present invention are employed as a multivalent linker system or drug carrier, they not only fulfill these functional requirements of ADC linkers but also allow tunable control over such parameters.

As exemplified in Example 1-7, the present invention enables the preparation of polysaccharide crosslinked particle-drug conjugates that meet various pharmacokinetic conditions and drug-to-antibody ratios (DAR). These conjugates may subsequently be reacted with targeting molecules such as antibodies, antigen-binding fragments thereof, mimetic antibodies, repebodies, aptamers, peptides, or small molecules by modifying the functional groups exposed on the surface of the polysaccharide-crosslinked colloidal particles (e.g., CDex) with additional reactive moieties.

According to the present invention, when such polysaccharide crosslinked particle-drug conjugates-satisfying various pharmacokinetic parameters and DAR values-are linked to targeting molecules such as antibodies, they can be internalized into target cells via a process known as receptor-mediated endocytosis after binding to the target cells.

At this stage, a sufficient intracellular concentration of the active drug must be achieved. However, the internalization process mediated by antigen-antibody complexes is typically inefficient, and the number of antigens expressed on the cell surface is generally limited to less than 1 × 10⁵ receptors per cell. Therefore, it is necessary to employ highly potent cytotoxic drugs that can induce sufficient cell killing even at low intracellular concentrations. For this reason, drugs used in ADCs are generally 100 to 1000 times more cytotoxic than conventional anticancer agents. The potency of the cytotoxic payload in an ADC is typically 100 to 1000 times greater than that of the drug when used in its free form. The goal of ADC development is to engineer drugs that exert highly specific effects on target cancer cells while minimizing severe side effects on normal tissues.

Furthermore, the multivalent linker system designed for enhanced colloidal stability according to the present invention can enable the design and provision of conjugates having a drug-to-antibody ratio (DAR) in the range of 8 to 80, even when using hydrophobic drugs, while mitigating problems such as aggregation of ADCs or increased plasma clearance. Since this allows for a DAR up to 80-representing up to a 10-fold improvement over the maximum DAR (~8) achievable with conventional ADCs-the range of drug candidates that can be selected is significantly broadened.

In addition, the polysaccharide crosslinked colloidal particle-drug conjugates of the present invention utilize polysaccharide-crosslinked colloidal particles-formed by intra- and/or intermolecular crosslinking of one to three branched polysaccharides or two to thirty cyclic polysaccharides via the -OH groups of their monosaccharide building blocks using a crosslinker-as drug carriers, wherein the drug is conjugated through crosslinker-derived functional groups exposed on the surface of the polysaccharide-crosslinked colloidal particles.

The drug conjugated to the polysaccharide-crosslinked colloidal particles may be a non-cytotoxic immunomodulatory drug. A non-limiting example of such an immunomodulatory drug is a Toll-like receptor (TLR) agonist.

The mechanism by which TLR agonists induce cancer cell death is described in detail in the literature (Remaut et al., Eur. J. Pharm. Biopharm, 2022, 172, 16*).*

TLR agonists are relatively safe therapeutic agents that, upon binding to Toll-like receptors (TLRs) expressed on the surface of immune cells and activating the associated signaling pathways, can induce immune-mediated cytotoxicity against cancer cells.

However, when administered systemically, TLR agonists have a high likelihood of non-specific activation of various immune cells, thereby posing a risk of immune overactivation and associated adverse effects. As a result, TLR agonists are currently limited to topical applications or are used in small amounts as immunological adjuvants in vaccines.

With regard to the potential use of TLR agonists as payloads in ADCs, although TLR agonists exhibit the aforementioned limitations when administered alone, they may enable localized immune activation near the tumor site when conjugated to an antibody-drug conjugate (ADC). Furthermore, if a technology is implemented that enables unbound or non-target-delivered TLR agonists to be eliminated from the body via urine or other excretion pathways after administration, the risk of systemic side effects can be further minimized.

Conventional ADCs are typically not capable of renal clearance, resulting in limited urinary excretion and the hepatic metabolism of the majority (~97%) of administered ADCs, which contributes to hepatotoxicity (P.B. Joseph et al., Cancers 2023, 15, 713). Accordingly, even when TLR agonists are used, the risk of hepatotoxicity inherent to conventional ADC platforms may still persist.

In Example 1, the provided ADC is in the form of an Antibody-CDex-Drug conjugate; however, issues such as the overall instability of the conjugate and hepatotoxicity remain to be addressed. In addition, the delivery efficiency of ADCs to the target site remains low, with only approximately 0.1% of the administered dose reaching the intended site (P.B. Joseph et al., Cancers 2023, 15, 713). This is even lower than the delivery efficiency (~0.6%) attributed to the enhanced permeability and retention (EPR) effect observed with nanoparticles (Warren C. W. Chan et al., Nature Reviews Materials 2016, 1, 1). According to the EPR effect, substances administered intravenously must be extremely small in size to extravasate through blood vessels; larger materials, such as nanoparticles, cannot typically pass through intact vascular endothelium. However, the neovasculature surrounding tumor cells is composed of loosely arranged endothelial cells, allowing nanoscale materials to pass through and accumulate in tumor tissues.

Accordingly, in Example 2, a next-generation platform was developed to overcome the limitations of conventional ADCs by designing a conjugate of CDex and a TLR agonist-classified as a type of polysaccharide-crosslinked colloidal particle-drug conjugate-capable of delivering drugs to the target site without the need for an antibody, for example, via localized administration methods. In this context, CDex, an exemplary polysaccharide-crosslinked colloidal particle, has a particle size of 7 nm or less, such that undelivered conjugates can be eliminated via renal excretion, thereby minimizing systemic toxicity.

In Example 4, regarding localized administration, it was confirmed through shoulder MR arthrography that particulate contrast agents with a hydrodynamic diameter of 2 nm or greater are eliminated from the joint cavity only via lymphatic drainage due to their size and are not cleared through venous routes. Furthermore, because NEMO-103 has a larger hydrodynamic size (approximately 4.0 nm), it is excreted exclusively through lymphatic vessels, resulting in prolonged retention within the joint cavity.

Various types of Toll-like receptors (TLRs), ranging from TLR1 to TLR9, have been identified, and among them, agonists targeting TLR2/4 and TLR7 have been approved by the FDA to date. Recent studies have demonstrated that TLR7 may be applied to the induction of apoptosis in breast cancer cells. In light of this, two TLR7 agonists (TLR_{A1} and TLR_{B1} agonists) were selected for evaluation based on the following criteria: (i) they possess an amine group that enables conjugation to CDex, and (ii) the introduced amine group does not interfere with the chemical site required for TLR7 binding. Efficacy testing was conducted using macrophages (Raw264.7), a type of immune cell, to confirm the activation of TLR signaling. Specifically, the expression of NFκB was assessed as a downstream marker of TLR signaling activation.

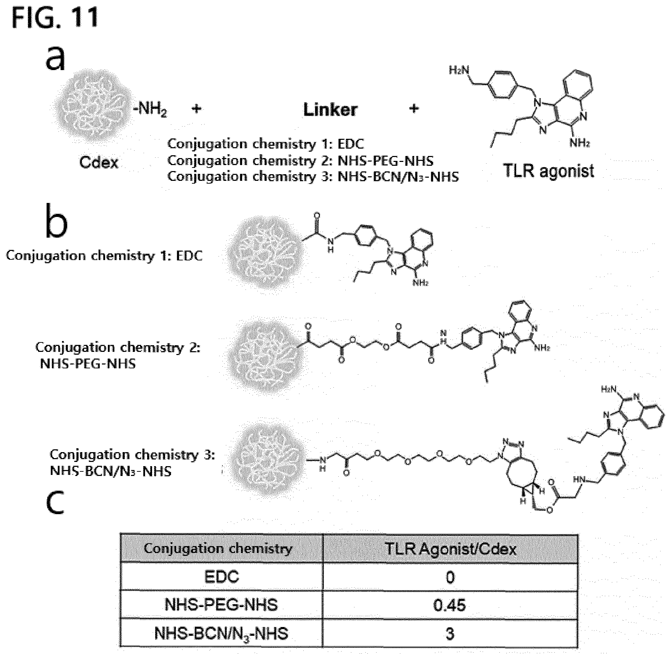

As shown in FIG. 11, both TLR_{A1} and TLR_{B1} agonists exhibited strong nuclear NFκB (green fluorescence) signals in comparison to the control group, as determined by fluorescence staining of NFκB, indicating effective activation of TLR signaling.

Furthermore, both the NFκB fluorescence assay and Western blot analysis demonstrated that cells treated with the TLR_{A1} agonist exhibited a relatively stronger NFκB signal compared to those treated with the TLR_{B1} agonist. Accordingly, in Example 3, the TLR_{A1} agonist was selected as the conjugated drug, and the conjugation process was subsequently developed.

### [Targeting Molecules]

The targeting molecule may be an antibody that targets a specific antigen or a receptor on the surface of a cell, or may be selected from the group consisting of an antigen-binding fragment thereof, a receptor, a ligand, a mimetic antibody, a repebody, an aptamer, a peptide, or a small molecule (e.g., TLR ligands, agonists, antagonists, enhancers, inhibitors, or blockers).

In certain embodiments, the targeting molecule may confer target specificity to the conjugate of the present invention.

The polysaccharide-crosslinked colloidal particles (e.g., CDex) according to the present invention have two or more crosslinker-derived functional groups exposed on their surface and can serve as a multivalent linker system or drug carrier. For example, such particles can be used as a linker system that allows multiple drugs to be conjugated to a single antibody and enables control over various pharmacokinetic parameters and drug-to-antibody ratios (DARs).

In one embodiment of the present invention, a conjugate in which one or more antibodies or antigen-binding fragments thereof and one or more drugs are conjugated via two or more crosslinker-derived surface functional groups of the polysaccharide-crosslinked colloidal particle is a type of antibody-drug conjugate (ADC).

Because antibodies exhibit strong binding affinity and high specificity toward antigens, they can selectively and potently modulate biological responses associated with a particular target. However, their intrinsic therapeutic efficacy is often limited.

An antibody-drug conjugate (ADC) is a construct in which a cytotoxic drug (payload), capable of destroying target cells such as cancer cells, is conjugated to an antibody that can guide the drug specifically to the target based on its binding affinity and specificity toward the antigen. ADCs are designed such that the antibody selectively targets the lesion site, allowing the drug to be delivered exclusively to the lesion without affecting normal tissues.

Antibody-drug conjugates (ADCs), which exert selective cytotoxic activity toward tumor cells while sparing normal cells, utilize the high target specificity of antibodies to deliver highly potent chemotherapeutic payloads selectively to tumor tissues. Accordingly, by combining a highly cytotoxic chemotherapeutic agent with an antibody possessing tumor-specific targeting capability, the therapeutic efficacy can be enhanced. Furthermore, ADCs have the potential to improve the therapeutic index of cytotoxic agents by enabling safer administration compared to their use as free drugs. In addition, ADCs can selectively deliver powerful payloads that are effective even at picomolar (pM) concentrations to tumor tissues, while minimizing systemic exposure, thereby achieving both potent antitumor efficacy and improved safety.

As used herein, the term "antibody" refers to a protein molecule that serves as a ligand specifically recognizing an antigen and includes immunoglobulin molecules that exhibit reactivity with a specific antigen. The term encompasses polyclonal antibodies, monoclonal antibodies, and whole antibodies. In addition, the term includes chimeric antibodies and bivalent or bispecific molecules, diabodies, triabodies, and tetrabodies. The term further encompasses single-chain antibodies possessing FcRn-binding capability, scFv fragments, derivatives of constant regions of antibodies, and engineered antibodies based on protein scaffolds. A whole antibody refers to a structure comprising two full-length light chains and two full-length heavy chains, wherein each light chain is linked to a corresponding heavy chain via disulfide bonds. The whole antibody includes isotypes such as IgA, IgD, IgE, IgM, and IgG, and IgG further includes subtypes such as IgG1, IgG2, IgG3, and IgG4.

Even when attached to the same antibody amino acid sequence, the type and length of the linker, as well as the site of conjugation on the antibody, may affect the in vivo conversion due to deconjugation of the linker, depending on the steric and electromagnetic environments. Furthermore, an ADC must retain the antigen-binding affinity of the native antibody prior to conjugation with the drug; that is, the presence of the conjugated drug should not interfere with the antibody-antigen interaction.

According to the present invention, when a polysaccharide-crosslinked colloidal particle engineered to have adjustable overall size and surface charge, high hydrophilicity through abundant surface-exposed hydrophilic functional groups, and capable of achieving a high DAR of 20 or more, is used as a multivalent linker system, it not only satisfies the aforementioned requirements for ADC linkers but also allows for precise control over these parameters. Therefore, the present invention enables the design of various customized drug-linker conjugate combinations in consideration of these factors.

The first critical step in developing an antibody-drug conjugate (ADC) is determining the target antigen to which the potent cytotoxic drug will be specifically delivered. By utilizing an antibody, high target specificity and prolonged systemic circulation due to an extended half-life can be achieved, allowing the cytotoxic drug to accumulate selectively in tumor cells while minimizing exposure to normal tissues. This reduces collateral damage and side effects while enhancing therapeutic efficacy. To this end, it is essential to identify a target antigen that can specifically distinguish tumor cells. The following criteria are typically required: First, the target antigen should be uniformly overexpressed on the surface of tumor cells while showing little or no expression in normal cells. A representative example is the human epidermal growth factor receptor 2 (HER2), which is known to be expressed more than 100-fold higher in HER2-positive breast cancer cells compared to normal cells. Therefore, prior to generating an antibody, various profiling analyses are conducted to assess the tumor-specific expression of candidate antigens, and once overexpression is confirmed, a monoclonal antibody that recognizes the antigen is produced. Second, strong binding affinity to the antigen is critical. Due to the internalization process mediated by receptor engagement, higher affinity for the antigenic epitope leads to enhanced internalization, which can improve therapeutic outcomes. Additionally, low immunogenicity is required. Adverse immune responses and neutralization of murine antibodies in humans can hinder anticancer efficacy. These limitations can be addressed through the use of chimeric antibodies, humanized antibodies, or fully human antibodies.

Once the antibody recognizes the antigen on the cancer cell, internalization of the antibody-drug complex into the cell must occur. To enhance internalization in cancer cells, bispecific antibodies may be employed.

MEDI4267 (Trastuzumab-META), currently under development by MedImmune and AstraZeneca, is a biparatopic antibody that targets two non-overlapping epitopes on HER2. It has been shown to induce HER2 receptor clustering, thereby promoting cellular internalization, lysosomal trafficking, and degradation.

In addition, bispecific antibodies that incorporate a tumor-targeting antibody along with a lysosomal marker such as CD63 or APLP2, or a prolactin receptor, have demonstrated enhanced cellular internalization compared to monospecific antibodies, upon recognition of the tumor antigen. One example is a bispecific antibody-drug conjugate (ADC) targeting HER2 and CD63, such as trastuzumab-based HER2×CD63-duostatin-3 (developed by Creative Biolabs), which demonstrated potent antitumor efficacy by reducing delivery to normal tissues and enhancing tumor-specific delivery.

According to the present invention, in a targeted drug delivery system comprising dextran-crosslinked nanoparticles having surface-exposed crosslinker-derived functional groups to which a targeting molecule is conjugated, the [linker]-[antibody] conjugation may be achieved by reacting thiol groups present in the antibody with maleimide or maleic hydrazide groups introduced into the multivalent linker system of the present invention, through Reaction Scheme 1 or via a "click" chemistry reaction.

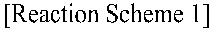

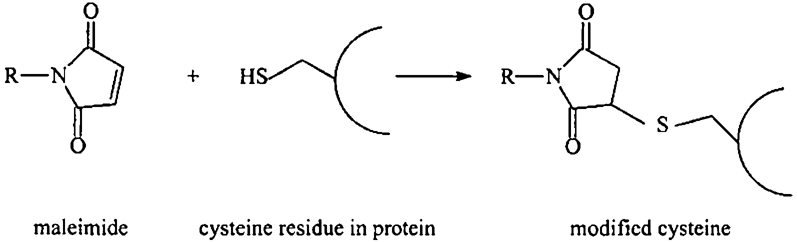

An example of a targeting molecule is an antibody fragment containing an antigen-binding region.

For instance, since many cells present in the human body (e.g., immune cells and epithelial cells) express Fc receptors on their surfaces that can bind to the Fc region of antibodies and induce undesirable non-specific binding, a single-chain variable fragment (scFv) lacking the Fc region may be used. The scFv antibody fragment is a recombinant antibody fragment engineered by cloning only the VH and VL genes of an antibody and connecting these two genes via a linker peptide using antibody gene engineering techniques. Accordingly, the scFv antibody fragment retains antigen-binding affinity while eliminating non-specific binding between the Fc region and Fc receptors. Moreover, due to its smaller molecular weight (approximately 25 kDa) compared to natural antibodies (approximately 150 kDa), it enhances the tissue penetration into target cells or target tissues, thereby maximizing the efficacy of targeted therapy.

scFv antibody fragments are typically expressed using *Escherichia coli,* making them amenable to genetic manipulation and allowing for easy production of various scFv variants. In particular, since the scFv structurally exists as a monomer with a single antigen-binding site, its antigen-binding affinity is lower than that of natural antibodies, which possess two antigen-binding sites. Therefore, the antigen-binding affinity of scFvs can be increased by genetically engineering domains capable of forming multimers such as dimers, trimers, or tetramers, and fusing them to the scFv.

Meanwhile, an example of a targeting molecule is a mimetic antibody composed of a single polypeptide chain. Like conventional antibodies, mimetic antibodies possess specific binding domains that adhere precisely to target molecules; however, they are structurally simpler and smaller in size. Although their smaller size may result in relatively reduced selectivity compared to full-size antibodies, mimetic antibodies can be more readily mass-produced and are less immunogenic, thereby minimizing the risk of side effects.

Non-limiting examples of mimetic antibodies include nanobodies, affibodies, adnectins, affimers, and DARPin scaffolds.

In addition, examples of targeting molecules include aptamers, which are not protein-based scaffolds or repebody. Aptamers exhibit deep tumor penetration distinct from antibodies and are advantageous in terms of ease of CMC (Chemistry, Manufacturing, and Controls) development.

Aptamers are DNA- or RNA-based biopolymers that, like antibodies, possess antigen-binding affinities in the nanomolar range and may serve as tumor-selective carriers for drugs.

Repebodies are a newly discovered class of immune proteins derived from fish that, like antibodies and nanobodies (i.e., single-chain antibodies derived from mammals such as llamas), are capable of recognizing antigens. They exhibit antigen-binding affinities in the nanomolar range and can serve as tumor-selective carriers for drugs. Due to their non-immunogenic nature, repebodies are well-suited for use as drug carriers. Furthermore, their low molecular weight enhances tumor tissue penetration, and they can be produced at scale with consistent and uniform quality. These properties make repebodies particularly suitable for the development of drug conjugates with improved tumor penetration.

### [Next-Generation ADC Linker Platform Technology]

Precision medicine is based on the understanding that individual responses to therapeutic interventions vary significantly from person to person. To realize precision medicine, the present invention provides a platform technology based on polysaccharide-crosslinked colloidal particles (e.g., CDex) that exhibit tunable overall size and net surface charge, can achieve a high drug-to-antibody ratio (DAR) of 20 or more if desired, and possess excellent colloidal stability due to a high density of hydrophilic functional groups exposed on the surface. When these particles are used as a multivalent linker system (see FIG. 3), it becomes possible not only to design a wide range of conjugates such as antibody-drug conjugates (ADCs) with desired circulation times or in vivo distribution and clearance pharmacokinetics, but also to customize them for enhanced therapeutic efficacy tailored to individual patients. In other words, by employing polysaccharide-crosslinked colloidal particles that exhibit excellent colloidal stability due to abundant hydrophilic groups on the surface as a multivalent linker system, it is possible to provide patient-specific linkers optimized based on individual predicted therapeutic responses, utilizing targeting agents, payloads, and biomarkers.

Among the components that constitute an ADC, the linker connecting the antibody and the drug must be stable in systemic circulation to prevent premature release of the drug from the antibody, thereby maintaining the drug in a prodrug form until it reaches the target site and minimizing damage to healthy tissues.

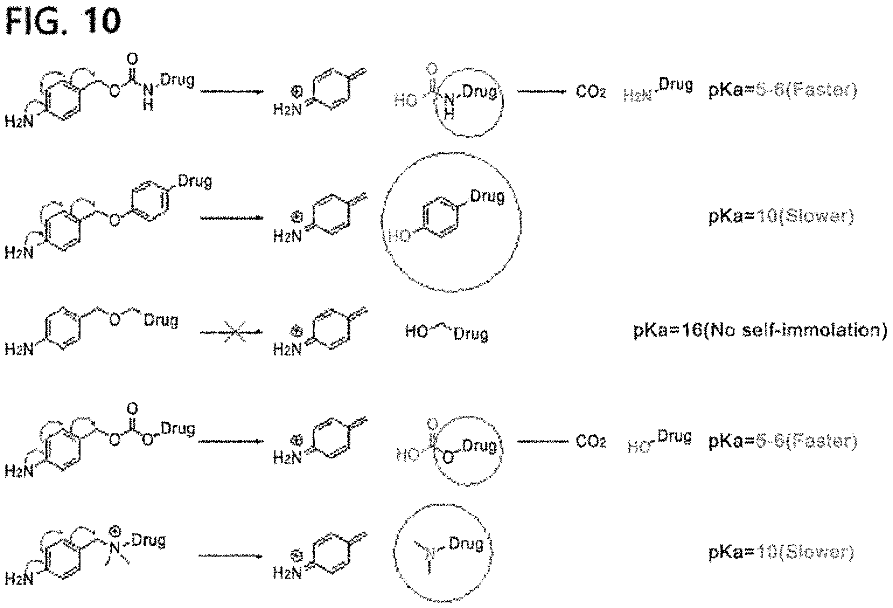

The ideal linker is one that remains stable while circulating systemically, yet is cleavable near or within the target cell to release the drug in a controlled manner, thereby ensuring that the ADC possesses both efficacy and safety. One of the major challenges in the development of safe and effective ADCs is to engineer an appropriate chemical linker between the drug and the monoclonal antibody. Unlike conventional ADC linkers, which involve complex design and synthesis, the polysaccharide-crosslinked colloidal particles used in the multivalent linker system of the present invention can be easily designed and synthesized to meet various desired conditions. Furthermore, as illustrated in FIG. 10, the drug-binding site within the linker-critical for efficient release of the active drug-can be introduced onto the surface of the polysaccharide-crosslinked colloidal particle via a facile synthetic route using the reactive functional groups derived from the crosslinker.

### 1) Linker Stability

In the multivalent linker system of the present invention, the polysaccharide-crosslinked colloidal particles can be readily designed and synthesized to ensure stability of the monoclonal antibody (mAb) during systemic circulation-reflecting the long half-life characteristic of mAbs-while avoiding any adverse impact on the antibody's stability or pharmacokinetics caused by the conjugation of the mAb to the polysaccharide-crosslinked colloidal particle-drug conjugate.

In general, catabolic reactions that may occur during systemic circulation involving linker-cytotoxic drug conjugates include, but are not limited to, the following: hydrazone cleavage, protease-mediated dipeptide cleavage, esterase-mediated carbamate cleavage, hydrolysis of acetate esters, disulfide bond cleavage, and succinimide ring opening. In addition to these known reactions, other undefined catabolic mechanisms may also exist.

Depending on the site of degradation within the linker-cytotoxic drug structure, the resulting product may retain its cytotoxic effect and remain active at the target site, or it may induce systemic toxicity during circulation. Conversely, if the cytotoxic activity is lost due to premature degradation, the drug may reach the target site but fail to elicit the intended pharmacological response. For example, in the case of thailanstatin, a splicing inhibitor developed as a payload, the drug retains its activity even after ester bond hydrolysis. However, for tubulin inhibitors such as cryptophycin or tubulysin, ester bond hydrolysis results in complete loss of activity. The dextran-crosslinked nanoparticle used in the multivalent linker system of the present invention can be engineered to address and overcome these issues.

Furthermore, in the multivalent linker system of the present invention, the dextran-crosslinked colloidal particles can be functionalized-via facile synthetic methods-at the surface-exposed functional groups derived from the crosslinker, to introduce drug-binding sites corresponding to a variety of chemical linkers commonly used in ADCs currently under clinical development. These include linkers incorporating hydrazone, disulfide, peptide, or thioether bonds, as shown in Table 2, and the associated drug release mechanisms.

**[Table 2]**

| **Linker** | **Drug Release Mechanism** |
|---|---|
| **Hydrazone** | Drug release in the acidic environment of the lysosome |
| **Peptide** | Efficient cleavage by lysosomal proteases such as cathepsin B |
| **Disulfide** | Cleavage via disulfide exchange with intracellular glutathione thiols |
| **Thioether** | Cleavage by intracellular proteolytic enzymes |

In principle, such chemical linkers induce the release of cytotoxic drugs inside tumor cells by exploiting differences in intracellular pH and enzyme concentrations. Ensuring in vivo stability of the drug-linker conjugate after administration remains one of the major challenges in ADC development, as chemically unstable linkers such as hydrazone and disulfide linkers are insufficiently stable in plasma.

Peptide-based linkers, by contrast, exhibit excellent plasma stability while maintaining a well-controlled balance between drug-linker stability and drug release capability. Cleavable dipeptide linkers such as valine-alanine (Val-Ala) and valine-citrulline (Val-Cit) undergo rapid hydrolysis in the presence of lysosomal extracts or purified human cathepsin B, meaning that the linker cleavage mechanism depends on the intracellular environment.

However, conventional linker systems such as Val-Cit or MAC-glucuronide linkers exhibit limited drug delivery efficiency, making it difficult to deliver a sufficient payload.

In contrast, the polysaccharide-crosslinked colloidal particles used in the multivalent linker system of the present invention can be engineered-via crosslinker-derived functional groups exposed on the surface of the particles-to overcome the limitations of the aforementioned chemical linkers and/or to maximize their advantages.

### 2) Non-cleavable and Cleavable Linkers

In the multivalent linker system of the present invention, polysaccharide-crosslinked colloidal particles can incorporate various cleavable and/or non-cleavable linkers at drug conjugation sites and/or at sites for conjugation to targeting molecules such as antibodies, through crosslinker-derived functional groups exposed on their surface.

Currently available linkers are broadly classified into two categories: cleavable and non-cleavable linkers. Representative types of cleavable linkers include acid-labile, redox-sensitive, and enzyme-labile linkers, while non-cleavable linkers are exemplified by thioether-based linkers (see Table 2).

Acid-labile linkers, which are chemically unstable, were developed in the early stages of ADC development. Although they are relatively less stable, they are still in use today. A representative example is the hydrazone linker, which remains stable under the neutral pH of blood (approximately pH 7.3-7.5), but undergoes hydrolysis in mildly acidic environments such as those found in the tumor microenvironment (pH 6.5-7.2), endosomes (pH 5.0-6.5), and lysosomes (pH 4.5-5.0), thereby releasing the drug. However, because acidic conditions are not exclusive to tumor sites and may also be present extracellularly, nonspecific drug release can occur. The first FDA-approved ADC, Mylotarg^{®}, is a representative example employing a hydrazone linker. Due to its low plasma stability, it was withdrawn from the U.S. market in 2010 but was re-approved in 2017. More recently, silyl ether linkers have been investigated. These exhibit improved plasma stability and have a plasma half-life exceeding 7 days, which is significantly longer than the 2-3 days observed for hydrazone linkers.

With respect to oxidation-reduction reaction linkers, disulfide linkers are a type of chemically unstable linker that function based on redox mechanisms. Once the ADC is internalized and the antibody component is degraded by enzymes, the linker is cleaved through disulfide exchange or by reducing agents such as glutathione, resulting in the release of the cytotoxic payload. Glutathione is a low-molecular-weight thiol known as an antioxidant that regulates cell proliferation and death and protects cells from inflammation and oxidative stress. It is present in intracellular environments at concentrations ranging from 0.5 to 10 mM, but in hypoxic tumor tissues, its concentration can be up to 1,000 times higher. In contrast, its concentration in plasma is relatively low (approximately 2-20 µM), which provides disulfide linkers with high plasma stability, thereby reducing nonspecific drug release and allowing more tumor-specific activation.

Peptide linkers, which are susceptible to protease-mediated cleavage, are relatively unaffected by plasma pH and exhibit excellent plasma stability due to the presence of protease inhibitors in plasma. These linkers typically have a plasma half-life of 7 to 10 days.

Proteases, particularly cathepsin B, are representative enzymes involved in this mechanism and are found within cells. Cathepsin B is associated with certain tumor tissues, such as invasive and metastatic cancers, and is present at elevated levels in such environments.

Peptide linkers are composed of dipeptides or tetrapeptides that are recognized and cleaved by lysosomal proteases once the ADC is internalized. Tetrapeptide linkers were used in early development but demonstrated limitations such as relatively slow drug release and aggregation when conjugated with hydrophobic drugs. These issues were resolved through the development of dipeptide linkers such as Val-Cit, Phe-Lys, Val-Lys, and Val-Ala, which have been successfully applied in several approved ADCs, including Adcetris^{®} and Vedotin^{®}.

In the case of Brentuximab vedotin (Adcetris), the antibody-drug conjugate (ADC) is constructed with a cleavable Val-Cit dipeptide linker that is selectively degraded by cathepsin B, a lysosomal protease present in the target cancer cell. The ADC remains stable during systemic circulation. ADCs incorporating cleavable dipeptide linkers such as Valine-Alanine (Val-Ala) and Valine-Citrulline (Val-Cit) bind to target antigens on cancer cells via the antibody portion of the ADC, forming an ADC-antigen complex that is internalized through the endosomal-lysosomal pathway. In such cases, intracellular release of the cytotoxic drug is controlled by the internal environment of the endosome or lysosome. Specifically, hydrazone linkers are unstable under acidic conditions and thus release the drug through acid-induced cleavage, whereas Val-Cit linkers are cleaved by cathepsin B in the lysosome to release monomethyl auristatin E (MMAE).

The tumor microenvironment (TME) contains a variety of proteases collectively referred to as matrix metalloproteinases (MMPs), which have been exploited in the development of methods to selectively activate anticancer antibodies in tumor tissues.

A representative approach involves using a linker that is selectively cleaved by proteases secreted in tumor tissues to connect an antibody with a protein that inhibits the antibody's function. In this configuration, the antibody's activity is suppressed under normal conditions to prevent unnecessary side effects, but within the tumor microenvironment, the linker is cleaved by matrix metalloproteinases (MMPs), thereby activating the antibody.

The β-glucuronide linker is a cleavable linker that undergoes hydrolysis by β-glucuronidase, a glycosidase abundantly present in lysosomes and known to be overexpressed in certain tumors. β-glucuronidase exhibits high enzymatic activity in acidic environments but retains only about 10% of its activity at neutral pH. This pH-dependent property enhances the plasma stability of ADCs using β-glucuronide linkers and minimizes off-target drug release. Due to the excellent plasma stability of the β-glucuronide linker, such ADCs exhibit extended systemic half-lives-for example, approximately 81 days.

The acid-sensitive linker provided through the polysaccharide-crosslinked colloidal particles of the present invention is stable in the neutral pH environment of blood (pH 7.3-7.5) but is designed to undergo hydrolysis and release the drug in mildly acidic environments such as those surrounding tumor cells (pH 6.5-7.2) or intracellular compartments following internalization, including endosomes (pH 5.0-6.5) and lysosomes (pH 4.5-5.0). Additionally, the acid-sensitive linker provided via the dextran-crosslinked nanoparticles of the present invention can be engineered to have a hydrophilic molecular structure that facilitates hydrolytic conditions.

In order for the linker to degrade under acidic conditions (pH ≤ 7) and release the free-form drug upon cleavage of the acid-sensitive linker, it is preferable that the acid-sensitive bonding site between the drug and the linker is connected via a carbonate or ester linkage.

A representative example of non-cleavable linkers includes thioether linkers, which exhibit higher plasma stability compared to cleavable linkers. Unlike cleavable linkers, non-cleavable linkers are not subject to degradation themselves; instead, drug release occurs only after the antibody is degraded intracellularly following cellular uptake in the ADC form.

The drug released in this manner typically carries a charge and thus has a reduced likelihood of diffusing into surrounding cells (i.e., exhibits a limited bystander effect). Although such ADCs lack cytotoxic effects on neighboring cells, they exert their effect only within target cells after internalization, thereby indicating relatively high safety. ADCs incorporating non-cleavable linkers rely more heavily on intracellular biological mechanisms within the target cells. Many studies have demonstrated that ADCs with non-cleavable linkers provide high stability and efficacy, making them valuable linkers for ADC development. One such example currently in clinical use is Kadcyla^{®}.

In the case of ado-trastuzumab emtansine (Kadcyla^{®}), a non-cleavable SMCC linker is used. Non-cleavable linkers release cytotoxic drugs upon lysosomal degradation of the ADC that has been internalized into the target cell. This not only avoids undesired systemic drug release but also allows modulation of the physicochemical properties of the conjugated drug, thereby tuning its affinity for the carrier or enhancing its potency.

The stability of an ADC during systemic circulation en route to its target is critical for achieving the desired therapeutic index, regardless of whether a cleavable or non-cleavable linker is employed.

The crosslinker-derived functional groups exposed on the surface of the polysaccharide-crosslinked colloidal particles may be engineered to introduce various types of linkers, as described above, at the conjugation site of the drug, in a manner that enables the release of the active drug.

For example, to achieve selective in vivo drug release, a cleavable portion may be incorporated into the crosslinker-derived functional group exposed on the surface of the polysaccharide-crosslinked colloidal particle, such that the bond with the drug and/or antibody is selectively cleaved only within or outside target cells, thereby allowing the release of the active drug.

The cleavable portion introduced into the crosslinker-derived functional group exposed on the surface of the polysaccharide-crosslinked colloidal particle may comprise, without limitation: (i) a linkage that is degradable in the acidic environment of lysosomes within cancer cells; (ii) an acid-sensitive bond cleavable under mildly acidic tumor microenvironments (pH ≤ 7); or (iii) a bond cleavable by extracellular matrix (ECM)-degrading enzymes secreted by cancer cells or by intracellular hydrolases that participate in peptide bond cleavage.

Furthermore, the polysaccharide-crosslinked colloidal particles used in the multivalent linker system of the present invention can exhibit high solubility in both organic solvents and aqueous media by controlling the crosslinker-derived functional groups exposed on their surfaces. This property addresses multiple issues associated with hydrophobic linkers. Specifically, the particles can be engineered to exhibit water solubility, low toxicity, low immunogenicity, and tunable linker size, resulting in improved pharmacokinetic profiles in vivo. Such design may enhance drug half-life and plasma concentration, leading to an increased area under the plasma concentration-time curve (AUC).

### [Drugs Conjugated to Polysaccharide-Crosslinked Colloidal Particles]

In the multivalent linker system or drug carrier of the present invention, the polysaccharide-crosslinked colloidal particles-such as dextran-crosslinked nanoparticles (CDex)-may be conjugated to various types of substances through control of crosslinker-derived functional groups exposed on their surfaces.

Accordingly, in the present invention, the polysaccharide-crosslinked colloidal particle itself and/or a molecule conjugated thereto via the crosslinker-derived functional groups may serve as a drug capable of diagnostic and/or therapeutic applications in various forms.

Non-limiting examples of drugs conjugated via the crosslinker-derived functional groups exposed on the surface of the polysaccharide-crosslinked colloidal particles include: low-molecular-weight organic drugs; metal-based drugs including radioactive isotopes; amino acids; polypeptides; and/or nucleic acids such as DNA, siRNA, or miRNA.

The drugs conjugated via the crosslinker-derived functional groups of the polysaccharide-crosslinked colloidal particles may be substances capable of binding to a drug targeting moiety. Non-limiting examples thereof include ligands, receptors, aptamers, proteins, hormones, neurotransmitters, growth factors, cytokines, chemokines, and small molecules.

The drugs conjugated via the crosslinker-derived functional groups of the polysaccharide-crosslinked colloidal particles may also function as agonists, antagonists, enhancers, inhibitors, or blockers of the target proteins of interest.

The drug conjugated to the crosslinker-derived functional group of the polysaccharide-crosslinked colloidal particle may be a low-molecular-weight cytotoxic agent that induces cancer cell death and/or a peptide antigen that activates immune responses within the tumor microenvironment. Additionally, the drug conjugated to the crosslinker-derived functional group of the polysaccharide-crosslinked colloidal particle may be a non-cytotoxic agent capable of enhancing immune responses.

Furthermore, the drug conjugated to the crosslinker-derived functional group of the polysaccharide-crosslinked colloidal particle may target a membrane receptor (e.g., TLR) embedded in a biological lipid membrane as a drug target protein. For example, the drug target protein embedded in the biological lipid membrane may be a receptor that undergoes conformational changes upon ligand binding.

Such conformational changes in the drug target protein may lead to the activation of intracellular signaling pathways and, in some cases, trigger physiological responses. These pathways may include alterations in gene expression, enzymatic activity, ion channel opening, or other cellular responses. Cellular responses may vary widely and include changes in cell metabolism, cell division, muscle contraction, neurotransmission, or other physiological processes.

Physiological responses refer to physiological changes exhibited by living organisms in reaction to environmental factors or stimuli and may include alterations in physical reactions or biological activities. Such physiological responses may include, for example, changes in bodily functions such as heart rate, respiratory rate, and blood pressure.

In addition, molecules (e.g., lipids, saccharides) or protein complexes that can trigger specific intracellular responses or signaling pathways also fall within the scope of the drug target proteins of the present invention.

In the present invention, the biological lipid membrane in which the drug target protein is located includes not only the plasma membrane but also lipid bilayers of intracellular organelles such as mitochondria.

Eukaryotic cells, including human cells, possess an endomembrane system in which intracellular organelles are present. The membranes constituting the endomembrane system are composed of lipid bilayers, and proteins are either embedded within or attached to the membrane.

### [Cytotoxic Drugs Conjugated to Polysaccharide-Crosslinked Colloidal Particles]

In antibody-drug conjugate (ADC) constructs, cytotoxic drugs serving as the payload play a critical role in eliminating cancer cells or senescent cells and are essential components for exhibiting therapeutic efficacy.

Early-generation ADCs employed small-molecule anticancer agents with relatively low cytotoxicity, such as doxorubicin and vinblastine, and consequently demonstrated limited antitumor efficacy. To address this, more potent cytotoxic drugs were incorporated into ADCs; however, many of these agents exhibited excessive toxicity and affected normal cells due to the bystander effect. Furthermore, since drugs must be conjugated with minimal impact on the antibody, the amount of payload that can be delivered is limited. This necessitates that cytotoxic drugs suitable for ADCs must be capable of killing the majority of tumor cells at low concentrations (nM or pM levels) and must exert therapeutic effects in a controlled drug-release manner.

In contrast, the conjugate system of the present invention, which employs polysaccharide-crosslinked colloidal particles (e.g., CDex) exhibiting high colloidal stability due to a high density of surface-exposed hydrophilic functional groups in a multivalent linker configuration, allows for high payload loading of cytotoxic drugs, enabling a drug-to-antibody ratio (DAR) of 20 or greater. As such, even if the linker is cleaved in the bloodstream, this system can mitigate stability issues. Moreover, by using low-toxicity small-molecule anticancer agents-despite their reduced cytotoxicity and potential for fewer bystander effects on normal cells-high antitumor efficacy can still be achieved in a controlled manner.

Cytotoxic drugs are structurally and mechanistically diverse; however, they are subject to stringent limitations, primarily because they must exhibit low immunogenicity and maintain chemical stability during systemic circulation. An unstable cytotoxic payload may undergo chemical degradation or modification during storage or after conjugation.

The present invention addresses these challenges by allowing modulation of the molecular weight, size, and surface charge of polysaccharide-crosslinked colloidal particles. By controlling these parameters, the total size and surface charge of the conjugate employing dextran-crosslinked nanoparticles as multivalent linkers can be tailored to achieve desired blood circulation time, renal clearance, and hepatic clearance profiles.

Accordingly, the multivalent linker system of the present invention not only expands the repertoire of cytotoxic drugs that may be used as payloads, but also enables the creation of a variety of conjugates-including ADCs-with tunable drug-to-antibody ratios (DARs) and cytotoxic mechanisms of action, thereby providing therapeutically effective antitumor agents.

Currently approved or clinically investigated ADCs mainly employ cytotoxic drugs belonging to two mechanistic classes: microtubule-disrupting agents and DNA-modifying agents. Recently, the approval of topoisomerase inhibitors has increased interest in payloads with alternative mechanisms of action.

Table 3 classifies the cytotoxic drugs most commonly used in clinical-stage ADCs according to their mechanisms of action.

**[Table 3]**

| **Cytotoxic Drug** | **Mechanism of Action** |
|---|---|
| Auristatins | Tubulin polymerase inhibitor |
| Maytansines | Tubulin depolymerisation |
| Calicheamicins | DNA cleavage |
| Duocarmycins | DNA minor groove alkylating agent |
| PBD dimers | DNA minor groove cross-linker |
| SN-38 | DNA topoisomerase inhibitor |

Microtubules play a critical role in the cell cycle, and if abnormalities occur in microtubules, cell division cannot proceed. Most known microtubule-disrupting agents are derived from natural products and exhibit high cytotoxicity. Unlike drugs that affect microtubules and act only at specific stages of the cell cycle, DNA-modifying agents can kill cells at any point in the cycle. A representative cytotoxic drug, monomethyl auristatin E (MMAE, also referred to as "vedotin" in drug formulations), binds to microtubules, arrests the cell cycle, and induces cell death. Another microtubule inhibitor developed by ImmunoGen is an ansamycin antibiotic derived from maytansine, a natural product isolated from the Ethiopian shrub of the genus *Maytenus serrata.* Maytansine binds to tubulin, inhibits microtubule assembly, and promotes microtubule disassembly, thereby interfering with cell division. Maytansine demonstrates cytotoxicity in many tumor cell lines and can suppress tumor growth. These derivatives are designed to be conjugated to linkers via thiol groups. For example, in the case of DM1, it is readily cleaved under intracellular reductive conditions. In contrast, DM4 contains a methyl-substituted disulfide bond, which reduces susceptibility to reduction.

Rapidly dividing cells, such as cancer cells, are significantly more sensitive to cytotoxic agents than slowly dividing normal cells, allowing for the selective elimination of cancer cells using DNA-modifying agents. Although DNA damage is the most common mechanism of action for many chemotherapeutic agents, clinical use of such agents often becomes limited due to a narrow therapeutic window and increased risk of toxicity as potency increases. However, by conjugating such highly potent drugs to antibodies with high targeting specificity, both safety and efficacy of the drug can be enhanced.

Most DNA-modifying agents are also derived from natural products. Bristol-Myers Squibb and Medarex have developed a variety of natural product-derived active pharmaceutical ingredients (APIs) that function through a DNA alkylation mechanism to induce mutagenesis. A synthetic toxin known as a duocarmycin analog, developed by the Netherlands-based company Synthon (formerly Syntarga), also binds to DNA in this manner. SYD985 (trastuzumab duocarmazine), which is currently in Phase III clinical trials and developed by Synthon, is known to employ duocarmycin.

Other drugs used as DNA-modifying agents include calicheamicin, pyrrolobenzodiazepine (PBD), and SN-38, which is the active metabolite of irinotecan. Calicheamicin, isolated from bacteria by Pfizer, binds to the DNA minor groove and subsequently induces DNA strand cleavage. Calicheamicin is used in both of Pfizer's antibody-drug conjugates (ADCs), Mylotarg and Besponsa. PBD dimers, newly developed cytotoxic drugs by Seattle Genetics following their auristatin-based ADC platform, were applied to an ADC targeting CD70 and entered clinical trials, but the development has since been discontinued. SN-38, the active metabolite of irinotecan, acts as an inhibitor of DNA topoisomerase. Compared to previously approved cytotoxic drugs used in ADCs-such as auristatins, maytansinoids, and calicheamicin-SN-38 is known to have lower potency. This allows for the development of ADCs with reduced side effects and higher drug-to-antibody ratios (DARs). Representative ADCs utilizing SN-38 as a cytotoxic payload include IMMU-130 and IMMU-132, which have been developed by Immunomedics and are currently in clinical stages.

### [Target Antigens]

In the intracellular delivery of ADCs, the extracellular concentration of the antigen is a critical determinant, as a sufficient amount of active drug must be internalized into the target cell to exert a therapeutic effect. A target antigen may be expressed at levels at least 10 times higher on cancer cells compared to normal cells.

In cases where the number of molecules of a tumor-specific antigen is limited, the therapeutic efficacy may be enhanced by employing a more potent toxin or by increasing the drug-to-antibody ratio (DAR).

In the present invention, the molecule exhibiting a targeting function, such as an antibody, may be an immune checkpoint inhibitor, which is a type of immuno-oncology agent.

When designing a conjugate such as an antibody-drug conjugate (ADC) of the present invention, the target is not limited to cancer cells, but may also include cells associated with infectious organisms and/or autoimmune diseases.

Accordingly, the cells targeted by the targeting molecule of the present invention may include cancer cells, cells associated with infectious diseases, and/or cells related to autoimmune disorders.

Non-limiting examples of target antigens include tumor-selective surface antigens such as Her2, FolR, and PSMA, as well as tumor-overexpressed antigens such as Trop2, which may be sparsely distributed in normal tissues.

The cancer cell target antigen may include, but is not limited to, the following: 5T4, ABL, ABCF1, ACVR1, ACVR1B, ACVR2, ACVR2B, ACVRL1, ADORA2A, AFP, Aggrecan, AGR2, AICDA, AIF1, AIGI, AKAP1, AKAP2, ALCAM, ALK, AMH, AMHR2, ANGPT1, ANGPT2, ANGPTL3, ANGPTL4, ANPEP, APC, APOC1, AR, aromatase, ASPH, ATX, AX1, AXL, AZGP1 (zinc-a-glycoprotein), B4GALNT1, B7, B7.1, B7.2, B7-H1, B7-H3, B7-H4, B7-H6, BAD, BAFF, BAG1, BAI1, BCR, BCL2, BCL6, BCMA, BDNF, BLNK, BLR1 (MDR15), BIyS, BMP1, BMP2, BMP3B (GDFIO), BMP4, BMP6, BMP8, BMP10, BMPR1A, BMPR1B, BMPR2, BPAG1 (plectin), BRCA1, C19orflO (IL27w), C3, C4A, C5, C5R1, CA6, CA9, CANT1, CAPRIN-1, CASP1, CASP4, CAV1, CCBP2 (D6/JAB61), CCL1 (1-309), CCLI1 (eotaxin), CCL13 (MCP-4), CCL15 (MIP-Id), CCL16 (HCC-4), CCL17 (TARC), CCL18 (PARC), CCL19 (MIP-3b), CCL2 (MCP-1), MCAF, CCL20 (MIP-3a), CCL21 (MEP-2), SLC, exodus-2, CCL22(MDC/STC-I), CCL23 (MPIF-I), CCL24 (MPIF-2/ eotaxin-2), CCL25 (TECK), CCL26(eotaxin-3), CCL27 (CTACK/ILC), CCL28, CCL3 (MIP-Ia), CCL4 (MIPIb), CCL5(RANTES), CCL7 (MCP-3), CCL8 (mcp-2), CCNA1, CCNA2, CCND1, CCNE1, CCNE2, CCR1 (CKR1/HM145), CCR2 (mcp-IRB/RA), CCR3 (CKR3/CMKBR3), CCR4, CCR5(CMKBR5/ChemR13), CCR6 (CMKBR6/CKR-L3/STRL22/DRY6), CCR7 (CKR7/EBI1), CCR8 or CDw198 (CMKBR8/TERI/CKR-L1), CCR9 (GPR-9-6), CCRL1 (VSHK1), CCRL2 (L-CCR), CD13, CD164, CD19, CDH6, CDIC, CD2, CD20, CD21, CD200, CD22, CD23, CD24, CD27, CD28, CD29, CD3, CD33, CD35, CD37, CD38, CD3E, CD3G, CD3Z, CD4, CD40, CD40L, CD44, CD45RB, CD47, CD52, CD56, CD69, CD70, CD72, CD74, CD79A, CD79B, CD8, CD80, CD81, CD83, CD86, CD97, CD99, CD117, CD125, CD137, CD147, CD179b, CD223, CD279, CD152, CD274, CDH1 (E- cadherin), CDH1O, CDH12, CDH13, CDH18, CDH19, CDH2O, CDH3, CDH5, CDH7, CDH8, CDH9, CDH17, CDK2, CDK3, CDK4, CDK5, CDK6, CDK7, CDK9, CDKN1A (p21Wap1/Cip1), CDKN1B (p27Kip1), CDKN1C, CDKN2A (p16INK4a), CDKN2B, CDKN2C, CDKN3, CEA, CEACAM5, CEACAM6, CEBPB, CERI, CFC1B, CHGA, CHGB, Chitinase, CHST1O, CIK, CKLFSF2, CKLFSF3, CKLFSF4, CKLFSF5, CKLFSF6, CKLFSF7, CKLFSF8, CLDN3, CLDN6, CLDN7 (claudin-7), CLDN18, CLEC5A, CLEC6A, CLEC11A, CLEC14A, CLN3, CLU (clusterin), CMKLR1, CMKOR1 (RDC1), CNR1, C-MET, COL18A1, COLIA1, COL4A3, COL6A1, CR2, Cripto, CRP, CSF1 (M-CSF), CSF2 (GM-CSF), CSF3 (GCSF), CTAG1B (NY-ESO-1), CTLA4, CTL8, CTNNB1 (β-catenin), CTSB (cathepsin B), CX3CL1 (SCYD1), CX3CR1 (V28), CXCL1 (GRO1), CXCL1O (IP-IO), CXCLI1 (1-TAC/IP-9), CXCL12 (SDF1), CXCL13, CXCL14, CXCL16, CXCL2 (GRO2), CXCL3 (GRO3), CXCL5 (ENA-78/LIX), CXCL6 (GCP-2), CXCL9 (MIG), CXCR3 (GPR9/CKR-L2), CXCR4, CXCR6 (TYMSTR/STRL33/Bonzo), CYB5, CYC1, CYSLTR1, DAB2IP, DES, DKFZp451J0118, DLK1, DNCL1, DPP4, E2F1, Engel, Edge, Fennel, EFNA3, EFNB2, EGF, EGFR, ELAC2, ENG, Enola, ENO2, ENO3, EpCAM, EPHA1, EPHA2, EPHA3, EPHA4, EPHA5, EPHA6, EPHA7, EPHA8, EPHA9, EPHA10, EPHB1, EPHB2, EPHB3, EPHB4, EPHB5, EPHB6, EPHRIN-A1, EPHRIN-A2, EPHRINA3, EPHRIN-A4, EPHRIN-A5, EPHRIN-A6, EPHRIN-B1, EPHRIN-B2, EPHRIN-B3, EPHB4, EPG, ERBB2 (HER-2), ERBB3, ERBB4, EREG, ERK8, estrogen receptor, Earl, ESR2, F3 (TF), FADD, FAP, farnesyl transferase, FasL, FASNf, FCER1A, FCER2, FCGR3A, FGF, FGF1 (aFGF), FGF10, FGF1 1, FGF12, FGF12B, FGF13, FGF14, FGF16, FGF17, FGF18, FGF19, FGF2 (bFGF), FGF20, FGF21, FGF22, FGF23, FGF3 (int-2), FGF4 (HST), FGF5, FGF6 (HST-2), FGF7 (KGF), FGF8, FGF9, FGFR1, FGFR2, FGFR3, FGFR4, FIGF (VEGFD), FIL1(EPSILON), FBL1 (ZETA), FLJ12584, FLJ25530, FLRT1 (fibronectin), FLT1, FLT-3, FOLR1, FOS, FOSL1(FRA-1), FR-alpha, FY (DARC), GABRP (GABAa), GAGEB1, GAGEC1, GALNAC4S-6ST, GATA3, GD2, GD3, GDF5, GFI1, GFRA1, GGT1, GM-CSF, GNAS1, GNRH1, GPC1, GPC3, GPNB, GPR2 (CCR10), GPR31, GPR44, GPR81 (FKSG80), GRCC1O (C1O), GRP, GSN (Gelsolin), GSTP1, GUCY2C, HAVCR1, HAVCR2, HDAC, HDAC4, HDAC5, HDAC7A, HDAC9, Hedgehog, HER3, HGF, HIF1A, HIP1, histamine and histamine receptors, HLA-A, HLA-DR, HLA-DRA, HLA-E, HM74, HMOXI, HSP90, HUMCYT2A, ICEBERG, ICOSL, ID2, IFN-a, IFNA1, IFNA2, IFNA4, IFNA5, EFNA6, BFNA7, IFNB1, IFN gamma, IFNW1, IGBP1, IGF1, IGFIR, IGF2, IGFBP2, IGFBP3, IGFBP6, DL-1, ILIO, ILIORA, ILIORB, IL-1, IL1R1 (CD121a), IL1R2(CD121b), IL-IRA, IL-2, IL2RA (CD25), IL2RB(CD122), IL2RG(CD132), IL-4, IL-4R(CD123), IL-5, IL5RA(CD125), IL3RB(CD131), IL-6, IL6RA, (CD126), IR6RB(CD130), IL-7, IL7RA(CD127), IL-8, CXCR1 (IL8RA), CXCR2, (IL8RB/CD128), IL-9, IL9R(CD129), IL-10, IL10RA(CD210), IL10RB(CDW210B), IL-11, IL11RA, IL-12, IL-12A, IL-12B, IL-12RB1, IL-12RB2, IL-13, IL13RA1, IL13RA2, IL14, IL15, IL15RA, IL16, IL17, IL17A, IL17B, IL17C, IL17R, IL18, IL18BP, IL18R1, IL18RAP, IL19, ILIA, ILIB, ILIF10, ILIF5, IL1F6, ILIF7, IL1F8, DL1F9, ILIHYI, ILIR1, IL1R2, ILIRAP, ILIRAPLI, ILIRAPL2, ILIRL1, IL1RL2, ILIRN, IL2, IL20, IL20RA, IL21R, IL22, IL22R, IL22RA2, IL23, DL24, IL25, IL26, IL27, IL28A, IL28B, IL29, IL2RA, IL2RB, IL2RG, IL3, IL30, IL3RA, IL4, 1L4, IL6ST (gp 130), ILK, INHA, INHBA, INSL3, INSL4, IRAK1, IRAK2, ITGA1, ITGA2, ITGA3, ITGA6 (α6 integrin), ITGAV, ITGB3, ITGB4 (β4 integrin), JAG1, JAK1, JAK3, JTB, JUN, K6HF, KAI1, KDR, KIT, KITLG, KLF5 (GC Box BP), KLF6, KLK10, KLK12, KLK13, KLK14, KLK15, KLK3, KLK4, KLK5, KLK6, KLK9, KRT1, KRT19 (Keratin 19), KRT2A, KRTHB6 (Hair-specific type II keratin), L1CAM, LAG3, LAMA5, LAMP1, LEP (Leptin), Lewis Y antigen ("LeY"), LILRB1, Lingo-p75, Lingo-Troy, LGALS3BP, LRRC15, LPS, LTA (TNF-b), LTB, LTB4R (GPR16), LTB4R2, LTBR, LY75, LYPD3, MACMARCKS, MAG or OMgp, MAGEA3, MAGEA6, MAP2K7 (c-Jun), MCP-1, MDK, MIB1, midkine, MIF, MISRII, MJP-2, MLSN, MK, MKI67 (Ki-67), MMP2, MMP9, MS4A1, MSMB, MT3 (Metallothionein-III), mTOR, MTSS1, MUC1 (mucin), MUC16, MYC, MYD88, NCK2, NCR3LG1, neurocan, NFKBI, NFKB2, NGFB (NGF), NGFR, NgR-Lingo, NgRNogo66, (Nogo), NgR-p75, NgR-Troy, NMEI (NM23A), NOTCH, NOTCH1, NOTCH3, NOX5, NPPB, NROBI1, NROB2, NRID1, NR1D2, NR1H2, NR1H3, NR1H4, NR112, NR113, NR2C1, NR2C2, NR2E1, NR2E3, NR2F1, NR2F2, NR2F6, NR3C1, NR3C2, NR4A1, NR4A2, NR4A3, NR5A1, NR5A2, NR6A1, NRP1, NRP2, NT5E, NTN4, NY-ESO1, ODZI, OPRDI, P2RX7, PAP, PART1, PATE, PAWR, P- cadherin, PCA3, PCD1, PD-L1, PCDGF, PCNA, PDGFA, PDGFB, PDGFRA, PDGFRB, PECAMI, L1-CAM, peg- asparaginase, PF4 (CXCL4), PGF, PGR, phosphacan, PIAS2, PI3 kinase, PIK3CG, PLAU (uPA), PLG, PLXDCI, PKC, PKC- 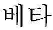 , PPBP (CXCL7), PPID, PR1, PRAME, PRKCQ, PRKD1, PRL, PROC, PROK2, PSAP, PSCA, PSMA, PTAFR, PTEN, PTHR2, PTGS2 (COX-2), PTN, PVRIG, RAC2 (P21Rac2), RANK, RANK ligand, RARB, RGS1, RGS13, RGS3, RNFI1O (ZNF144), Ron, ROBO2, ROR1, RXR, S100A2, SCGB 1D2 (lipophilin B), SCGB2A1 (mammaglobin B), SCGB2A2 (mammaglobin A), SCYE1 (endothelial monocyte-activating cytokine), SDF2, SERPENA1, SERPINA3, SERPINB5 (maspin), SERPINEI (PAI-I), SERPINFI, SHIP-1, SHIP-2, SHB1, SHB2, SHBG, SfcAZ, SLAMF7, SLC2A2, SLC33A1, SLC43A1, SLC44A4, SLC34A2, SLIT2, SPP1, SPRR1B (Spr1), ST6GAL1, ST8SIA1, STAB1, STATE, STEAP, STEAP2, TB4R2, TBX21, TCP1O, TDGF1, TEK, TGFA, TGFB1, TGFB1I1, TGFB2, TGFB3, TGFBI, TGFBR1, TGFBR2, TGFBR3, THIL, THBS1 (Thrombospondin-1), THBS2, THBS4, THPO, TIE (Tie-1), TIMP3, tissue factor, TLR1, TLR2, TLR3, TLR4, TLR5, TLR6, TLR7, TLR8, TLR9, TLR10, TLR11, TNF, TNF-a, TNFAIP2 (B94), TNFAIP3, TNFRSFI1A, TNFRSF1A, TNFRSF1B, TNFRSF21, TNFRSF5, TNFRSF6 (Fas), TNFRSF7, TNFRSF8, TNFRSF9, TNFSFIO (TRAIL), TNFRSF10A, TNFRSFI10B, TNFRSF12A, TNFRSF17, TNFSF1 1 (TRANCE), TNFSF12 (APO3L), TNFSF13 (April), TNFSF13B, TNFSF14 (HVEM-L), TNFRSF14 (HVEM), TNFSF15 (VEGI), TNFSF18, TNFSF4 (OX40 ligand), TNFSF5 (CD40 ligand), TNFSF6 (FasL), TNFSF7 (CD27 ligand), TNFSF8 (CD30 ligand), TNFSF9 (4-1BB ligand), TOLLIP, Toll-like receptors, TOP2A (topoisomerase Iia), TP53, TPM1, TPM2, TRADD, TRAF1, TRAF2, TRAF3, TRAF4, TRAF5, TRAF6, TRKA, TREM1, TREM2, TROP2, TRPC6, TSLP, TWEAK, Tyrosinase, uPAR, VEGF, VEGFB, VEGFC, versican, VHL C5, VLA-4, WT1, Wnt-1, XCL1 (Lymphotactin), XCL2 (SCM-Ib), XCRI (GPR5/CCXCR1), YY1, ZFPM2, CLEC4C (BDCA-2, DLEC, CD303, CDH6, CLECSF7), CLEC4D (MCL, CLECSF8), CLEC4E (Mincle), CLEC6A (Dectin-2), CLEC5A (MDL-1, CLECSF5), CLEC1B (CLEC-2), CLEC9A (DNGR-1), CLEC7A (Dectin-1), CLEC11A, PDGFRa, SLAMF7, GP6 (GPVI), LILRA1 (CD85I), LILRA2 (CD85H, ILT1), LILRA4 (CD85G, ILT7), LILRA5 (CD85F, ILT11), LILRA6 (CD85b, ILT8), LILRB1, NCR1 (CD335, LY94, NKp46), NCR3 (CD335, LY94, NKp46), NCR3 (CD337, NKp30), OSCAR, TARM1, CD30, CD300C, CD300E, CD300LB (CD300B), CD300LD (CD300D), KIR2DL4 (CD158D), KIR2DS, KLRC2 (CD159C, NKG2C), KLRK1 (CD314, NKG2D), NCR2 (CD336, NKp44), PILRB, SIGLEC1 (CD169, SN), SIGLEC5, SIGLEC6, SIGLEC7, SIGLEC8, SIGLEC9, SIGLEC10, SIGLEC11, SIGLEC12, SIGLEC14, SIGLEC15 (CD33L3), SIGLEC16, SIRPA, SIRPB1 (CD172B), TREM1 (CD354), TREM2, KLRF1 (NKp80), 17-1A, SLAM7, MSLN, CTAG1B/NY-ESO-1, MAGEA3/A6, ATP5I (Q06185), OAT (P29758), AIFM1 (Q9Z0X1), AOFA (Q64133), MTDC (P18155), CMC1 (Q8BH59), PREP (Q8K411), YMEL1 (O88967), LPPRC (Q6PB66), LONM (Q8CGK3), ACON (Q99KI0), ODO1 (Q60597), IDHP (P54071), ALDH2 (P47738), ATPB (P56480), AATM (P05202), TMM93 (Q9CQW0), ERGI3 (Q9CQE7), RTN4 (Q99P72), CL041 (Q8BQR4), ERLN2 (Q8BFZ9), TERA (Q01853), DAD1 (P61804), CALX (P35564), CALU (O35887), VAPA (Q9WV55), MOGS (Q80UM7), GANAB (Q8BHN3), ERO1A (Q8R180), UGGG1 (Q6P5E4), P4HA1 (Q60715), HYEP (Q9D379), CALR (P14211), AT2A2 (055143), PDIA4 (P08003), PDIA1 (P09103), PDIA3 (P27773), PDIA6 (Q922R8), CLH (Q68FD5), PPIB (P24369), TCPG (P80318), MOT4 (P57787), NICA (P57716), BASI (P18572), VAPA (Q9WV55), ENV2 (P11370), VAT1 (Q62465), 4F2 (P10852), ENOA (P17182), ILK (O55222), GPNMB (Q99P91), ENV1 (P10404), ERO1A (Q8R180), CLH (Q68FD5), DSG1A (Q61495), AT1A1 (Q8VDN2), HYOU1 (Q9JKR6), TRAP1 (Q9CQN1), GRP75 (P38647), ENPL (P08113), CH60 (P63038), or CH10 (Q64433).

### [Functional Modifications of Polysaccharide-Crosslinked Colloidal Particles as In Vivo Administrable Modifiers]

In the case of a conjugate in which the polysaccharide-crosslinked colloidal particle (e.g., CDex) of the present invention is used as a multivalent linker system or as a drug carrier, the polysaccharide-crosslinked colloidal particle may provide additional functionalities as a modifier, as described below.

### 1. Modifier for In Vivo Introduction/Administration

In the present specification, the terms "in vivo introduction" and "in vivo administration" are used interchangeably.

The term "body fluid" in this specification refers to an in vivo environment, and includes, for example, extracellular fluids such as blood, lymph, and interstitial fluid, as well as intracellular fluids.

The polysaccharide-crosslinked colloidal particles according to the present invention may provide or enhance dispersion stability and colloidal stability in body fluids for target substances such as drugs, through hydration of hydrophilic functional groups exposed on the surface thereof. Accordingly, by utilizing the polysaccharide-crosslinked colloidal particle platform technology, which allows for precise control of various physicochemical properties, it is possible to regulate, for example, the blood concentration of a drug within an appropriate range.

### 2. Colloidal Stability Imparting Modifier

Colloidal stability is a critical requirement for the biomedical application of nanomaterials. Hydrophobic drugs, when their surface becomes unstable under physiological conditions (e.g., salt concentration, pH), may aggregate due to van der Waals forces. Such aggregation may undesirably alter the physicochemical properties or pharmacokinetics of the drug, or result in their deposition in tissues and subsequent failure of clearance.

In contrast, a conjugate in which a drug is modified with the polysaccharide-crosslinked colloidal particle according to the present invention can improve colloidal stability in biological fluids through the hydration of hydrophilic functional groups exposed on the surface of the polysaccharide-crosslinked colloidal particle.

The conjugate in which the drug is modified with a polysaccharide-crosslinked colloidal particle according to the present invention has been confirmed to exhibit superior dispersion stability in solution under various salt concentrations and pH conditions, as compared with control conjugates modified with conventional dextran or dextran derivatives (see FIG. 8 of Korean Patent Application No. 10-2023-0016863, incorporated herein by reference). Furthermore, it has also been confirmed to exhibit colloidal stability in blood (see FIG. 9 of Korean Patent Application No. 10-2023-0016863).

### 3. Hydrodynamic Size Modifier

The polysaccharide-crosslinked colloidal particles according to the present invention can modulate the hydrodynamic diameter of a drug.

Therefore, the in vivo pharmacokinetic properties of a conjugate in which a drug is modified with the polysaccharide-crosslinked colloidal particles of the present invention are determined not by the original size or hydrophobicity of the drug itself, but by the hydrodynamic diameter of the modified polysaccharide-crosslinked colloidal particles.

The present invention enables precise control of the total hydrodynamic diameter of a drug-conjugated complex by employing polysaccharide-crosslinked colloidal particles designed to have a desired hydrodynamic diameter, through adjustment of the molecular weight of the polysaccharide-crosslinked colloidal particles (see Example 1).

In addition, in the conjugate in which a drug is modified with the polysaccharide-crosslinked colloidal particles of the present invention, the colloidal particles that contribute to the total hydrodynamic diameter are spherical nanoparticles formed by intramolecular and/or intermolecular crosslinking of 1 to 3 branched polysaccharides or 2 to 30 cyclic polysaccharides in an aqueous solvent via -OH groups of their monosaccharide building blocks with a crosslinker. In the polysaccharide-crosslinked colloidal particles of the present invention, the crosslinker substitution ratio can be controlled in the range of 2% to 50% relative to the number of functional groups on the polysaccharides. Further, 2% to 98% of the crosslinkers may be configured such that one terminal end is not involved in crosslinking and remains exposed on the outer surface, thereby enabling precise control over the swelling degree of the colloidal particles. Accordingly, not only the polysaccharide-crosslinked colloidal particles themselves, but also the conjugates modified therewith, can be engineered to have a uniform size distribution.

Thus, the present invention provides desired in vivo pharmacokinetic properties to drug-conjugated complexes by precisely controlling the type and molecular weight of the branched or cyclic polysaccharide, and/or the type and amount of crosslinker used in the formation of the polysaccharide-crosslinked colloidal particles.

Nanoparticles exhibit size-dependent organ-specific uptake and biodistribution in the human body. In particular, nanoparticles with a diameter of 50 nm or greater are rapidly sequestered by Kupffer cells in the liver, leading to their predominant accumulation in hepatic tissue.

However, even small nanoparticles may undergo unintended aggregation in the absence of adequate dispersibility, which can result in extensive clearance by the reticuloendothelial system (RES).

In this regard, in conjugates where drugs are modified with the polysaccharide-crosslinked colloidal particles according to the present invention, the colloidal particles expose hydrophilic functional groups on their surface to the aqueous environment. As a result, the nanoparticles do not aggregate in vitro or in vivo, and exhibit excellent dispersibility, colloidal stability, and/or storage stability. This leads to rapid and uniform distribution in the bloodstream and contributes to consistent therapeutic effects due to uniform particle size. Moreover, the particles maintain a uniform hydrodynamic diameter of 100 nm or less, which reduces phagocytic uptake by the hepatic reticuloendothelial system (RES) and thereby prolongs the circulation time in blood. In some cases, the particles may be engineered to avoid systemic accumulation and instead be excreted through renal filtration without undergoing hepatic metabolism.

For example, nanoparticles with a hydrodynamic diameter of approximately 1 to 30 nm may circulate in the blood for an extended period without being phagocytosed. Furthermore, to enable renal excretion via the kidneys, the particle diameter generally must be 6 to 8 nm or smaller. If the diameter exceeds this range, renal excretion becomes difficult. Therefore, when engineering the polysaccharide-crosslinked colloidal particles of the present invention for urinary excretion through the kidneys, the total hydrodynamic diameter may be designed to be 8 nm or less, preferably 6 nm or less, with a uniform size distribution.

Conversely, suppression of urinary excretion can increase the circulation time of the drug in the bloodstream, thereby allowing the drug concentration in the blood to remain elevated for an extended period. Additionally, it can enhance delivery efficiency to the reticuloendothelial system (RES), thereby improving drug delivery to RES-enriched organs such as the liver and spleen. In cases where the circulation time of the drug-modified polysaccharide-crosslinked colloidal particle in the bloodstream is increased, or delivery efficiency to the RES is enhanced, the drug-modified polysaccharide-crosslinked colloidal particle may be engineered to have a uniform size distribution with a total hydrodynamic diameter of 8 nm or greater.

The present invention allows the drug-modified polysaccharide-crosslinked colloidal particles to be designed and synthesized such that they maintain colloidal stability through hydration in biological fluids without undergoing opsonization. Moreover, the polysaccharide-crosslinked colloidal particles of the present invention may be engineered such that, within 1 hour after the release of the targeting molecule or drug, they are not degraded in vivo but are instead filtered through the vasculature to the kidney and collected in the bladder, enabling the majority of the particles to be excreted via urine. Accordingly, the particles are not only recyclable but may also be utilized to collect information from tissues at the administration site or from the bloodstream via adsorption or other means. This is based on the observation that the polysaccharide-crosslinked colloidal particles of the present invention exhibit excellent colloidal stability without aggregation, thereby maintaining a compact hydrodynamic diameter smaller than the renal filtration threshold and demonstrating superior excretion capacity in vivo. For example, the polysaccharide-crosslinked colloidal particles of the present invention can be precisely engineered to have an average hydrodynamic size of 10 nm or less, preferably 8 nm or less, while maintaining colloidal stability without aggregation in vivo, thus enabling complete renal excretion without structural alteration.

### 4. Surface Charge Modifier

The polysaccharide-crosslinked colloidal particles of the present invention are capable of modulating the surface charge of drugs.

The surface characteristics of the polysaccharide-crosslinked colloidal particles can be finely controlled by adjusting the type and/or density of surface-exposed functional groups. Accordingly, when used as multivalent linker systems or drug carriers, the surface properties of the resulting conjugates can be precisely engineered.

Zeta potential is an index of the repulsive forces between particles, and most aqueous colloidal systems are stabilized by electrostatic repulsion. The greater the repulsive force between particles, the less likely they are to approach each other and form aggregates. Because surface charge can prevent nanoparticle aggregation, nanoparticles having a zeta potential of ±10 mV or greater are generally considered stable in aqueous solutions. However, nanoparticles bearing high surface charge may exhibit reduced stability in electrolyte-rich solutions, such as biological fluids, due to ion-mediated aggregation. It has been reported that when amines (positively charged) and carboxylates (negatively charged) coexist on the surface, forming a zwitterionic state in which the net zeta potential is near neutral, the nanoparticles remain stable both in aqueous solutions and under physiological conditions such as in blood.

Furthermore, upon in vivo administration, nanomaterials interact with biomolecules such as proteins via electrostatic or non-specific binding mediated by their surface charge. Such interactions may result in the formation of protein coronas, which increases the likelihood of recognition by immune cells. Accordingly, the ability to modulate the surface charge of nanomaterials is crucial.

As described above, the polysaccharide-crosslinked colloidal particles of the present invention allow for precise control of surface charge by modulating the type and/or density of crosslinker-derived terminal functional groups exposed on the particle surface (see FIG. 2 of Korean Patent Application No. 10-2023-0016863).

For example, a combination of positively charged amine groups and negatively charged carboxylate groups can generate a zwitterionic surface state with near-neutral charge (see Examples 5 and 6 of Korean Patent Application No. 10-2023-0016863).

Within the range where the crosslinker-derived terminal functional groups do not cause toxicity and colloidal stability is maintained in biological fluids, the surface charge (zeta potential) of the polysaccharide-crosslinked colloidal particles may range from -30 mV to +10 mV.

Therefore, the present invention enables precise control over the molecular weight and/or surface charge of the polysaccharide-crosslinked colloidal particles used in multivalent linker systems or as drug carriers, thereby imparting the resulting conjugates with desirable pharmacokinetic properties in vivo.

In addition to aqueous stability, the surface charge of nanoparticles significantly affects their interaction with endogenous proteins. In general, nanoparticles larger than 30 nm administered intravenously are readily recognized by the immune system and subsequently cleared from the bloodstream via phagocytic uptake by macrophages. Even for smaller nanoparticles, those bearing excessive positive or negative surface charges may adsorb blood components, primarily serum proteins, thereby increasing their effective size and promoting clearance by phagocytes.

For example, in the conjugates of the present invention wherein the polysaccharide-crosslinked colloidal particles serve as a multivalent linker system or as a drug carrier, the surface charge of the conjugate may be governed by the surface charge of the polysaccharide-crosslinked colloidal particles. This surface charge significantly influences the pharmacokinetics and behavior of intravenously administered nanomaterials. Specifically, when serum proteins bind nonspecifically to the nanomaterial via electrostatic interactions-i.e., opsonization-nanomaterial-protein complexes are formed, which promote phagocytic uptake and organ accumulation via the mononuclear phagocyte system (MPS). Even if the original nanomaterials possess a size amenable to renal filtration, their association with proteins increases their hydrodynamic size beyond the renal clearance threshold, thereby preventing urinary excretion. To avoid such unintended organ accumulation and enable renal clearance, it is essential to impart anti-opsonization properties to the nanoparticles.

Accordingly, the conjugates comprising polysaccharide-crosslinked colloidal particles as multivalent linkers or drug carriers can be engineered such that their surface charge, derived from the colloidal particle, is finely tuned to fall within the range of -30 mV to +10 mV. This tuning minimizes non-specific adsorption of serum proteins and allows modulation of blood circulation time and renal excretion capability (see FIG. 11 of Korean Patent Application No. 10-2023-0016863).

The conjugates of the present invention may also be engineered to promote or evade opsonization by modulating the surface charge of the polysaccharide-crosslinked colloidal particles, which serve as multivalent linkers or drug carriers, such that the surface charge is positive, negative, or zwitterionic (i.e., exhibiting both positive and negative charges).

The desired surface charge can be achieved by modulating the crosslinker substitution ratio on the polysaccharide-crosslinked colloidal particles to be within 2% to 50% relative to the total number of functional groups on the polysaccharide. In addition, 2% to 98% of the crosslinkers may be designed such that one terminal group remains unreacted in the crosslinking process and is instead exposed on the surface of the particle.

### 5. Opsonization Inhibitors (Anti-Opsonization)

The polysaccharide-crosslinked colloidal particles used as multivalent linkers or drug carriers in the present invention may also function as opsonization inhibitors.

The surface charge of the polysaccharide-crosslinked colloidal particles of the present invention can be engineered to range from -30 mV to +10 mV, thereby preventing nonspecific binding to plasma proteins such as albumin.

To evaluate whether the surface charge modulation of the polysaccharide-crosslinked colloidal particles, which serve as multivalent linkers or drug carriers in the present invention, can effectively control actual binding with serum proteins-that is, the anti-opsonization effect-the in vivo administrable complexes of the present invention, which are surface-modified with the polysaccharide-crosslinked colloidal particles, were mixed with serum proteins (fetal bovine serum, FBS). Changes in hydrodynamic diameter or surface charge upon serum protein binding were assessed using electrophoresis. As shown in Examples 15 and FIG. 9 of Korean Patent Application No. 10-2023-0016863, depending on the type of polysaccharide-crosslinked colloidal particles exhibiting different surface charges, i.e., depending on the final surface charge, the prevention of serum protein binding was confirmed by analyzing electrophoretic band shifts and broadening.

Opsonins are phagocytosis-promoting factors present in serum, including immunoglobulins and other plasma proteins that bind to foreign particles and promote their uptake by phagocytes. Bacteria opsonized by such proteins are rapidly destroyed by macrophages.

When nanoparticles are exposed to actual in vivo environments-such as the bloodstream, interstitial fluid, or the extracellular matrix-proteins adsorb nonspecifically onto the nanoparticle surface, forming a "protein corona." This corona effect leads to opsonization, whereby proteins coat the nanoparticle surface, which ultimately determines the nanoparticles' apparent size, colloidal stability, surface characteristics, cellular uptake efficiency, in vivo biodistribution, intracellular localization, pharmacokinetics, tissue distribution, and toxicity.

Nanomedicines may be rapidly cleared from the bloodstream via the mononuclear phagocyte system (MPS), thus reducing or nullifying their therapeutic efficacy. The corona effect may also impair the targeting capability of conjugates in which a targeting moiety is bound to the nanoparticle surface, due to alterations in surface properties. Moreover, nanoparticle-protein interactions have been reported to induce hypersensitivity reactions in certain patients.

In contrast, the polysaccharide-crosslinked colloidal particles of the present invention may be used to modify drugs as opsonization inhibitors, thereby addressing the above-described issues by adjusting the final surface charge of conjugates in which the polysaccharide-crosslinked colloidal particles are used as multivalent linkers or drug carriers. For example, by combining surface-exposed functional groups derived from the crosslinker-such as amine groups imparting a positive charge and carboxyl groups imparting a negative charge-the polysaccharide-crosslinked colloidal particles of the present invention may be engineered to exhibit a zeta potential of 0 mV, and thus may serve as anti-opsonization modifiers for in vivo administration that inhibit phagocytic uptake by macrophages.

### 6. Blood Circulation Half-Life Regulator

The polysaccharide-crosslinked colloidal particles of the present invention may regulate the blood circulation half-life of a drug.

By adjusting the type of crosslinker and/or the number of surface-exposed functional groups derived from the crosslinker, the polysaccharide-crosslinked colloidal particles of the present invention can be used as multivalent linkers or drug carriers to prevent immune phagocytosis and/or regulate the circulation time in the bloodstream of the resulting conjugate.

The half-life of a nanodrug in systemic circulation is closely related to how quickly and efficiently it extravasates from the microvasculature into the target tissue microenvironment, and thus modulation of circulation time is critical for effective drug delivery. One of the most influential factors determining the circulation half-life of nanoparticles is nonspecific adsorption due to binding with plasma proteins. To reduce nonspecific binding with plasma proteins, polyethylene glycol (PEG) or PEGylation is commonly used. However, in the present invention, the conjugates incorporating polysaccharide-crosslinked colloidal particles as multivalent linkers or drug carriers can effectively suppress the corona effect, thereby reducing nonspecific interactions with the mononuclear phagocyte system (MPS).

### 7. Pharmacokinetic Modulator

The polysaccharide-crosslinked colloidal particles according to the present invention may serve to modulate the pharmacokinetic properties of a drug.

The conjugates incorporating the polysaccharide-crosslinked colloidal particles as multivalent linkers or drug carriers according to the present invention may be engineered to provide desired circulation time, biodistribution, or excretion profiles-such as renal clearance pharmacokinetics-by controlling the overall size and surface charge of the colloidal particles. This may be achieved by adjusting at least one parameter selected from: the molecular weight of the branched or cyclic polysaccharide, the length of the polysaccharide backbone, the type of crosslinker used during the crosslinking reaction, the amount and rate of crosslinker administration during synthesis, and any additional chemical modification of functional groups. In particular, the in vivo pharmacokinetic characteristics of conjugates incorporating the polysaccharide-crosslinked colloidal particles as multivalent linkers or drug carriers may be tailored by modulating the molecular weight and surface charge of the colloidal particles (see Example 17 and FIG. 11 of Korean Patent Application No. 10-2023-0016863).

Whether the conjugate undergoes phagocytosis by macrophages following in vivo administration, metabolic degradation, circulatory half-life, extravasation across capillary walls for parenchymal delivery, tissue accumulation, renal clearance into urine, systemic absorption into the vascular compartment following in vivo administration, or potential collection and reuse via urinary excretion-all of these can be controlled by adjusting the size and surface charge of the conjugate incorporating the polysaccharide-crosslinked colloidal particles as a multivalent linker system or drug carrier according to the present invention.

Furthermore, to achieve desired pharmacokinetic and pharmacodynamic profiles, the conjugates of the present invention may be designed in a variety of ways by combining control over particle size and surface charge with parameters such as the molecular weight of the polysaccharide, the length of its backbone, the type of crosslinker, the amount and rate of crosslinker addition during synthesis, and/or additional chemical modifications of the functional groups used in the polysaccharide-crosslinked colloidal particles.

### 8. Modulator for Avoidance of Phagocytosis by Macrophages

Through the control of the polysaccharide-crosslinked colloidal particles of the present invention, phagocytosis of drugs by macrophages can be avoided.

Most nanoparticulate drug formulations, such as synthetic nanocarriers, liposomes, and polymeric nanoparticles, face the limitation of being rapidly cleared from the bloodstream before reaching the diseased site due to phagocytosis by the reticuloendothelial system, a component of the body's immune system. The reticuloendothelial system is also referred to as the mononuclear phagocyte system (MPS).

According to the present invention, polysaccharide-crosslinked colloidal particles formed by intra- and/or inter-molecular crosslinking of one to three branched polysaccharides or two to thirty cyclic polysaccharides via hydroxyl groups of their monosaccharide building blocks using crosslinkers possess structural characteristics that allow them to be controlled by various modulatory factors such that they are neither phagocytosed by macrophages nor bound by opsonin proteins in the bloodstream. Accordingly, conjugates incorporating the polysaccharide-crosslinked colloidal particles as multivalent linker systems or drug carriers can be engineered to exhibit desired blood circulation and renal clearance profiles.

Because the polysaccharide-crosslinked colloidal particles of the present invention can be engineered to avoid phagocytosis by macrophages during in vivo administration, distribution, and elimination, the conjugates incorporating such particles as multivalent linker systems or drug carriers are not absorbed by macrophage-mediated phagocytosis in reticuloendothelial system organs such as the liver, spleen, bone marrow, and lymph nodes.

### 9. Modifier for Body Location Tracking for MRI

The polysaccharide-crosslinked colloidal particles of the present invention may be used as body location tracking modifiers for MRI imaging of drugs.

In a conjugate incorporating the polysaccharide-crosslinked colloidal particle as a multivalent linker system or drug carrier according to the present invention, (i) functional nanoparticles for in vivo administration, which are bound to surface-exposed reactive groups derived from crosslinkers, and/or (ii) the polysaccharide-crosslinked colloidal particles themselves may be engineered to function as MRI contrast agents.

Accordingly, conjugates incorporating the polysaccharide-crosslinked colloidal particle as a multivalent linker system or drug carrier, which are engineered to function as MRI contrast agents, can be used for positional tracking in magnetic resonance angiography (MR angiography), MR arthrography, MR cisternography, MR myelography, MR lymphangiography, MR cholangiopancreatography, or MRI-based imaging of the brain and abdomen.

The polysaccharide-crosslinked colloidal particles of the present invention may be engineered to function as T1 MRI contrast agents that produce bright signals on MRI images by coordinating divalent or trivalent iron ions to hydrophilic groups derived from crosslinkers exposed on the surface of a spherical core, which is formed by intramolecular and/or intermolecular crosslinking of 1 to 3 branched polysaccharides or 2 to 30 cyclic polysaccharides at hydroxyl groups of their monosaccharide building blocks using a crosslinker. Furthermore, the divalent or trivalent iron ions may remain exposed on the surface, facilitating access by water molecules and thereby accelerating proton relaxation of water molecules, which further enhances T1 MRI contrast effects.

Therefore, the iron-coordinated polysaccharide-crosslinked colloidal particles of the present invention, due to their characteristics as T1 MRI contrast agents, can provide imaging that visualizes their location when present in microvasculature, ureters, liver, spleen, articular cavities, spinal canal, and/or various organs in vivo. In addition, MRI may be used to determine whether the particles accumulate in tissue and/or extravasate through vascular walls. Accordingly, the iron-coordinated polysaccharide-crosslinked colloidal particles may serve as body location tracking agents.

The iron-coordinated polysaccharide-crosslinked colloidal particles of the present invention may also be engineered to allow MRI-based monitoring of their in vivo fate, including whether they are phagocytosed by macrophages, metabolically degraded, circulate in the bloodstream, are delivered to cellular parenchyma via capillary walls, accumulate in tissue, are excreted into urine via the kidneys or into feces, are absorbed into the vascular system after administration, extravasate through blood vessel walls, or are collectable via urine for potential reuse.

Moreover, the iron-coordinated polysaccharide-crosslinked colloidal particles of the present invention enable quality control of associated pharmaceutical compositions in animal studies by verifying, through in vivo biodistribution analyses based on the administration site and/or route, whether the compositions exhibit the intended pharmacokinetic behavior and whether they have been manufactured with uniform quality. Furthermore, the accumulated bioinformation may be used to precisely design in vivo administrable complexes tailored to patient characteristics (e.g., condition and/or medical history), disease type, route of administration, and pharmacological mechanism of a drug delivered via the polysaccharide-crosslinked colloidal particles of the present invention.

The iron-coordinated polysaccharide-crosslinked colloidal particles of the present invention, while functioning as contrast agents, may also be conjugated to drug carriers such as fragments, antibodies, aptamers, or artificial antibodies (e.g., repebodies) that include target antigen-binding domains. In such cases, the particles can be targeted to cells expressing the target antigen on their surface or to regions within the body that contain the target antigen, enabling visualization and quantification of such regions, as well as quantification of the distribution or concentration of the target antigen based on T1 MRI signal intensity. Furthermore, when an antibody specific to a tumor-associated antigen is used, enhanced therapeutic efficacy may be achieved, such as high tumor uptake and rapid clearance from healthy tissues.

### ADVANTAGEOUS EFFECTS

The present invention enables the design of various conjugates, such as antibody-drug conjugates (ADCs), to exhibit desired blood circulation time or desired in vivo biodistribution and excretion pharmacokinetics, by employing polysaccharide-crosslinked colloidal particles (e.g., CDex) as multivalent linker systems or drug carriers. These particles not only allow control over the overall size and surface charge but also can achieve a high drug-to-antibody ratio (DAR) of approximately 20 or more when desired. Furthermore, due to the abundance of hydrophilic functional groups exposed on the surface, they are highly hydratable and exhibit excellent colloidal stability. Such features also enable the conjugates to be designed for enhanced efficacy in a patient-specific manner.

### BRIEF DESCRIPTION OF THE DRAWINGS/FIGURES

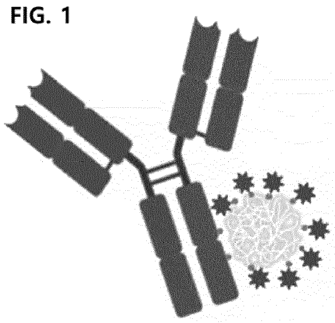

FIG. 1 is a conceptual diagram of a next-generation antibody-drug conjugate (ADC) utilizing dextran-crosslinked nanoparticles (CDex) developed according to the present invention.
FIG. 2 is a conceptual diagram illustrating the limitations of conventional ADC technology that the polyvalent linker system of the present invention aims to address.
FIG. 3 is a conceptual diagram showing that the CDex-based ADC linker technology according to the present invention, when used to replace conventional ADC linkers, enables a high drug-to-antibody ratio (DAR) and maintained colloidal stability, while also improving the efficacy and safety profile of ADCs.
FIG. 4(a) shows a schematic representation of the chemical conjugation between Cdex and NHS-Fluorescein; and (b) shows a graph demonstrating the control of the Fluorescein/Cdex ratio by adjusting the dextran molecular weight of the dextran backbone in Cdex and the Cdex:NHS-Fluorescein reaction ratio.
FIG. 5(a) shows a schematic diagram of the chemical conjugation of Cdex with bis-NHS and Doxorubicin (Dox); and (b) shows a graph illustrating the control of the number of Dox molecules conjugated per Cdex by adjusting the Cdex:Dox reaction ratio.
FIG. 6(a) shows the colloidal stability test result of Cdex-Fluorescein (demonstrating no change in hydrodynamic size for 24 hours in 1 M NaCl); and (b) shows the colloidal stability test result of Cdex-Doxorubicin (also demonstrating no change in hydrodynamic size for 24 hours in 1 M NaCl).
FIG. 7(a) shows a schematic representation of the cleavage of Cdex-DTSSP-Dox by dithiothreitol (DTT) or glutathione (GSH); (b) shows the UV-Vis absorption spectra of Cdex-DTSSP-Dox before and after DTT treatment; (c) shows the UV-Vis absorption spectra of Cdex-DTSSP-Dox before and after GSH treatment; and (d) shows the quantification of Dox/Cdex ratio before and DTT or GSH treatement.
FIG. 8 (a) shows the DAR as a function of the reaction ratio of Her2 Ab:Fluorescein; and (b) shows the DAR as a function of the reaction ratio of Her2 Ab:Cdex-Fluorescein.
FIG. 9 is a graph showing the colloidal stability test result of Her2 Ab-Cdex-Fluorescein.
FIG. 10 illustrates examples of mechanisms of various self-immolative spacers (source: https://njbio.com/linkers-for-adcs/).
FIG. 11(a) shows the three conjugation chemistries used to link a TLR agonist to Cdex; (b) shows the resulting chemical structures of Cdex@TLR prepared using each conjugation method; and (c) shows the ratio of TLR agonist conjugated per Cdex according to each conjugation chemistry.
FIG. 12(a) shows the hydrodynamic diameter of Cdex@TLR_{A1}; and (b) shows its colloidal stability in 1 M NaCl solution.
FIG. 13 shows immunofluorescence images of NFκB p65 signaling after 2-hour treatment in Examples 2-5.
FIG. 14 shows the results of Western blot analysis confirming NFκB p65 signaling after 4-hour treatment of TLR agonist in cells. G, R, A1, and B1 refer to Gardiquimod, Resiquimod, TLR_{A1}, and TLR_{B1}, respectively.

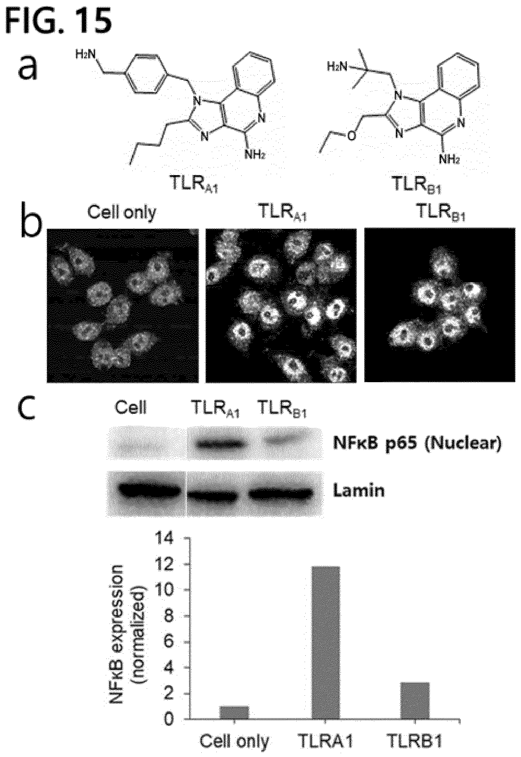

FIG. 15 illustrates a screening method for TLR agonists through TLR signal activation efficacy assays. (a) shows the molecular structures of TLR_{A1} and TLR_{B1} agonists; (b) shows NFκB expression confirmed by fluorescence staining; and (c) shows NFκB expression confirmed by Western blot analysis.
FIG. 16 shows immunofluorescence images of NFκB p65 signaling in cells after treatment with TLR agonists for 4 hours and 6 hours, respectively.
FIG. 17 shows the results of cytotoxicity assessment using CCK-8 after treatment with CDex, TLR_{A1} agonist, CDex@TLR_{A1}, and CDex@TLR_{B1} for 4 hours at varying concentrations.
FIG. 18 shows the experimental design (a) and results (b) of an in vivo efficacy evaluation conducted in a xenograft mouse model treated with control, Doxorubicin, TLR_{A1} agonist, Cdex@TLR_{A1}, Cdex@TLR_{A1}X2 (double dose), and Cdex@TLR_{B1}.
FIG. 19 shows the structural formulas of exemplary branched polysaccharides or cyclic polysaccharides that are suitable for use in the polysaccharide-crosslinked colloidal particles in Example 3.
FIG. 20 illustrates confirmation of the formation of crosslinked structures using a UV-Vis spectrophotometer for Dextran T-5 (a), Maltodextrin (b), α-cyclodextrin (c), β-cyclodextrin (d), and Inulin (e) in Examples 3-1 to 3-5.
FIG. 21 shows MRI phantom images of materials synthesized by introducing iron into the crosslinked structures of Dextran T-5 (a), Maltodextrin (b), α-cyclodextrin (c), β-cyclodextrin (d), and Inulin (e) in Example 3-10 and their *r₁* and *r₂* relaxivity values (f).
FIG. 22 shows T1-weighted MRI images obtained from mice intravenously injected with materials synthesized by introducing iron into crosslinked structures synthesized from Dextran T-5, Maltodextrin, α-cyclodextrin, β-cyclodextrin and Inulin in Example 3-11 (a) and signal-to-noise ratios (SNRs) before and after administration (b).
FIG. 23 shows T1-weighted MRI images obtained from mice intravenously injected with materials synthesized by introducing iron into crosslinked structures synthesized from Dextran T-5, Maltodextrin, α-cyclodextrin, β-cyclodextrin, and Inulin in Example 3-12, thereby confirming renal excretion.
FIG. 24 compares the structure (a), MRI phantom image (b), and relaxivity (d) of the dextran-crosslinked nanoparticle-based T1 MRI contrast agent NEMO-103 of Example 4-2 with those of a representative clinical gadolinium-based contrast agent (GBCA), Dotarem (c, e).
FIG. 25 shows the imaging phase timeline of magnetic resonance arthrography (MRA) used in the clinical comparison study between the dextran-crosslinked T1 MRI contrast agent NEMO-103 (iron-based contrast agent, IBCA) and GBCA.
FIG. 26 illustrates the clinical comparison study protocol between NEMO-103 and GBCA in Example 4-3.
FIG. 27 shows MRA images captured at 30 minutes (a) and 60 minutes (b) after administration of NEMO-103 in humans, and a comparative analysis in terms of contrast-to-noise ratio (CNR) (c), distension (d), and overall image quality (e).
FIG. 28 shows MRA images captured at 30 minutes after administration of NEMO-103 (a) and a gadolinium-based contrast agent (GBCA, b) in humans, with comparative evaluation in terms of CNR (c), distension (d), and overall image quality (e).
FIG. 29 shows MRA images captured at 60 minutes after administration of NEMO-103 (a, c) and GBCA (b, e) in humans, with comparative assessment of CNR (c), distension (d), and overall image quality (e).

### Mode for carrying out the invention

Hereinafter, the present invention will be described in further detail with reference to specific examples. However, these examples are provided for the purpose of illustrating the technical features of the invention more clearly and are not intended to limit the scope of the invention in any way.

### Example 1

### 1-1. Control of the Number of Conjugatable Functional Groups per Cdex Linker for High DAR

To 1 g of dextran (molecular weight: 5 kDa) dissolved in 4.2 mL of ultrapure water was added 8.3 mL of 2.5 M NaOH solution. Subsequently, 3.3 mL of epichlorohydrin was added and the mixture was stirred. Then, 7 mL of diethylenetriamine (DETA) was added, and the reaction mixture was further stirred for 24 hours, followed by purification via ultrafiltration. The resulting dextran-crosslinked particle (Cdex-5) had a molecular weight of 15 kDa and a hydrodynamic diameter of 3 nm as measured by DLS. The number of amine groups per Cdex, determined using the o-phthalaldehyde (OPA) amine assay, was 12 (see Table 4).

When dextran with a molecular weight of 10 kDa was used instead of 5 kDa dextran, under otherwise identical conditions, the resulting dextran-crosslinked particle (Cdex-10) had a molecular weight of 30 kDa, a hydrodynamic diameter of 4 nm, and up to 35 amine groups per Cdex as determined by the OPA assay (see Table 4).

These results demonstrate that the number of functional groups on Cdex can be controlled by adjusting the molecular weight of the dextran used in the reaction.

**[Table 4]**

| | MW of dextran | MW of Cdex | Hydrodynamic size | No. of amine group/Cdex |
|---|---|---|---|---|
| Cdex-5 | 5 kDa | 10 kDa | 3 nm | Up to 12 |
| Cdex-10 | 10 kDa | 30 kDa | 4 nm | Up to 35 |

As shown in Table 4, the number of amine groups on dextran-crosslinked particles (Cdex) can be controlled by adjusting the molecular weight of the starting dextran. When Cdex is used as a linker system for ADCs (antibody-drug conjugates), a higher number of conjugatable functional groups is advantages.

That is, the maximum number of amine groups on Cdex can be tuned by selecting the appropriate molecular weight of dextran during synthesis. For example, when 5 kDa dextran was used, the number of amine groups was 12; when 10 kDa dextran was used, the number increased to 35.

### 1-2. Introduction of Various Types of Functional Groups

In order for the Cdex-based linker system of the present invention to be universally applicable as a linker in antibody-drug conjugate (ADC) platform technologies, it must allow the introduction of various types of functional groups. Specifically, different functional groups are required for Ab-linker or Ab-drug conjugation. As demonstrated below, the introduction of amine, carboxyl, thiol, and hydroxy groups was achieved.

Amine group: The surface functional groups on the Cdex synthesized in Example 1-1 were amine groups.

Carboxyl group: To 10 mL of the Cdex synthesized in Example 1-1 was added 30 mg of succinyl anhydride (SA), and the mixture was stirred for 12 hours. The final product was purified by ultrafiltration.

Thiol group: To 10 mL of the Cdex synthesized in Example 1-1 was added N-succinimidyl S-acetylthioacetate, and the mixture was stirred for 1 hour. N-hydroxyacetamide was then added, and the mixture was further stirred for 12 hours. The final product was purified by ultrafiltration.

Hydroxy group: To 10 mL of the Cdex synthesized in Example 1-1 was added nitrous acid, and the mixture was stirred for 12 hours. The final product was purified by ultrafiltration.

### 1-3. Modulation of the Number of Drugs Conjugatable to Cdex (1): Fluorescent Organic Molecules with Drug-like Structures

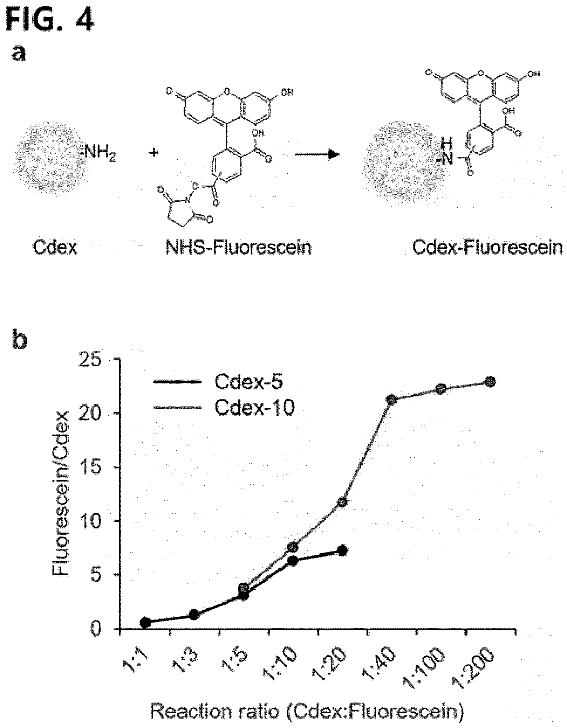

1 mg of Cdex-5 synthesized in Example 1-1 was added to 600 µL of 100 mM phosphate buffer. NHS-Fluorescein was added in amounts of 47.3, 142, 236.7, 473.4, and 946.8 µg, respectively, followed by stirring for 30 minutes (FIG. 4a). The resulting molar ratios of Cdex-5:NHS-Fluorescein were 1:1, 1:3, 1:5, 1:10, and 1:20, respectively. The resulting Cdex-Fluorescein conjugates were purified using a size exclusion column. As shown in FIG. 4b, the number of fluorescein molecules bound per Cdex-5 particle, as measured by UV-visible spectrophotometry, was 0.6, 1.2, 3.1, 6.3, and 7.2 at the respective ratios (black bars, FIG. 4b). When Cdex-10 was used instead of Cdex-5, and the Cdex-10:NHS-Fluorescein reaction ratios were adjusted to 1:5, 1:10, 1:20, 1:40, 1:100, and 1:200, the number of fluorescein molecules bound per Cdex-10 particle was 3.7, 7.5, 12.9, 21.9, 22.2, and 22.9, respectively (purple bars, FIG. 4b). These results indicate that the amount of drug conjugated to Cdex can be modulated by adjusting the molecular weight of dextran and the reaction ratio with the drug.

In summary, Example 1-3 demonstrates that conjugation of drugs to Cdex is feasible via the amine groups on Cdex. Fluorescein, which exhibits a molecular structure similar to common anticancer drugs and emits fluorescence, was conjugated to Cdex, and the number of conjugated molecules was controlled. NHS-Fluorescein, containing an NHS moiety reactive to amine groups, was used to demonstrate this concept of controlling the number of drug molecules.

Accordingly, two methods can be employed to modulate the number of fluorescein molecules conjugated per Cdex particle:
(1) Adjusting the number of amine groups per Cdex by varying the molecular weight of dextran: up to 35 amines/Cdex; and
(2) Adjusting the reaction ratio of Cdex:NHS-Fluorescein: up to 23 fluorescein molecules/Cdex.

### 1-4. Modulation of the Number of Drugs Conjugatable to Cdex (2): Anticancer Drug

Doxorubicin is an anticancer drug containing an amine group and can be conjugated to the amine groups on the surface of Cdex via bis-NHS molecules.

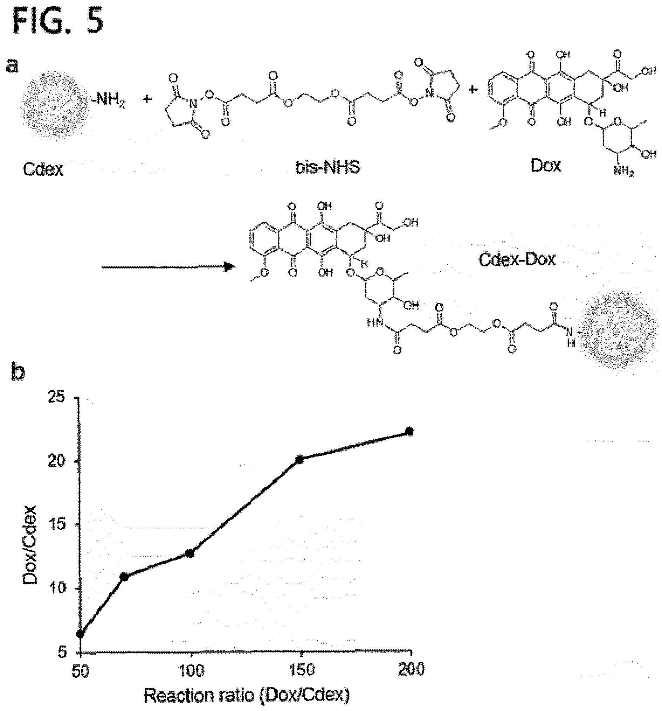

1 mg of Cdex-10 synthesized in Example 1-1 was added to 750 µL of 100 mM phosphate buffer, followed by the addition of 2.3 mg of bis-NHS (ethylene glycol-bis(succinic acid N-hydroxysuccinimide ester)), and the mixture was reacted for 30 minutes (FIG. 5a). The resulting Cdex-NHS was purified by ultrafiltration. To 110 µg of the purified Cdex-NHS was added 16.1, 22.5, 32.2, 48.3, or 64.4 µg of doxorubicin (Dox), followed by reaction for 3 hours in 50% DMSO solution. The reaction ratios of Cdex-NHS:Dox were 1:50, 1:70, 1:100, and 1:150, respectively. The resulting Cdex-Dox conjugates were subjected to dialysis in 50% DMSO for 12 hours. As measured by UV-visible absorption spectroscopy, the number of doxorubicin molecules conjugated per Cdex particle (Dox/Cdex) at the respective reaction ratios was 6.4, 10.9, 12.7, 18.5, and 22.1 (FIG. 5b). These results demonstrate that the amount of drug conjugated to Cdex can be modulated by adjusting the reaction ratio between Cdex and the drug.

This example shows that doxorubicin, a representative anticancer agent, can be conjugated to Cdex, and the number of conjugated molecules can be controlled. Similar to Example 1-3, the number of doxorubicin molecules per Cdex-10 particle (Dox/Cdex) could be modulated up to 22 by adjusting the Cdex:Dox reaction ratio.

### 1-5. Excellent Colloidal Stability of Drug-Conjugated Cdex

One of the current issues in ADC technology is the poor colloidal stability in blood, which stems from the inherently low colloidal stability of conventional ADCs. Such low colloidal stability can lead to aggregation upon in vivo administration, thereby accelerating plasma clearance by the liver and significantly reducing drug delivery efficiency to the target site. This phenomenon arises because both the drug and the linker used in ADCs are typically hydrophobic, and since modifying the drug structure is challenging, the problem can be addressed by maximizing the aqueous dispersibility of the linker.

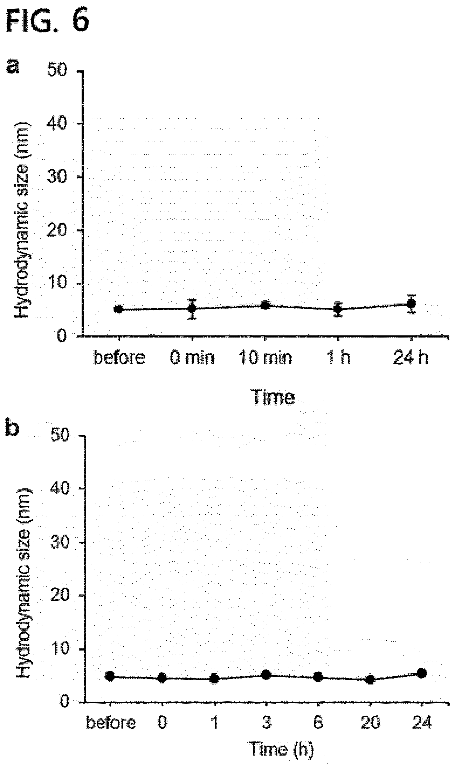

To evaluate this, 80 µg of each of the Cdex-Fluorescein and Cdex-Doxorubicin compounds synthesized in Examples 1-3 and 1-4, respectively, were dispersed in 1 M NaCl solution, and their hydrodynamic diameters were monitored for 24 hours using dynamic light scattering (DLS). It was confirmed that both Cdex-Fluorescein (FIG. 6a) and Cdex-Doxorubicin (FIG. 6b) maintained the same hydrodynamic diameter (5 nm) as the unconjugated Cdex over 24 hours.

Given that the physiological salt concentration in vivo is approximately 150 mM, the above dispersibility was further tested under a more extreme condition of 1,000 mM to verify salt stability.

These results demonstrate that the excellent salt stability of Cdex allows it to maintain superior colloidal stability even when various drugs (a. Fluorescein, b. Doxorubicin) are conjugated thereto.

### 1-6. Drug Release via Introduction of a Cleavable Linker

One of the core technologies in ADCs is the controlled release of a drug at a desired location (e.g., inside a target cell).

A representative example is the disulfide bond, which is cleaved in intracellular environments such as those found in lysosomes (e.g., due to glutathione (GSH) or low pH), making it suitable for use as a cleavable linker in ADCs.

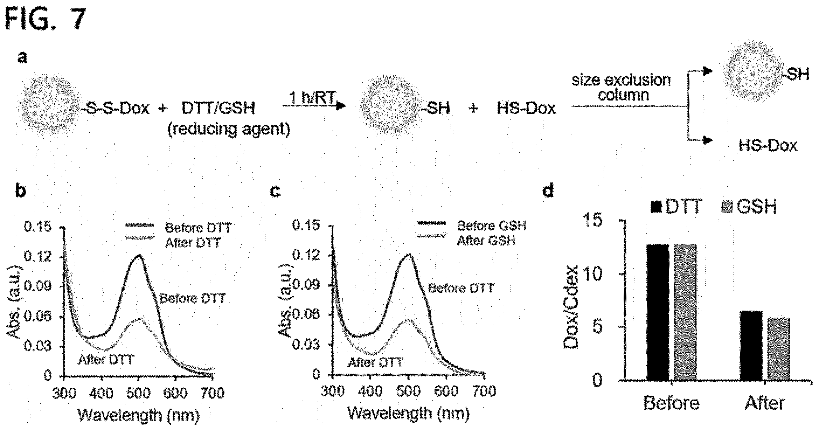

This example demonstrates the concept of intracellular environment-triggered drug release by introducing DTSSP (3,3'-dithiobis(sulfosuccinimidyl propionate)), a molecule containing a disulfide bond, between Cdex and doxorubicin (FIG. 7a).

Specifically, 1 mg of Cdex-10 synthesized in Example 1-1 was added to 750 µL of 100 mM phosphate buffer, followed by the addition of 2.03 mg of DTSSP and stirring for 30 minutes. The resulting Cdex-S-S-NHS was purified by ultrafiltration. Then, 32.2 µg of doxorubicin (Dox) was added to 110 µg of the purified Cdex-S-S-NHS, followed by stirring for 3 hours. The synthesized Cdex-S-S-Dox was subjected to dialysis in 50% DMSO for 12 hours. When the Cdex-S-S-Dox solution was mixed 1:1 (v/v) with 20 mM DTT (1,4-dithiothreitol) or GSH solution, and subsequently purified using a size exclusion column, it was separated into Dox-SH and Cdex-SH (FIG. 7a). UV-visible absorption spectra measured before and after treatment with DTT (FIG. 7b) and GSH (FIG. 7c) revealed a significant decrease in absorbance in the DTT- and GSH-treated groups (yellow), confirming the cleavage of the Dox-SH from Cdex. Quantitative analysis showed that upon treatment of Cdex-S-S-Dox with DTT or GSH, 6 (50%) and 7 (60%) of the 12 Dox molecules conjugated to the surface of Cdex, respectively, were released. These findings confirm that the incorporation of disulfide bonds allows for the controlled release of drugs from Cdex.

### 1-7. Conjugation of Drug-Conjugated Cdex to Antibodies (Enabling High DAR)

This example demonstrates that a drug (fluorescein)-conjugated Cdex can be directly conjugated to an antibody, and the resulting drug-to-antibody ratio (DAR) was evaluated. For comparison, a control group was prepared in which the drug was directly conjugated to the antibody.

As the antibody, an anti-Her2 IgG, which targets breast cancer cells, was used. The drug was NHS-fluorescein, which can be conjugated to the amine groups of the antibody, allowing for DAR measurement.

In the test group, the remaining amine groups on the surface of Cdex-Fluorescein (with a Fluorescein/Cdex ratio of 23) were conjugated to the amine groups of the antibody using a bis-NHS crosslinker.

### 1-7-1. Synthesis of Her2 Ab-Fluorescein:

31.5 µg of Her2 Ab was added to 80 µL of 100 mM phosphate buffer, followed by the addition of 8.07, 16.14, or 32.28 µg of NHS-fluorescein. The reaction was allowed to proceed for 3 hours. The corresponding reaction molar ratios were 1:75, 1:150, and 1:300, respectively. The resulting Her2 Ab-Fluorescein conjugates were purified by ultrafiltration, and the amount of fluorescein conjugated per Her2 Ab molecule (DAR) was determined using UV-visible spectroscopy. The measured DARs for the respective reaction conditions were 2.8, 5.3, and 6.5 (FIG. 8a).

### 1-7-2. Synthesis of Her2 Ab-Cdex-Fluorescein:

To 1 mg of Cdex-Fluorescein (Fluorescein/Cdex = 23, synthesized in Example 1-3), 3 mg of bis-NHS was added and reacted for 30 minutes. The resulting NHS-Cdex-Fluorescein was purified via ultrafiltration. Then, 102, 255, or 510 µg of Her2 Ab was added to the purified NHS-Cdex-Fluorescein and reacted for 3 hours. The corresponding reaction molar ratios were 1:20, 1:50, and 1:100, respectively. The resulting Her2 Ab-Cdex-Fluorescein conjugates were purified by ultrafiltration, and the amounts of Cdex and fluorescein conjugated per Her2 Ab were quantified using UV-visible spectroscopy. The number of Cdex per Her2 Ab molecule was determined to be 2.1, 3.0, and 3.6, respectively, and the corresponding DARs were 48.5, 68.5, and 82.1 (FIG. 8b).

These results demonstrate that when a drug is conjugated to an antibody via Cdex, it is possible to attach over 10 times more drug molecules compared to conventional conjugation methods.

In summary, in this example, when fluorescein was directly conjugated to the Her2 antibody (Her2 antibody-fluorescein direct conjugates), the maximum number of drug molecules conjugated was 6.5. In contrast, using drug-conjugated Cdex, up to 3.6 Cdex nanoparticles could be conjugated to each antibody, which translates to a DAR of 82. Therefore, this method results in a DAR enhancement of more than 10-fold compared to conventional conjugation techniques.

### 1-8. Excellent Colloidal Stability of Antibody-Cdex-Drug Conjugates

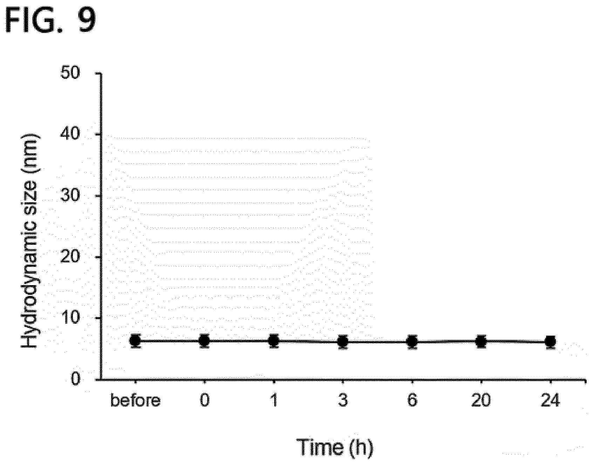

The Her2 antibody conjugated with drug-conjugated Cdex (containing 23 drug molecules per Cdex), synthesized in Example 1-7, was dispersed in 1 M NaCl, and its hydrodynamic diameter was measured over 24 hours to evaluate colloidal stability. The initial hydrodynamic diameter was measured at 7 nm, and no significant change in size was observed during the 24-hour period (FIG. 9). This indicates that even at a DAR exceeding 80, colloidal stability is maintained, demonstrating that high drug loading and excellent colloidal stability can be simultaneously achieved.

### 1-9. In Vitro Efficacy Testing

To demonstrate superior efficacy at the cellular level, an antibody-drug conjugate (ADC) comprising Cdex-doxorubicin conjugated to a Her2 antibody was tested and compared with control groups.

More specifically, the Her2 antibody conjugated with Cdex-doxorubicin was applied to breast cancer cells to evaluate efficacy.

### 1-9-1. Targeting Test:

Both Her2-positive and Her2-negative breast cancer cell lines were used to confirm selective binding to the Her2-positive cells.

### 1-9-2. Drug Release Test:

Various cleavable linkers, as shown in FIG. 10, can be used.

In this test, both a cleavable linker (DTSSP) and a non-cleavable linker (bis-NHS) were used for conjugating the drug to Cdex. The cleavable linker is expected to result in enhanced therapeutic efficacy due to intracellular drug release.

### 1-9-3. Efficacy Confirmation:

Compared to conventional methods (e.g., Her2 antibody-doxorubicin direct conjugates), the Cdex-based ADC shows superior efficacy attributable to its higher DAR, highlighting the therapeutic advantage of increased drug loading.

### Example 2. Polysaccharide Crosslinked Colloidal Particle-Drug Conjugate (Cdex@TLR as an Anticancer Agent)

### 2-1. Preparation of Nanostructure (Cdex) Using Dextran T-10

180 µmol of dextran T-10 (average molecular weight of 10,000 Da) was dissolved in 9 mL of distilled water. To this solution, 75 mmol of epichlorohydrin and 75 mmol of NaOH were added. Subsequently, 380 mmol of ethylenediamine was added, and the reaction mixture was stirred at room temperature (RT) for 24 hours. Then, 25 mg of succinic anhydride was added to induce succinylation at room temperature for another 24 hours. The resulting product was purified by dialysis using a 10 kDa molecular weight cutoff (MWCO) filter.

### 2-2. Introduction of a Drug Capable of Conjugation to Cdex (TLR7/8 Agonist)

Various types of TLR7 agonists are currently commercially available. Among them, two TLR7 agonists (TLR_{A1} and TLR_{B1}) were selected that contain an amine group capable of forming a bond with the crosslinker-derived amine groups exposed on the surface of the Cdex synthesized in Example 2-1, while not interfering with the chemical site necessary for TLR7 binding.

As illustrated in FIG. 11, Cdex and the selected TLR agonists were conjugated via chemical coupling strategies using (i) EDC (1-ethyl-3-(3-dimethylaminopropyl)carbodiimide), (ii) NHS (N-hydroxysuccinimide)-PEG (polyethylene glycol)-NHS, and (iii) BCN (bicyclo[6.1.0]nonyne)-NHS / N₃ (azide)-NHS (FIGS. 11a and 11b).

For the chemical coupling using N₃-PEG-NHS, 30 mg of N₃-PEG-NHS was dissolved in 2 mL of DMSO and added to the Cdex material from Example 2-1. The reaction mixture was stirred at room temperature for 2 hours. Then, 10 mg of BCN-NHS and 10 mg of TLR_{A1} agonist were mixed and dissolved in 2 mL of DMSO, followed by addition to the reaction solution and stirring for another 2 hours. The final product was purified via dialysis using a 10 kDa MWCO filter. For the chemical coupling using NHS-PEG-NHS, 10 mg of TLR_{A1} agonist and 20 mg of NHS-PEG-NHS were added directly to the Cdex material, followed by stirring for 2 hours and purification by dialysis using a 10 kDa MWCO filter.

As a result, the number of TLR_{A1} agonist molecules conjugated to Cdex was found to be 3 for the NHS-BCN/N₃-NHS coupling method, and 0.45 for the NHS-PEG-NHS method. Thus, the NHS-BCN/N₃-NHS coupling strategy was determined to be the most efficient in terms of chemical conjugation efficiency (FIG. 11c).

### 2-3. Quantification of Drug Conjugated to Cdex

The material prepared in Example 2-2 was diluted to a concentration of 0.5 µg/mL and placed in a cuvette. The absorbance of the material was measured across the wavelength range of 200 to 500 nm using a UV-Vis spectrophotometer. While Cdex without conjugated drug showed no absorption at 350 nm, drug-conjugated Cdex exhibited absorbance at 350 nm. Based on this result, the amount of drug conjugated to Cdex was quantified, and the number of TLR agonist molecules conjugated per mole of Cdex was determined. It was confirmed that more than three TLR agonist molecules were conjugated to each Cdex (FIG. 11c).

### 2-4. Hydrodynamic Size and Colloidal Stability of Cdex@TLR

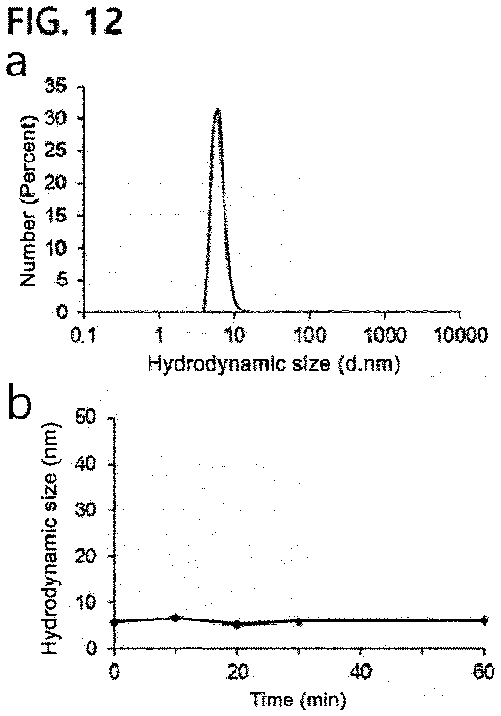

As shown in FIG. 12, the Cdex conjugated with TLR agonists as prepared in Example 2-2 exhibited a hydrodynamic size of 6 nm (FIG. 12a). It was further confirmed that the particles remained stably dispersed without aggregation even in 1 M NaCl solution-a condition significantly harsher than general physiological environments-indicating excellent colloidal stability (FIG. 12b).

### 2-5. In Vitro Efficacy Evaluation of Cdex@TLR (Assessment of NF-κB p65 Signaling)

In the context of Toll-like receptors (TLRs), the efficacy of the polysaccharide-crosslinked colloidal particle of the present invention as a drug delivery system can be evaluated by examining whether it induces the TLR signaling pathway (NF-κB signaling) in cells expressing TLRs.

To confirm whether NF-κB signaling was activated via TLR signaling upon treatment with Cdex@TLR from Example 2-2, the representative immune cell line RAW 264.7 (a murine macrophage cell line) was used.

Specifically, two samples-Cdex@TLR_{A1} and Cdex@TLR_{B1}-prepared in Example 2-2, along with control groups (non-conjugated Cdex and free TLR7/8 agonists: TLR_{A1} and TLR_{B1}), were applied to mouse RAW 264.7 macrophage cells. The induction of NF-κB p65 signaling was assessed by immunofluorescence (IF) staining under the treatment concentrations and durations listed in Table 5.

**[Table 5]**

| Treatment Group | Sample Name | Treatment Concentration (µM) | Treatment Duration (h) | Detection Method |
|---|---|---|---|---|
| Control | Untreated | - | 2, 4, 6 | IF |
| | Cdex | 2.0, 3.0 | | |
| TLR 7/8 Agonist | TLR_{A1} agonist | 6.0, 9.0 | | |
| | TLR_{B1} agonist | | | |
| Cdex@TLR | Cdex@TLR_{A1} | | | |
| | Cdex@TLR_{B1} | | | |

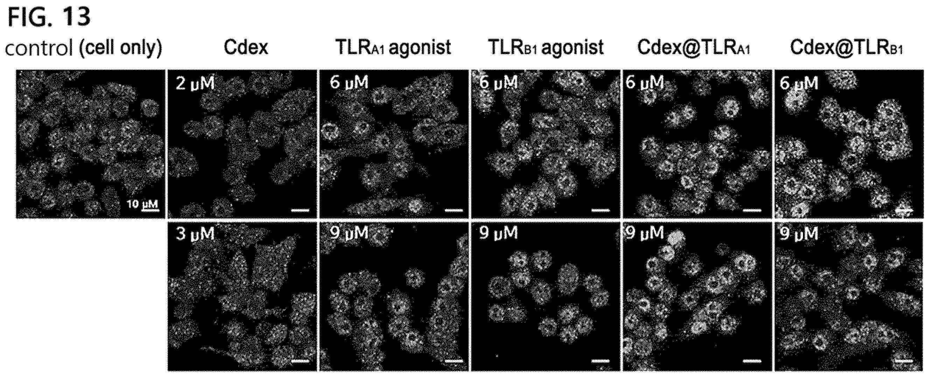

As shown in FIG. 13, upon 2-hour treatment and subsequent NF-κB p65 immunofluorescence staining, control groups (cell only, Cdex) exhibited diffuse fluorescence throughout the cytoplasm. In contrast, the TLR 7/8 agonist groups (TLR_{A1} agonist, TLR_{B1} agonist) and the conjugated forms (Cdex@TLR_{A1}, Cdex@TLR_{B1}) showed strong NF-κB p65 fluorescence predominantly localized within the nuclei rather than the cytoplasm. It is well-established that activation of the TLR signaling pathway leads to nuclear translocation of NF-κB p65, resulting in stronger nuclear fluorescence signals. Furthermore, the nuclear NF-κB p65 fluorescence intensity was stronger in the Cdex@TLR_{A1} and Cdex@TLR_{B1} groups than in the free agonist groups (TLR_{A1}, TLR_{B1}), indicating that the conjugated forms more effectively activated the TLR signaling pathway. A comparison of fluorescence intensity at different treatment concentrations showed that the NF-κB p65 nuclear fluorescence was similar at both 6 µM and 9 µM.

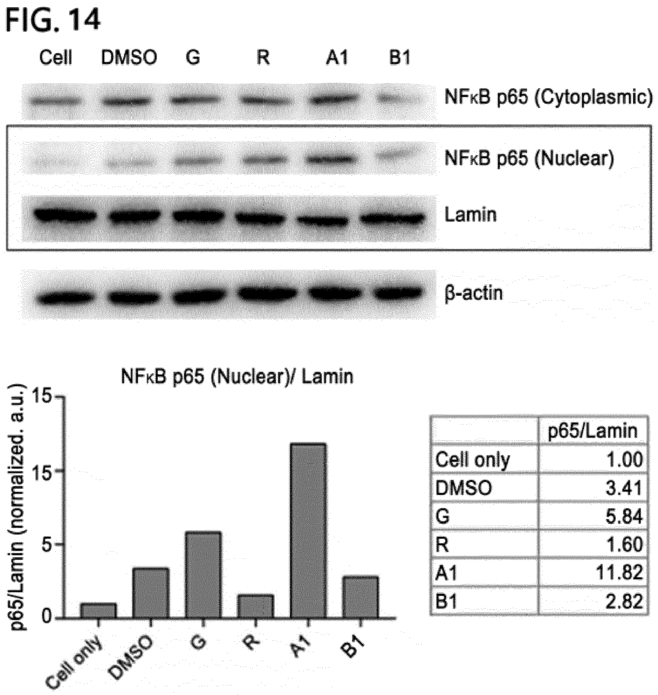

Among various TLR 7/8 agonists, the most effective compound for inducing NF-κB p65 nuclear translocation was identified by measuring expression levels after cellular treatment. Gardiquimod, Resiquimod, TLR_{A1}, and TLR_{B1} agonists were each applied at 6 µM to mouse RAW 264.7 macrophage cells for 4 hours. After treatment, cell lysates were fractionated into cytoplasmic and nuclear portions, and NF-κB p65 levels were assessed via Western blotting (FIG. 14). Lamin (nuclear marker) and β-actin (cytoplasmic marker) were used as housekeeping proteins for normalization. The results revealed that the TLR_{A1} agonist induced the highest NF-κB p65 expression in the nuclear fraction. In contrast, Resiquimod and TLR_{B1} agonist showed relatively lower nuclear NF-κB p65 expression, especially when compared to the DMSO vehicle control.

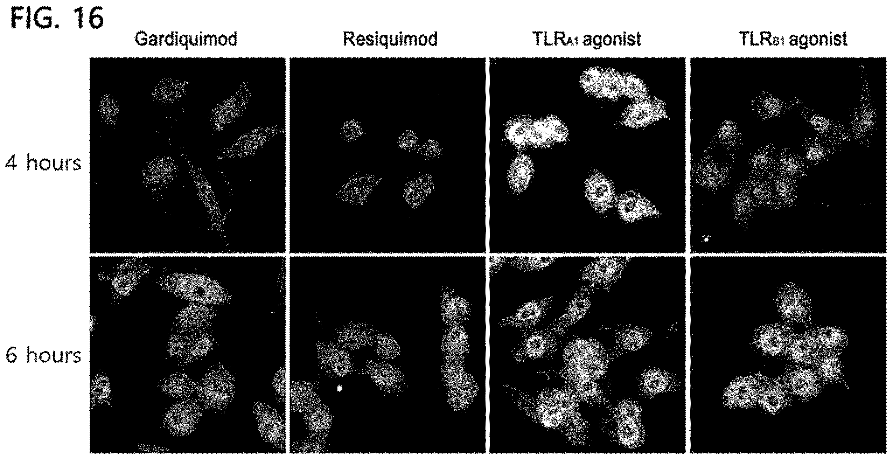

In a similar experimental setup, Gardiquimod, Resiquimod, TLR_{A1}, and TLR_{B1} agonists were each applied at 6 µM to RAW 264.7 cells for 4 and 6 hours. NF-κB p65 activation was assessed by immunofluorescence staining (FIG. 16). Among all tested agonists, TLR_{A1} agonist showed the highest nuclear **NF-κB** p65 expression after 4-hour treatment, indicating that it was the most potent activator of TLR signaling in vitro.

### Example 2-6: In Vitro Cytotoxicity Evaluation of Cdex@TLR

Two types of ADC platforms, Cdex@TLR_{A1} and Cdex@TLR_{B1}, which demonstrated efficacy, along with control groups including Cdex and TLR 7/8 agonists (TLR_{A1}, TLR_{B1} agonist), were evaluated for cytotoxicity across various treatment concentrations and time points. The evaluation was conducted using a mouse RAW 264.7 macrophage cell line known to express Toll-like receptors (TLRs).

Specifically, the ADC platforms were categorized into untreated control, low, medium, and high concentrations, and subsequently applied to cells. Cell viability was assessed using the CCK-8 (Cell Counting Kit-8) assay to determine cytotoxic effects based on concentration and exposure time.

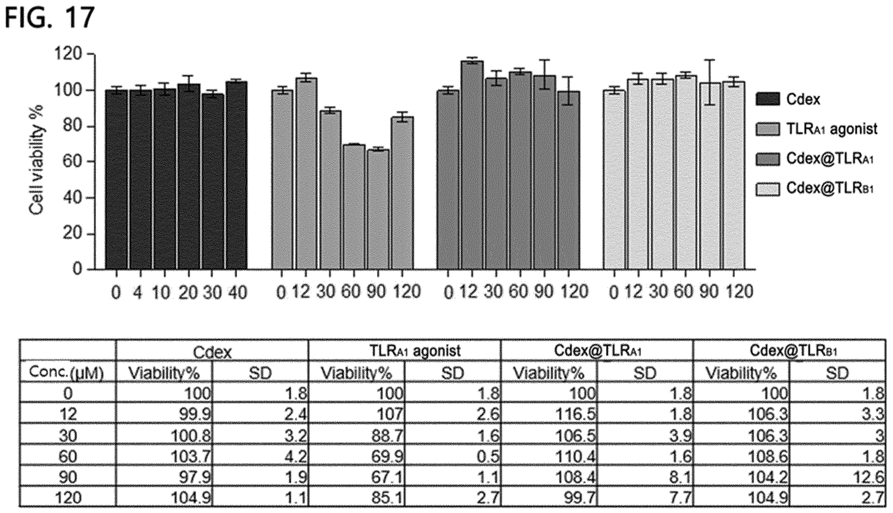

After 4 hours of treatment, CCK-8 assay results showed that Cdex alone exhibited no cytotoxicity at the tested concentrations. In contrast, TLR_{A1} agonist showed reduced cell viability, with viability dropping to approximately 89% at a concentration of 30 µM. However, both Cdex@TLR_{A1} and Cdex@TLR_{B1} demonstrated no cytotoxicity across all tested concentrations (see FIG. 17).

### Example 2-7: In Vivo Antitumor Efficacy Assessment in a Cancer Xenograft Mouse Model

The antitumor efficacy of the drug was assessed by monitoring changes in tumor volume following administration. Cdex@TLR_{A1} and Cdex@TLR_{B1} were administered in a cancer xenograft mouse model to evaluate antitumor immune responses and subsequent tumor cell death.

Balb/c nude mice (6 weeks old) were used to establish a BT-474 human breast cancer xenograft model for in vivo efficacy evaluation. Approximately 1 × 10⁶ BT-474 breast cancer cells were mixed with Matrigel at a 1:1 volume ratio and subcutaneously implanted into the dorsal flank of each mouse in a volume of approximately 100 µL.

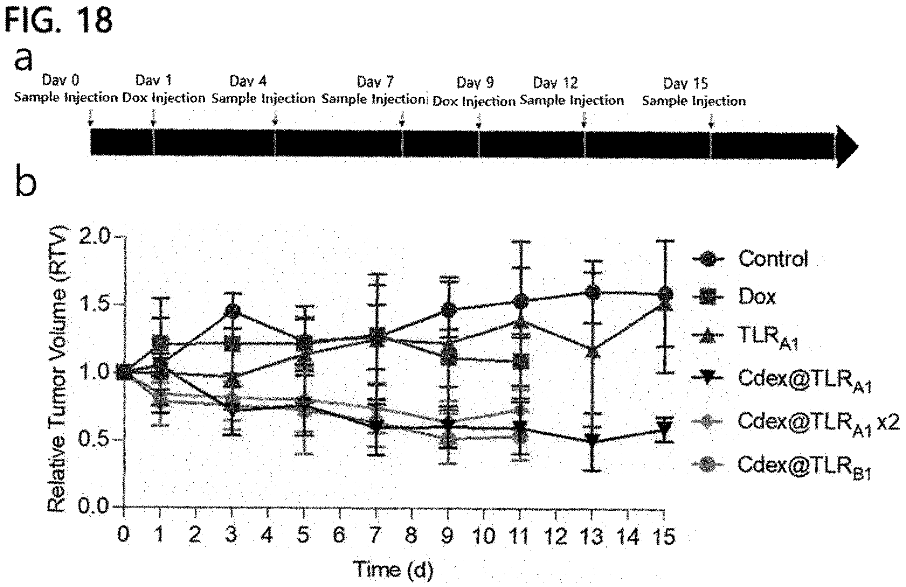

Once tumor volumes reached approximately 50 mm³, Cdex@TLR_{A1} and Cdex@TLR_{B1} were directly injected into the tumor masses, and tumor growth was monitored over a 2-week period to evaluate drug efficacy. The test substances were administered on days 0, 4, 7, 12, and 15 (see FIG. 18a). To enhance antitumor effects, doxorubicin was administered to all groups except the negative control group on days 1 and 9. A separate "doxorubicin only" group was included for comparative purposes (see FIG. 18b and Table 6).

**[Table 6]**

| group | Sample Name | TLR/Cdex Ratio | Dose Concentration | Dose Volume (µL) | Number of Animals |
|---|---|---|---|---|---|
| 1 | Control (1XPBS) | | - | 20 | 3 |
| 2 | Doxorubicin (Dox) only | | 2 mg/kg | 20 | 4 |
| 3 | TLR_{A1} agonist | | 6 nmol/mL (0.3 nmol) | 20 | 3 |
| 4 | Cdex@TLR_{A1} | 2.4 | 6 nmol/mL (0.3 nmol) | 20 | 3 |
| 5 | Cdex@TLR_{A1} x2 | 2.4 | 12 nmol/mL (0.6 nmol) | 20 | 4 |
| 6 | Cdex@TLR_{B1} | 2.8 | 6 nmol/mL (0.3 nmol) | 20 | 3 |

Compared to the control group (PBS injection), a time-dependent decrease in relative tumor volume was observed in the groups treated with Cdex@TLR_{A1}, Cdex@TLR_{A1} ×2, and Cdex@TLR_{B1}, in contrast to the groups treated with Doxorubicin or TLR_{A1} alone.

Compared to the control group receiving PBS injection, a time-dependent reduction in relative tumor volume was observed in the groups treated with Cdex@TLR_{A1}, Cdex@TLR_{A1} ×2, and Cdex@TLR_{B1}. This indicates the therapeutic efficacy of these formulations in tumor suppression. In contrast, treatment with Doxorubicin alone or TLR_{A1} agonist alone led to a reduction in tumor growth rate; however, no significant decrease in tumor size was observed.

### Example 3

This example investigates whether crosslinking various polysaccharides including dextran (see FIG. 19) can form polysaccharide-crosslinked colloidal particles usable as T1 MRI contrast agents.

### 3-1. Synthesis of Nanostructures Using Dextran T-5

A total of 180 µmol of dextran T-5 (FIG. 19 (a-i), average molecular weight: 5,000 Da) was dissolved in 9 mL of distilled water, followed by the addition of epichlorohydrin and NaOH. Subsequently, diethylenetriamine was added, and the mixture was stirred at room temperature (RT) for 24 hours. The resulting product was purified using a 5 kDa molecular weight cut-off (MWCO) filter.

### 3-2. Synthesis of Nanostructures Using Maltodextrin

The procedure was identical to that described in Example 3-1, except that maltodextrin (FIG. 19 (a-ii), average molecular weight: 990 Da) was used in place of dextran T-5.

### 3-3. Synthesis of Nanostructures Using α-Cyclodextrin

The procedure was identical to that described in Example 3-1, except that α-cyclodextrin (FIG. 19 (b-i), average molecular weight: 970 Da) was used in place of dextran T-5.

### 3-4. Synthesis of Nanostructures Using β-Cyclodextrin

The procedure was identical to that described in Example 3-1, except that β-cyclodextrin (FIG. 19 (b-ii), average molecular weight: 1,100 Da) was used in place of dextran T-5.

### 3-5. Synthesis of Nanostructures Using Inulin

The procedure was identical to that described in Example 3-1, except that inulin (FIG. 19 (c), average molecular weight: 2,500 Da) was used in place of dextran T-5.

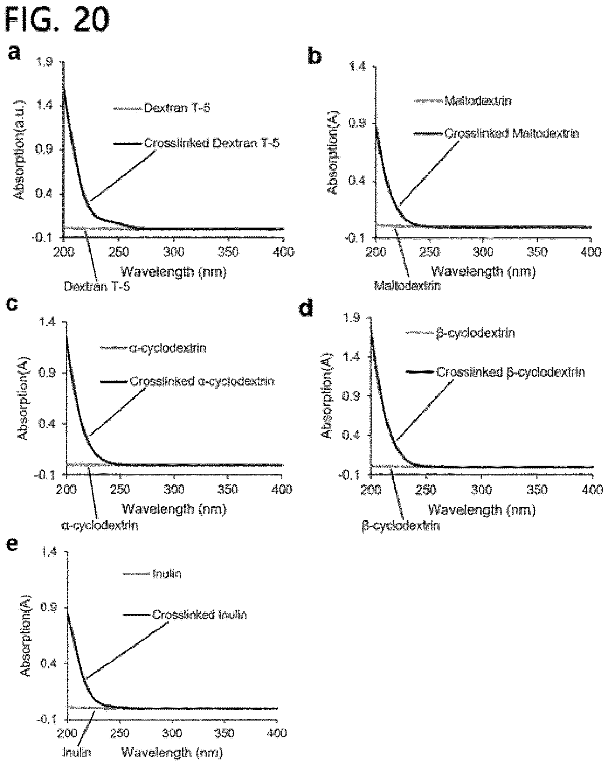

To confirm the crosslinking of the polysaccharides synthesized in Examples 3-1 to 3-5, each sample was prepared at an equal concentration, placed in a cuvette, and analyzed using a UV-Vis spectrophotometer in the 200-400 nm wavelength range. While typical polysaccharides do not exhibit absorbance in the visible range, nanoparticles formed by crosslinking of polysaccharides scatter transmitted light and thereby exhibit an absorption phenomenon at shorter wavelengths. As shown in FIG. 20, the polysaccharides before crosslinking showed no absorbance in the observed range (FIG. 20, orange line), whereas the crosslinked polysaccharides exhibited absorption in the short-wavelength range (200-250 nm), confirming the formation of nanostructures via crosslinking of polysaccharides (FIG. 20, black line).

The nanostructures synthesized in Examples 3-1 through 3-5 contained surface amine groups introduced via diethylenetriamine. To quantify the number of surface amine groups per nanostructure, an o-phthalaldehyde assay was performed. The results showed that dextran T-5 contained 12.7 amine groups, maltodextrin 4.5, α-cyclodextrin 8.1, β-cyclodextrin 9.0, and inulin 8.0. This indicates that the synthesis method consistently introduces amine groups onto the surface of all nanostructures.

### 3-6. Introduction of Carboxyl Groups onto Nanostructures

To each of the nanostructures prepared in Examples 3-1 to 3-5, 25 mg of succinic anhydride was added and the mixture was stirred for 24 hours at room temperature to induce a succinylation reaction. The product was purified using a 5 kDa MWCO filter.

The number of residual amine groups in the materials from Example 3-6 was measured using the o-phthalaldehyde assay. The number of residual amine groups per nanostructure was as follows: dextran T-5, 0.7; maltodextrin, 0.2; α-cyclodextrin, 0.2; β-cyclodextrin, 0.2; and inulin, 0.4. A value of less than or equal to 1 indicates that essentially all amine groups were converted into carboxyl groups. Therefore, it was confirmed that the amine groups on the nanostructures derived from various polymers were successfully replaced with carboxyl groups in this reaction.

### 3-7. Iron Loading onto Nanostructures

To the carboxylated nanostructures of Example 3-6, 45 µL of ferric chloride hexahydrate solution was added. The pH was adjusted to 8 using 2.5 M NaOH, and the mixture was reacted at room temperature for 1 hour. The product was purified using a 5 kDa MWCO filter to yield the iron-loaded nanostructures.

The iron content and polymer content of the materials from Example 3-7 were analyzed. Surface-bound iron was measured using inductively coupled plasma (ICP) analysis, and the content of each polymer was determined by the phenol-sulfuric acid method. The amount of iron bound per 1 mg of polymer was as follows: dextran T-5, 0.03 mg; maltodextrin, 0.026 mg; α-cyclodextrin, 0.033 mg; β-cyclodextrin, 0.026 mg; and inulin, 0.029 mg. These results confirmed that similar amounts of iron were introduced across different polymer-based nanostructures under the same reaction conditions.

### 3-8. Measurement of Hydrodynamic Diameter and Surface Charge of Iron-Loaded Nanostructures

The hydrodynamic diameter of the iron-loaded nanostructures obtained in Example 3-7 was analyzed using dynamic light scattering (DLS). The results indicated that dextran T-5 exhibited a diameter of 3.6 nm, maltodextrin 6.8 nm, α-cyclodextrin 2.9 nm, β-cyclodextrin 2.8 nm, and inulin 3.8 nm, confirming that the synthesized nanostructures possess similar hydrodynamic sizes. Surface charge measurements showed values of -3.01 mV for dextran T-5, -6.62 mV for maltodextrin, -9.06 mV for α-cyclodextrin, -7.32 mV for β-cyclodextrin, and -2.83 mV for inulin. These results confirm that the synthesized iron-loaded nanostructures exhibit comparable surface charge and hydrodynamic diameter.

### 3-9. Viscosity Comparison of Nanostructures

The nanostructures from Example 3-7 were prepared at a concentration of 5 mg/mL, and viscosity measurements were performed at 25°C. The viscosity values of the crosslinked polymers were 1.09 mPa·s for dextran T-5, 1.43 mPa·s for maltodextrin, 1.26 mPa·s for α-cyclodextrin, 1.30 mPa·s for β-cyclodextrin, and 1.14 mPa·s for inulin, indicating similar viscosity profiles among the nanostructures.

### 3-10. T1 MRI Contrast Performance Evaluation of Nanostructures

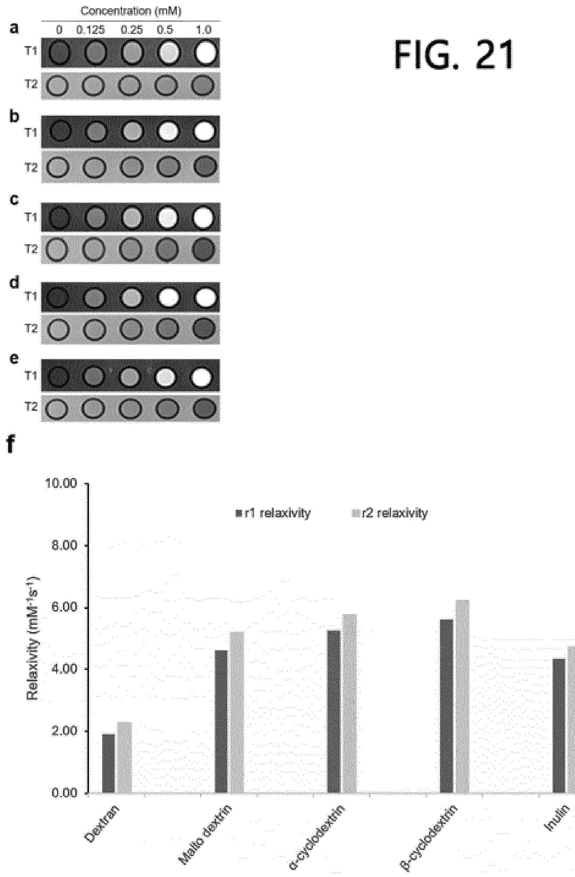

To evaluate the T1 MRI performance of the materials from Example 3-7, both the spin-spin relaxivity coefficient (r₂) and spin-lattice relaxivity coefficient (r₁) were measured, and the r₂/r₁ ratio was calculated. The r₂/r₁ ratio is a critical indicator in determining whether a contrast agent is suitable for T1 or T2 MRI. As shown in FIG. 21, 3.0 Tesla MRI analysis revealed the following: dextran T-5 exhibited an r₁ of 1.90 and r₂ of 2.27 (r₂/r₁ = 1.19) (a, f); maltodextrin exhibited r₁ = 4.60, r₂ = 5.19 (r₂/r₁ = 1.13) (b, f); α-cyclodextrin exhibited r₁ = 5.26, r₂ = 5.78 (r₂/r₁ = 1.10) (c, f); β-cyclodextrin exhibited r₁ = 5.62, r₂ = 6.24 (r₂/r₁ = 1.11) (d, f); and inulin exhibited r₁ = 4.33, r₂ = 4.73 (r₂/r₁ = 1.09) (e, f). These results indicate that all synthesized nanostructures possess r₂/r₁ ratios close to 1, thereby confirming their suitability as T1 MRI contrast agents.

### 3-11. In Vivo Contrast Efficacy Evaluation of Iron-Loaded Nanostructures

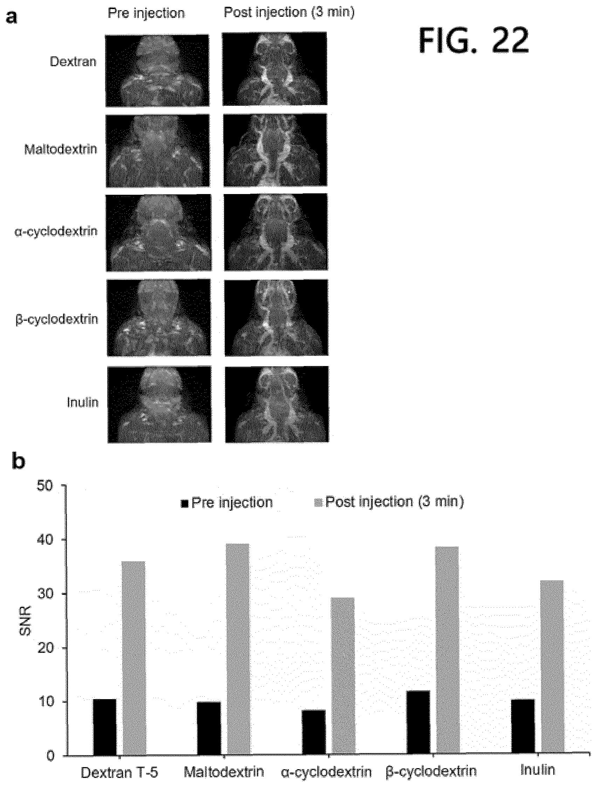

The nanostructures from Example 3-7 were administered intravenously to mice, and T1-weighted images were acquired using 9.4 Tesla MRI. Male Balb/c mice (5 weeks or older) were anesthetized and injected via the tail vein with the nanostructures. T1-weighted MRI images were obtained at various time points, and signal-to-noise ratio (SNR) was analyzed. As shown in FIG. 22, all iron-loaded nanostructures from Example 3-7 exhibited bright vascular signals (e.g., in the jugular vein) within approximately 3-5 minutes post-injection.

### 3-12. Renal Excretion of Iron-Loaded Nanostructures

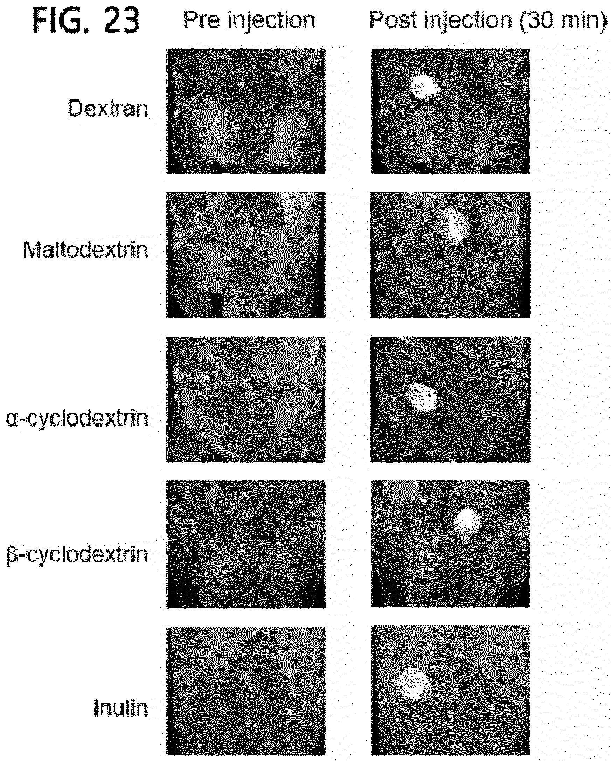

Following intravenous administration of the nanostructures from Example 3-7, T1-weighted MRI scans were performed for up to 1 hour using a 9.4 Tesla MRI to evaluate renal clearance. As shown in FIG. 23, T1 signals began to appear in the bladder within 30 minutes for most iron-loaded nanostructures. These observations indicate that the administered agents passed through the kidneys and were excreted into the bladder, confirming renal clearance.

### Example 4: Shoulder MR Arthrography Using the Dextran-Crosslinked T1 MRI Contrast Agent NEMO-103

### 4-1. Preparation of the Dextran-Crosslinked T1 MRI Contrast Agent NEMO-103

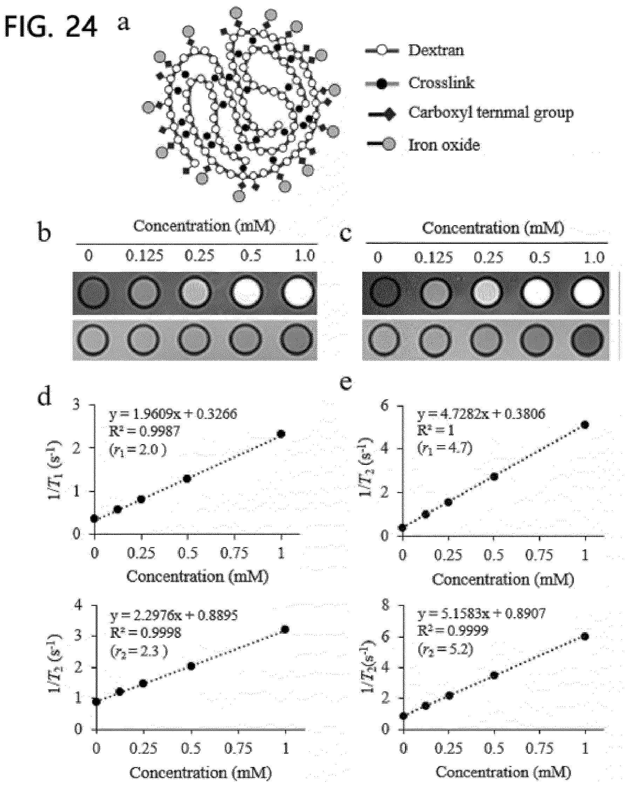

Dextran-crosslinked nanoparticles were synthesized by mixing an aqueous solution of 20 mM dextran T10 with sodium hydroxide solution and epichlorohydrin, followed by the addition of ethylenediamine. The resulting dextran-crosslinked nanoparticles were purified using a 10 kDa molecular weight cutoff (MWCO) filter. The terminal amine groups of the dextran-crosslinked nanoparticles were then substituted with carboxyl groups via reaction with succinic anhydride. After further purification using a 10 kDa MWCO filter, the carboxylated dextran-crosslinked nanoparticles were reacted with ferric chloride solution for 1 hour and subsequently purified with a 10 kDa MWCO filter to yield the dextran-crosslinked T1 MRI contrast agent NEMO-103 (FIG. 24a).

### 4-2. Phantom study

In a phantom study using a 3T MRI scanner, NEMO-103 exhibited bright T1 signal intensity on T1-weighted images, comparable to that of Dotarem^{®} (FIGS. 24b and 24c). The measured r₁ and r₂ relaxivity values of NEMO-103 were 2.0 mM⁻¹s⁻¹ and 2.3 mM⁻¹s⁻¹, respectively (FIGS. 24d and 24e). The r₂/r₁ ratio, which is an important parameter for T1 MRI contrast agents, was calculated to be 1.15. Given that an ideal T1 contrast agent exhibits an r₂/r₁ ratio close to 1, the observed ratio of 1.15 indicates that NEMO-103 is an ideal T1 MRI contrast agent. For comparison, the measured r₁ and r₂ values of Dotarem^{®} were 4.7 mM⁻¹s⁻¹ and 5.2 mM⁻¹s⁻¹, respectively, resulting in an r₂/r₁ ratio of 1.11.

### 4-3. Image Quality Assessment

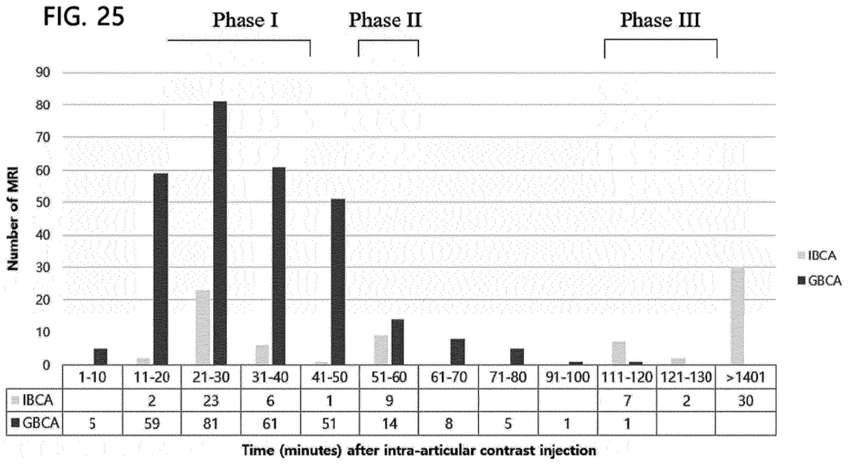

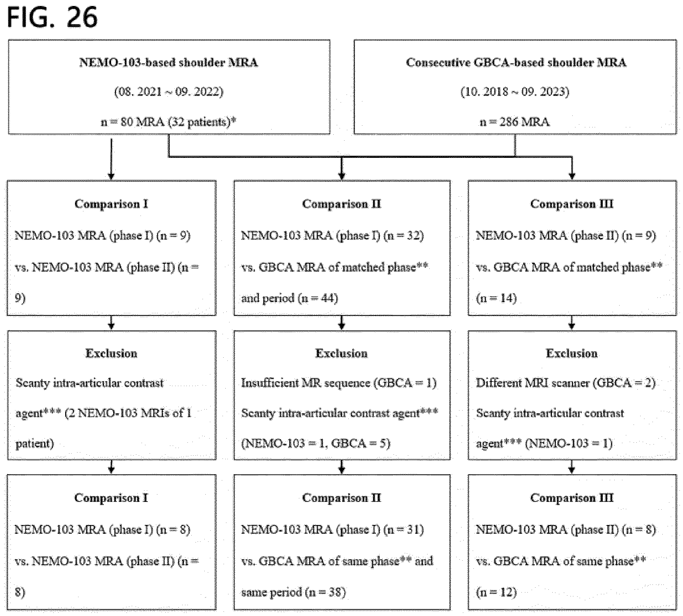

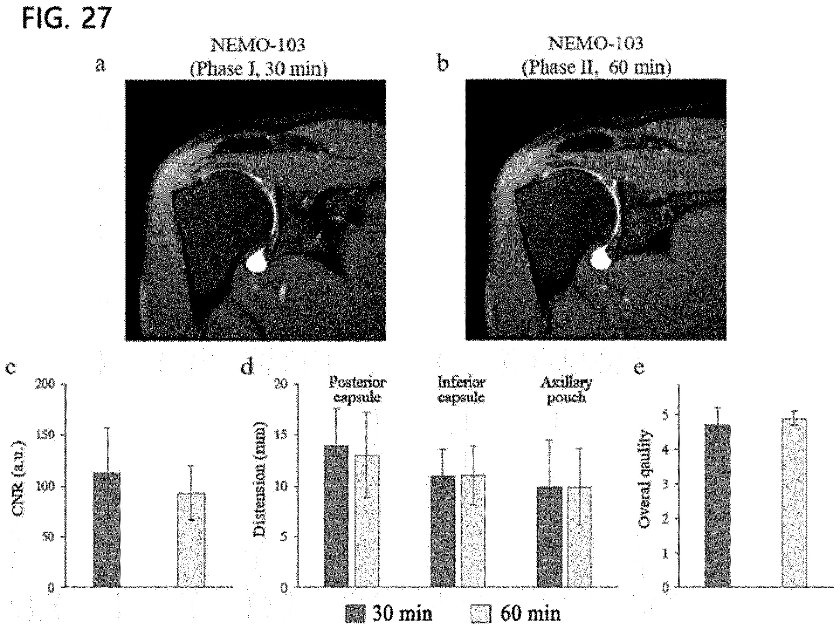

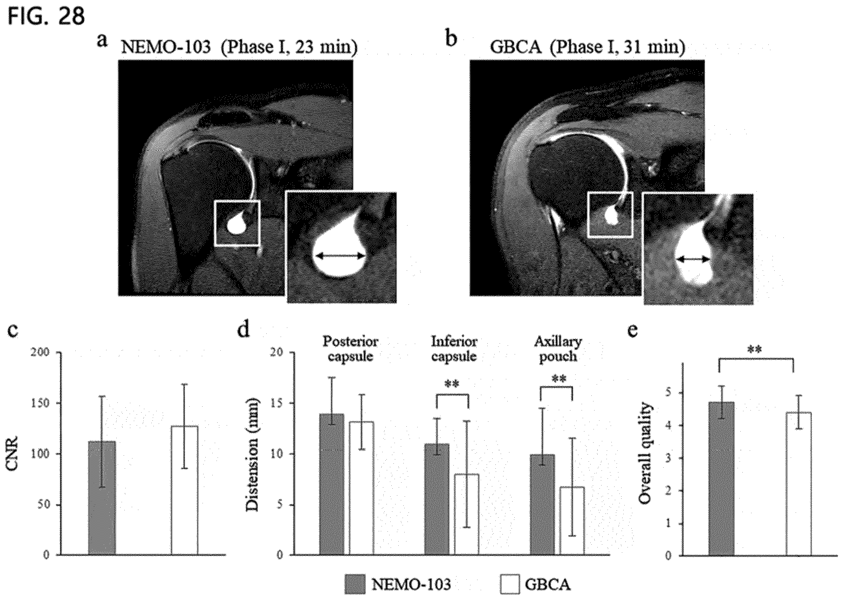

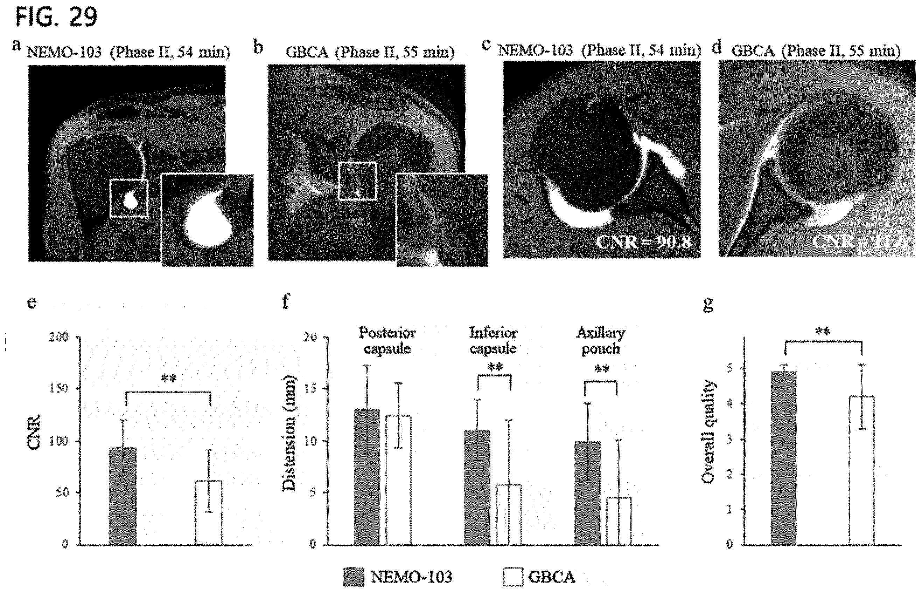

FIGS. 25 and 26 illustrate the phase design and image quality assessment method of a clinical comparative study between NEMO-103, a dextran-crosslinked T1 MRI contrast agent, and Dotarem^{®}, a representative clinical gadolinium-based contrast agent (GBCA) for T1 MRI. Phase I refers to MR arthrography (MRA) images acquired within 30 minutes after contrast agent administration. Phase II refers to MRA images acquired between 30 and 60 minutes post-administration, and Phase III refers to MRA images acquired between 110 and 130 minutes post-administration. In Comparison 1, no quantitative or qualitative differences were observed between NEMO-103-based MRA images acquired during Phase I and Phase II (FIG. 27). In Comparison 2, when comparing NEMO-103-based MRA images from Phase I with GBCA-based MRA images, no difference was observed in contrast-to-noise ratio (CNR); however, statistically significant differences were found in capsular distension (p < 0.05, FIG. 28). Specifically, NEMO-103-based MRA images showed higher scores in overall image quality. Although no significant difference in posterior capsular distension was observed between the two groups, NEMO-103-based MRA images showed superior distension in both the inferior capsule and the axillary pouch. In Comparison 3, which compared NEMO-103-based MRA images from Phase II with GBCA-based MRA images, more prominent differences were observed (FIG. 29). The CNR was significantly higher in the NEMO-103 group than in the GBCA group. In addition, more pronounced differences in inferior capsular and axillary pouch distension were observed. As a result, NEMO-103-based MRA images achieved superior outcomes in both inferior capsular and axillary pouch distension.

### 4-4. Visual Turing Test (VTT)

In the VTT for Comparison 1, radiologists were instructed to distinguish between NEMO-103-based MRA images obtained in Phase I and Phase II. The combined accuracy of the 8 testers was 46.8% (146/312), showing no statistically significant difference compared to random guessing (50.0%, 156/312; p = 0.423). In the VTT for Comparison 2, radiologists were instructed to differentiate between NEMO-103-based and GBCA-based MRA images acquired in Phase I. The combined accuracy of the 32 testers averaged 53.3% (624/1170), with no statistically significant difference compared to random guessing (50.0%, 585/1170; p = 0.107).

### [Discussion]

Amid growing safety concerns surrounding gadolinium-based contrast agents (GBCAs), iron (Fe)-based alternatives have emerged as a subject of active investigation. The present example evaluates the efficacy of NEMO-103 in comparison with a GBCA by assessing contrast-to-noise ratio (CNR) and MR arthrography (MRA) image quality. In direct intra-articular shoulder MRA, NEMO-103 (0.035 mg Fe/kg) and GBCA (0.037 mg Gd/kg) were each administered via direct joint injection, and the resulting CNR and visual Turing test (VTT) outcomes at 30 minutes post-injection (Comparison 2) demonstrated nearly equivalent image quality. Both contrast agents effectively delineated key anatomical structures, including tissue contrast, sharpness, rotator cuff tendons, labral structures, and adjacent cartilage. Furthermore, NEMO-103 maintained an enhanced CNR even at 120 minutes post-injection.

According to this example, the MRA image quality at 30 minutes (Phase I) and 60 minutes (Phase II) post-injection could not be distinguished in the visual Turing test, suggesting the potential extension of the imaging time window following injection. This may be advantageous for improving the operational efficiency of MR imaging facilities in clinical settings. According to current reports, particulate contrast agents with a hydrodynamic diameter of 3 nm are excreted from the joint cavity solely via lymphatic vessels due to their larger size and are not cleared through the venous system. Given that NEMO-103 has a larger hydrodynamic size (approximately 4.0 nm), it can only be excreted via lymphatic drainage, leading to prolonged retention within the joint cavity. In contrast, GBCAs (<1 nm) are rapidly cleared through both lymphatic and venous routes, thus limiting the time window available for image acquisition. However, in the MRA images taken 24 hours after administration, complete clearance of NEMO-103 from the joint cavity was confirmed, and no residual contrast agent was detected.

On the other hand, there was no statistically significant difference in posterior capsular distension between the MRA images based on NEMO-103 and those based on GBCA. This is presumed to be attributable to the patient's positioning during shoulder MRA. In the supine position, joint fluid tends to shift posteriorly, resulting in relatively less residual fluid in anatomical structures such as the undersurface of the subscapularis tendon and the axillary pouch. Over time, as the contrast agent is resorbed, this difference becomes more pronounced in the case of GBCA. Consequently, GBCA exhibited a relatively higher incidence of axillary pouch obliteration compared to NEMO-103. Moreover, the anterior aspect of the glenohumeral joint includes critical structures such as the subscapularis tendon and the anteroinferior glenoid labrum. Since multiple structures must be evaluated in this region, resorption of joint fluid and positional shifts caused by the supine posture may affect the conspicuity of lesions in this anterior compartment.

Unlike the long-established use of GBCAs, the novel contrast agent NEMO-103 requires safety evaluation. According to the clinical study report, treatment-emergent adverse events (TEAEs) were observed in 3 out of 32 subjects (9.4%) who underwent NEMO-103-based MRA, including two cases of localized injection-site reactions (muscle pain) and one case of hypertension. These adverse events were considered "unrelated to the investigational drug" in terms of causality, and all subjects recovered without medical intervention. Furthermore, no drug-related adverse events (ADRs), serious adverse events (SAEs), or severe adverse events occurred, and no subject discontinued participation due to these events. Evaluation of residual NEMO-103 in the liver and spleen at 24 hours post-injection confirmed complete clearance in all 30 subjects (100%) who underwent delayed imaging. The relative signal intensity (rSI) differences between the pre-contrast abdominal MRI and the 24-hour delayed MRI were all positive (liver: 13.2 ± 4.0; spleen: 5.5 ± 0.9), indicating elimination of the contrast agent. Two subjects did not undergo 24-hour delayed imaging that included liver and spleen evaluation.

Another advantage of NEMO-103 lies in its potential to eliminate reliance on conventional off-label use. Traditional GBCA-based MR arthrography requires a laborious dilution process of approximately 1:200, which increases the risk of contamination and infection, while also hindering standardization of contrast agent concentration. In contrast, NEMO-103 is supplied as a vial formulation pre-diluted to a concentration suitable for MR arthrography, thereby offering relative freedom from issues such as infection, contamination, and heterogeneity in contrast concentration.

As a retrospective study involving a limited number of patients, the present investigation has several limitations. First, since the subjects were not patients who underwent arthroscopic surgery, the diagnostic accuracy of NEMO-103-based shoulder MRA and GBCA-based shoulder MRA for individual lesions could not be evaluated with reference to surgical findings. However, by emphasizing the role of the contrast agent within the joint cavity-such as enhancement of contrast between surrounding structures and distension of the joint space-meaningful differences over time between MRA images using NEMO-103 and those using GBCA were observed. In addition, through a visual Turing test (VTT) involving a large number of testers, NEMO-103 demonstrated image quality comparable to that of GBCA from various perspectives. Future clinical trials may be designed to involve patients requiring surgical intervention, using operative records as the gold standard to evaluate the diagnostic accuracy of both contrast agents. Second, this study did not assess both contrast agents in the same subject. Individual variability may exist in joint cavity distension and contrast agent resorption. However, performing two MRAs using different contrast agents within a short time interval in the same patient presents a substantial challenge in study design. To overcome this limitation, the time interval between contrast agent injection and image acquisition was carefully reviewed, and phase matching was applied for comparative analysis. Third, it is noteworthy that the GBCA group was significantly older than the NEMO-103 group. Although not definitively proven, potential age-related differences in contrast agent resorption rates may exist. Nevertheless, considering that synovial fluid volume tends to increase with more severe degeneration and symptoms such as pain, it is plausible that the GBCA group, consisting of actual patients, had a greater synovial fluid volume than the relatively younger, volunteer-based NEMO-103 group.

In conclusion, NEMO-103-based MR arthrography of the shoulder is, at minimum, interchangeable with GBCA-based MR arthrography in terms of contrast-to-noise ratio (CNR), joint distension, image quality, and VTT performance. Moreover, it demonstrated enhanced resistance to contrast agent resorption over time, suggesting a potential extension of the feasible imaging time window

## Claims

1. A conjugate comprising two or more molecules linked via a multivalent linker system comprising a polysaccharide-crosslinked colloidal particle, wherein:
the polysaccharide-crosslinked colloidal particle comprises two or more crosslinker-derived surface functional groups thereof; and
the polysaccharide-crosslinked colloidal particle is formed by intra- and/or inter-molecular crosslinking of 1 to 3 branched polysaccharides or 2 to 30 cyclic polysaccharides at hydroxyl groups of the monosaccharide building blocks thereof.

2. The conjugate of claim 1, wherein the polysaccharide-crosslinked colloidal particle is engineered to have a hydrodynamic diameter of 2 to 20 nm, preferably 10 nm or less, more preferably 8 nm or less, and to have surface carboxyl groups such that the surface charge is between -20 mV and 0 mV, whereby the particle is filtered through the kidney, is impermeable to vascular walls of normal blood capillaries, and is selectively drained into lymphatic vessels.

3. The conjugate of claim 1, wherein a targeting molecule is bound to the crosslinker-derived surface functional groups of the polysaccharide-crosslinked colloidal particle.

4. The conjugate of claim 1, wherein at least one antibody or antigen-binding fragment thereof, and at least one drug molecule, are simultaneously conjugated via two or more crosslinker-derived functional groups on the polysaccharide-crosslinked colloidal particle.

5. The conjugate of claim 4, wherein the average number of polysaccharide-crosslinked colloidal particles conjugated to each antibody molecule is in the range of 1 to 4.

6. The conjugate of claim 4, wherein the number of drug molecules conjugated thereto is controllable by adjusting the number of crosslinker-derived functional groups introduced into the polysaccharide-crosslinked colloidal particle.

7. The conjugate of claim 6, wherein the drug-to-antibody ratio (DAR) is from 8 to 80.

8. The conjugate of claim 1, wherein the polysaccharide-crosslinked colloidal particle and/or the molecule conjugated thereto via the crosslinker-derived functional group is a diagnostic agent and/or a therapeutic agent.

9. The conjugate of claim 1, wherein the crosslinker-derived surface functional group of the polysaccharide-crosslinked colloidal particle is a terminal group of the crosslinker or a modified derivative thereof.

10. The conjugate of claim 1, wherein the crosslinker-derived surface functional group of the polysaccharide-crosslinked colloidal particle is selected from the group consisting of an amine group, a carboxyl group, a hydroxyl group, and a thiol group.

11. The conjugate of claim 1, wherein the crosslinker-derived surface functional group of the polysaccharide-crosslinked colloidal particle binds a drug via a coordination bond, a covalent bond, a hydrogen bond, and/or an electrostatic interaction.

12. The conjugate of claim 1, wherein the hydrodynamic size and net surface charge of the polysaccharide-crosslinked colloidal particle are engineered by adjusting at least one parameter selected from the group consisting of: the molecular weight of the branched or cyclic polysaccharide, the backbone length of the branched or cyclic polysaccharide, the type of crosslinker, the amount and rate of crosslinker added during synthesis, and the degree of further chemical modification, such that a desired blood circulation time, biodistribution, and/or pharmacokinetics in vivo are achieved.

13. The conjugate of claim 1, wherein the polysaccharide-crosslinked colloidal particle is engineered to inhibit phagocytic uptake by macrophages.

14. The conjugate of claim 1, wherein the type and/or amount of the crosslinker used to prepare the polysaccharide-crosslinked colloidal particle is adjusted to inhibit phagocytic uptake by macrophages and/or to prolong blood circulation time.

15. The conjugate of claim 2, wherein a hydrophobic drug is conjugated to the linker comprising the polysaccharide-crosslinked colloidal particle, the particle having high hydrophilicity due to the hydration of hydrophilic surface functional groups whose density can be controlled, thereby enhancing water dispersibility of the conjugate.

16. The conjugate of claim 1, wherein the substitution ratio of the crosslinker in the polysaccharide-crosslinked colloidal particle is adjusted to 2% to 50% relative to the number of reactive groups on the polysaccharide, and wherein 2% to 98% of the crosslinker used is configured such that one terminal end remains unreacted and exposed on the surface, thereby controlling the surface charge of the polysaccharide-crosslinked colloidal particle.

17. The conjugate of claim 1, wherein the polysaccharide-crosslinked colloidal particle is engineered to control the circulation half-life of a drug in the bloodstream and/or to modulate its in vivo biodistribution and excretion.

18. The conjugate of any one of claims 1 to 17, wherein the polysaccharide-crosslinked colloidal particle exhibits magnetic resonance imaging (MRI) contrast enhancement functionality by chelating divalent or trivalent iron ions to crosslinker-derived surface functional groups.

19. The conjugate of any one of claims 1 to 17, wherein the polysaccharide-crosslinked colloidal particle is prepared by a method comprising:
Step 1 of preparing an aqueous solution of dextran or a dextran derivative;
Step 2 of dropwise adding an epoxide and an alkaline aqueous solution at room temperature;
Step 3 of dropwise adding divalent or higher-valent polyamines at room temperature to form dextran-crosslinked nanoparticles bearing terminal amine groups;
Step 4 of precipitating the resulting product;
Step 5 of redispersing the precipitated product in water;
optionally, Step 6 of recovering the dextran-crosslinked nanoparticles bearing terminal amine groups by dialysis;
Step 7 of adding an organic acid anhydride to the dextran-crosslinked nanoparticles bearing terminal amine groups, so as to substitute at least a portion or all of the terminal amine groups with carboxylic acid and/or carboxylate groups;
optionally, Step 8 of purifying a solution of dextran-crosslinked nanoparticles bearing carboxylic acid and/or carboxylate groups to obtain water-dispersible dextran-crosslinked nanoparticles;
optionally, Step 9 of adding an iron precursor (e.g., iron chloride) or an aqueous solution of iron oxide nanoparticles to the water-dispersible dextran-crosslinked nanoparticles prepared in the previous step, to obtain (i) dextran-crosslinked nanoparticles having a shell formed of divalent or trivalent iron ions, or (ii) complexes in which the surface of iron oxide nanoparticles is surface-modified with the dextran-crosslinked nanoparticles; and
optionally, Step 10 of purifying or concentrating the nanostructures obtained in the previous step by ultrafiltration.

20. A polysaccharide-crosslinked colloidal particle-drug conjugate, comprising:
a colloidal particle formed by intra- and/or inter-molecular crosslinking of one to three branched polysaccharides or two to thirty cyclic polysaccharides at hydroxyl groups of the monosaccharide building blocks thereof using a crosslinker; and
a drug conjugated to the colloidal particle via surface functional groups derived from the crosslinking molecules,
wherein the colloidal particle functions as a drug carrier.

21. The conjugate of claim 20, wherein the polysaccharide-crosslinked colloidal particle:
has a hydrodynamic diameter of 2 to 20 nm, preferably 10 nm or less, more preferably 8 nm or less, and even more preferably 6 nm or less;
has surface carboxyl groups that provide a surface charge between -20 mV and 0 mV; and
is designed to be filtered through the kidney, to be impermeable to vascular walls of normal blood capillaries, and to be selectively drained into lymphatic vessels.

22. A targeted drug carrier comprising a polysaccharide-crosslinked colloidal particle, wherein:
the colloidal particle is configured to serve as a drug carrier and is formed by intra- and/or inter-molecular crosslinking of one to three branched polysaccharides or two to thirty cyclic polysaccharides at hydroxyl groups of their monosaccharide building blocks using a crosslinker; and
a targeting molecule is conjugated to the surface of the colloidal particle via a crosslinker-derived functional group.

23. The targeted drug carrier of claim 22, wherein the targeting molecule is a Toll-like receptor (TLR) ligand, agonist, antagonist, enhancer, inhibitor, or blocker.
